# EUROPEAN PATENT SPECIFICATION

(11) **EP 3 923 971 B1**
(45) Date of publication and mention of the grant of the patent: **20.12.2023**
(21) Application number: 20704031.2
(22) Date of filing: 13.02.2020
(51) Int. Cl.: A61K 38/17, A61K 9/00, A61K 48/00, A61K 31/7105, C12Q 1/6883, C12N 15/67, A61P 11/00, A61K 31/198, G01N 33/50

(54) **ANALYZING THE EFFECT OF A POLYRIBONUCLEOTIDE ON CILIOGENESIS**
ANALYSE DER WIRKUNG EINES POLYRIBONUKLEOTIDS AUF DIE CILIOGENESE
ANALYSE DE L'EFFET D'UN POLYRIBONUCLÉOTIDE SUR LA CILIOGÉNÈSE

(30) Priority: 14.02.2019 EP 19157210
(43) Date of publication of application: 22.12.2021
(60) Divisional application: 23164871.8
(73) Proprietor: ethris GmbH, 82152 Planegg (DE)
(72) Inventor: RUDOLPH, Carsten, 82152 Krailling (DE); MUMMERT, Verena, 82377 Penzberg (DE); KUBISCH-DOHMEN, Rebekka, 82061 Neuried (DE); DOHMEN, Christian, 82061 Neuried (DE); GEIGER, Johannes, 81249 Munich (DE); ANEJA, Manish, 72108 Rottenburg am Neckar (DE); WEISS, Ludwig, 86438 Kissing (DE); OMRAN, Heymut, 48151 Münster (DE); PENNEKAMP, Petra, 48149 Münster (DE); WOHLGEMUTH, Kai, 48317 Drensteinfurt (DE); CINDRIC, Sandra, 81245 München (DE); LOGES, Niki Tomas, 48429 Rheine (DE); RAIDT, Johanna, 48145 Münster (DE); TER STEEGE, Adrian, 53127 Bonn (DE)
(74) Representative: Vossius & Partner Patentanwälte Rechtsanwälte mbB
(86) International application number: PCT/EP2020/053774
(87) International publication number: WO 2020/165352

(56) References cited:
- WO-A1-2017/205767
- WO-A1-2018/203092
- US-A1- 2015 126 589
- ZIHLIF NADWA ET AL: "Markers of airway inflammation in primary ciliary dyskinesia studied using exhaled breath condensate.", PEDIATRIC PULMONOLOGY JUN 2006, vol. 41, no. 6, June 2006 (2006-06), pages 509-514, XP002792982, ISSN: 8755-6863
- TERRÉ BERTA ET AL: "GEMC1 is a critical regulator of multiciliated cell differentiation.", THE EMBO JOURNAL 02 05 2016, vol. 35, no. 9, 2 May 2016 (2016-05-02), pages 942-960, XP002792983, ISSN: 1460-2075
- Mustafa Mohamed Munye: "Towards gene therapy for primary ciliary dyskinesia (Thesis)", Institute of Child Health, University College London , July 2014 (2014-07), pages 1-304, XP002799489, London Retrieved from the Internet: URL:https://discovery.ucl.ac.uk/id/eprint/ 1435816/2/Thesis%20FINAL%20submitted.pdf [retrieved on 2020-06-22]
- MUNYE MUSTAFA M ET AL: "Novel Human Bronchial Epithelial Cell Lines for the Study of Gene Therapy Approaches To Treat Primary Ciliary Dyskinesia", MOLECULAR THERAPY, vol. 21, no. Suppl. 1, June 2013 (2013-06) , page S168, XP9521131, & 16TH ANNUAL MEETING OF THE AMERICAN-SOCIETY-OF-GENE-AND-CELL-THERAPY (ASGCT); SALT LAKE CITY, UT, USA; MAY 15 -18, 2013
- MUNYE M ET AL: "Towards gene therapy for primary ciliary dyskinesia", CILIA, BIOMED CENTRAL LTD, LONDON, UK, vol. 1, no. Suppl 1, 16 November 2012 (2012-11-16), page P109, XP021123150, ISSN: 2046-2530, DOI: 10.1186/2046-2530-1-S1-P109
- JEREMY C MCINTYRE ET AL: "Gene therapy rescues cilia defects and restores olfactory function in a mammalian ciliopathy model", NATURE MEDICINE, vol. 18, no. 9, 1 September 2012 (2012-09-01), pages 1423-1428, XP055707286, New York ISSN: 1078-8956, DOI: 10.1038/nm.2860
- GABRIELLE WHEWAY ET AL: "An siRNA-based functional genomics screen for the identification of regulators of ciliogenesis and ciliopathy genes", NATURE CELL BIOLOGY, vol. 17, no. 8, 1 August 2015 (2015-08-01) , pages 1074-1087, XP55707232, GB ISSN: 1465-7392, DOI: 10.1038/ncb3201
- HORANI AMJAD ET AL: "Genetics and biology of primary ciliary dyskinesia", PAEDIATRIC RESPIRATORY REVIEWS, W.B. SAUNDERS, AMSTERDAM, NL, vol. 18, 11 September 2015 (2015-09-11), pages 18-24, XP029446039, ISSN: 1526-0542, DOI: 10.1016/J.PRRV.2015.09.001
- JUNEKEHLET MARTHIN ET AL: "Patient-specific three-dimensional explant spheroids derived from human nasal airway epithelium: a simple methodological approach for ex vivo studies of primary ciliary dyskinesia", CILIA, BIOMED CENTRAL LTD, LONDON, UK, vol. 6, no. 1, 23 March 2017 (2017-03-23), pages 1-9, XP021243370, DOI: 10.1186/S13630-017-0049-5
- JOON KIM ET AL: "Functional genomic screen for modulators of ciliogenesis and cilium length", NATURE, vol. 464, no. 7291, 15 April 2010 (2010-04-15), pages 1048-1051, XP055182068, ISSN: 0028-0836, DOI: 10.1038/nature08895

## Description

The present invention relates to a method for analyzing the effect of a polyribonucleotide on ciliogenesis, wherein said polyribonucleotide encodes a protein involved in and/or required for ciliogenesis.

Oxygen is vital for many multicellular organisms as it is crucial for the energy supply of the cells. In mammals, oxygen comprised in the air can enter the organism via the respiratory system, which refers to the organs involved in breathing. These include for example nose, throat, larynx, trachea, bronchi, and lungs. In the latter, gas from the environment is exchanged with gas comprised in the internal blood circulatory system, which transports the oxygen from the lungs to cells in different parts of the organism. However, besides the required oxygen also other components can enter the organism via the respiratory system such as irritating agents comprised in the air including pollutants, disease causing agents and pathogens such as viruses and bacteria. Hence, defense mechanisms exist such as cough reflexes and sneezing for expelling the irritating agent from the respiratory system. For expulsion, the irritating agent is embedded in viscous mucus that is secreted by epithelial cells in the respiratory system and then transported across the epithelial surface by ciliated cells.

Ciliated cells can transport mucus and irritating agents across the epithelial surface by a synchronized beat of mutiple motile cilia. Cilia are membrane-enclosed tubular structures that extend from the epithelial surface into the space of the respiratory system that is in contact with the environment. Within the cells, the axoneme of a cilium is anchored to a basal body via anchoring structures. An axoneme is a central bundle of microtubules in which nine outer doublet microtubules surround a central pair of singlet microtubules (i.e. in respiratory cilia). The outer doublet microtubules and the central pair of microtubules are connected by radial spokes. Each of the nine outer doublet microtubules consists of an A- and a B-tubulus with the doublets being circumferentially interconnected by a nexin-dynein regulatory complex. Inner dynein arm and outer dynein arm are connected to each A-tubulus. These dynein arms contain motor proteins that can walk along the microtubules, which results in bending and thus, beating of the cilium (cf. e.g. Lodish et al, 2000, Molecular Cell Biology, 4th edition, New York: W. H. Freeman, Section 19.4). Investigations of ciliary structures using the model *Trypanosoma brucei* indicated that some of these structural components exhibit a rapid turnover, whereas skeletal components of the radial spokes, the central pair and the outer dynein arms are primarily incorporated at the distal end of the ciliary structure during development (Vincensini et al., Biol Cell, 2018, 110:1-15).

As the synchronized beating of ciliated cells is crucial for the transport of fluids across epithelial surfaces, perturbations of ciliary structures can cause serious disorders. Motility defects including a reduced amplitude and/or frequency of beating and/or asynchronicity can occur as a result of a reduction or loss of dynein arms, a disorganisation of the microtubule arrangement including mislocalization and changes in the total number of microtubules per axoneme and combinations thereof for example. As far as known, changes in these functional axonemal elements are caused by underlying genetic defects. These are mostly due to changes in the sequence of a gene that encodes an axonemal component including the ones mentioned above. As genes can be transcribed into polyribonucleotides such as mRNAs, which in turn can be translated into proteins, the DNA sequence of a gene affects the synthesis of the respective protein in view of amount and functionality. As ciliary disorders, i.e. ciliopathies, caused by sequence changes are inherited and thus, are associated with motility defects of cilia at all developmental stages including embryo or newborns, ciliopathies can have severe consequences for the organism.

A well-known example of a ciliary disorder is primary ciliary dyskinesia (PCD), a progressive disorder that is often associated with declining lung function. Thus, long-term treatments including for example chest percussions and postural drainage are required for enhancing mucus clearance with increasing frequency, and in severe cases even lung transplantation. Further, PCD patients suffer from recurrent infections in lungs and/or ears, often also from subfertility, hydrocephalus and body laterality, i.e. left-right axis, defects, as well as retinal and/or neurological problems. But despite the severity of most ciliopathies, no standardized effective strategies for treating ciliopathies like PCD exist so far. Current therapies are for example extrapolated from cystic fibrosis and have in most cases not even been validated for the specific ciliopathy to be treated, such as for example PCD. Hence, there is a need to have at hand solutions for being able to efficiently treat subjects suffering of a ciliopathy such as PCD.

The present application addresses the need for restoring ciliary function in subjects suffering of a ciliopathy, such as PCD, by providing the embodiments as recited in the claims.

The present invention relates to a method for analyzing the effect of a polyribonucleotide on ciliogenesis, wherein said polyribonucleotide encodes a protein involved in and/or required for ciliogenesis, said method comprising the steps of:
(a) obtaining a nose brush of a subject having a ciliopathy, said nose brush comprising undifferentiated basal cells and differentiated ciliated cells,
(b) culturing the cells obtained from step (a) as a submerse cell culture for obtaining undifferentiated basal cells and dedifferentiated ciliated cells,
(c) culturing undifferentiated basal cells and dedifferentiated ciliated cells obtained from step (b) as an air liquid interface cell culture and performing an air lift,
(d) transfecting cells obtained from step (c) with a polyribonucleotide encoding a protein involved in and/or required for ciliogenesis,
(e) culturing the transfected cells obtained from step (d) for obtaining differentiated ciliated cells, and
(f) determining the effect of said polyribonucleotide on ciliogenesis using a lactate dehydrogenase measurement, a NucGreen assay, a high speed video microscopy, a ciliary beat frequency measurement, a mucociliary clearance assay, and/or immunofluorescence staining.

For obtaining cells of the respiratory epithelium of a patient, a nose brush is advantageous as it is easy to handle and the procedure fast and painless for the patient. Cells obtained from a nose brush comprise undifferentiated basal cells and differentiated ciliated cells that can be cultured as a submerse cell culture. By culturing the cells as submerse cell culture undifferentiated basal cells and dedifferentiated ciliated cells can be obtained as the submerse conditions induce a dedifferentiation of epithelial cells. The thus obtained dedifferentiated ciliated cells as well as the undifferentiated basal cells of the nose brush are then to be cultured as an air liquid interface (ALI) cell culture.

An air liquid interface cell culture of step (c) refers to a cell culture that is characterized in that the basal surface of the cells is in contact with a liquid culture medium, whereas the apical surface is exposed to air. Cells can be seeded for example onto a permeable membrane of a cell culture insert, which is initially supplied with culture medium to both the apical and basal compartments. This can also be referred to as "submerse cell culture". Once confluence is reached, the cells are then subjected to an "air-lift" step, where the medium is supplied only to the basal chamber. This mimics the conditions found in, for example, the respiratory system and induces cell differentiation including ciliogenesis in undifferentiated ciliary cells.

Thus, cells mimicking the epithelium of the respiratory system including, for example, basal and ciliated cells can be obtained for further investigation *in vitro.*

The obtained epithelium mimicking cells can then be transfected with a polyribonucleotide encoding a protein involved in and/or required for ciliogenesis. Thus, said polyribonucleotide is introduced into the cells which can be done artificially by viral infection or means other than viral infection to express the exogenous polyribonucleotide in the cells. The transfected cells are then cultured for obtaining differentiated ciliated cells from basal and undifferentiated ciliated cells. Thus, the effect of a polyribonucleotide such as an mRNA on ciliogenesis can be determined in *vitro* while preserving an environment of the cells that mimics the epithelium of the respiratory system as it can be found for example in a subject.

The effect of a polyribonucleotide on ciliogenesis can be determined by various methods known to a person skilled in the art. These methods include for example a lactate dehydrogenase measurement, a NucGreen assay, a high speed video microscopy, a ciliary beat frequency measurement, a mucociliary clearance assay, and/or immunofluorescence staining. Thus, a lactate dehydrogenase measurement (LDH) can be performed to determine the amount of LDH released in the environment surrounding the cells. The respective assay can be a colorimetric assay, wherein the amount of released LDH is measured with an enzymatic reaction which converts for example iodonitrotetrazolium or a tetrazolium salt into a red color formazan. Thus, the assay can be easily read out by spectroscopy.

Alternatively or additionally, a NucGreen assay can be performed. NucGreen is a permanent green fluorescing nucleic acid stain upon binding to nucleic acids and can be used to investigate toxicity-related effects of transfection for example. Alternatively or additionally, immunofluorescence staining of the protein encoded by the transfected polyribonucleotide can be performed to determine its amount and cellular localization including co-localization with other proteins. For determining the amount of the expressed protein, it is also possible to use Western Blot technology.

Furthermore, ciliary structural defects or lack of cilia can be detected for example using specialised microscopy. Screening tests include measurement of nasal nitric oxide production rate, ciliary motility by high speed video microscopy of nasal cells, or *in vivo* tests including a saccharin test for example. More specific diagnosis requires for example examination of cilia by immunofluorescence and transmission electron microscopy.

Alternatively or additionally, ciliary beat frequency measurement can be applied to investigate beat frequency, number of beating cilia and ciliated cells, and beating synchronicity using high speed video microscopy. The same applies to mucociliary clearance assays that can be performed to determine the rate of mucus clearance which is affected by ciliary function.

As regards the polyribonucleotide to be employed in the described method, the same applies as described above in connection with the pharmaceutical composition of the present invention for use in treating a ciliopathy. Moreover, also the other features of such a polyribonucleotide can be as described above.

Furthermore, the polyribonucleotide might contain a sequence encoding a marker. Examples include tdTomato (as comprised e.g. in **SEQ ID NO: 3**), green fluorescent protein (GFP), and enhanced GFP (eGFP) as described in the appended examples together with respective detection methods. This is especially advantageous for validating a successful transfection of a cell with the polyribonucleotide.

In some embodiments of the method for analyzing the effect of a polyribonucleotide on ciliogenesis, the cells are transfected within 0 to 12 days, preferably within 0 to 11 days, more preferably within 0 to 6 days, even more preferably 0 to 4 days and most preferably 0 to 48 hours after the air lift is performed in step (c). This is advantageous as the air lift step induces ciliogenesis of basal and undifferentiated ciliary cells.

In some embodiments of the method for analyzing the effect of a polyribonucleotide on ciliogenesis, the cells are transfected with a polyribonucleotide, preferably an mRNA, encoding a protein involved in and/or required for ciliogenesis using a lipidoid having the structure shown in formula (V), (VI), and/or (VII).

In some embodiments of the method for analyzing the effect of a polyribonucleotide on ciliogenesis, the cells are cultured in steps (b) to (e) using Medium G. The composition of Medium G is typically as shown in **Table 2**).

**Table 2**

| **Substance** | **Volume** | **Final concentration** |
|---|---|---|
| DMEM/Ham's F12 | 100 mL | |
| Ultroser G | 2 mL | 2 % |
| Fetal Clone II | 2 mL | 2 % |
| Insulin | 100 µL | 2.5 µg/mL |
| Bovine Brain Extract | 250 µL | 22.5 µg/mL |
| Transferrin | 100 µL | 2.5 µg/mL |
| Hydrocortisone | 20 µL | 20 nM |
| 3,3',5-Triiodo-L-thyronine | 3.33 µL | 500 nM |
| Epinephrine | 10 µL | 1.5 µM |
| Retinoic Acid | 10 µL | 10 nM |
| Phosphorylethanolamine | 5 µL | 250 nM |
| Ethanolamine | 100 µL | 250 nM |
| DAPT | 10 µL | 1 µM |

In some embodiments of the method for analyzing the effect of a polyribonucleotide on ciliogenesis, the cells are cultured in steps (b) to (e) using one of the media shown in **Table 3**. The media shown in **Table 3** are some commercially available media on the market that can be used to grow ALI culture.

**Table 3**

| **Supplier** | **Growth Phase** | **Differentiation Phase** |
|---|---|---|
| LONZA | ALI-G | ALI-D |
| StemCell | ALI-Ex | ALI-M |
| Epithelix | MucilAir | MucilAir |
| PELOBiotech | Pelo | Pelo |

In some embodiments of the method for analyzing the effect of a polyribonucleotide on ciliogenesis, the nose brush further comprises fibroblasts and wherein growth of said fibroblasts is inhibited in steps (b) to (e). This is advantageous as fibroblasts grow faster compared to basal and undifferentiated ciliary cells and can thus hamper the investigation of the effect of a polyribonucleotide on ciliogenesis as described above.

Before transferring the cells into the flask of the stationary phase, fibroblasts can be separated from the rest of the cells due to a short incubation time of 1-2 h. During that time, fibroblasts can settle down and adhere to the cell flask. All other cells remain in the media as suspension cells and can be easily transferred afterwards to the "stationary phase flask".

The method according to the present invention preferably results in the identification of a polyribonucleotide that has a positive effect on ciliogenesis and that can be used to restore ciliary cell structure in function and thus, in a pharmaceutical composition for use in treating a ciliopathy in a subject suffering of a ciliopathy. As regards features of the polyribonucleotide the function of which is analyzed in the method according to the present invention, these will be described further below.

The present invention is based on the finding that it is indeed possible to restore proper ciliary function by transfecting cells (which show a defect in a certain protein of a protein complex of the cilia and the defect of which leads to a loss of proper ciliary function) with polyribonucleotides which encode a functional version of said protein. However, it was also found that ciliated cells have to be transfected at an early stage during differentiation in order to achieve this effect. This leads to practical problems since the precursor cells of the epithelial cells which carry the cilia, i.e. basal cells, are not accessible for transfection via the airway system since they are located deeper down in the epithelium and are not exposed on the surface. A transfection of the epithelial cells by administration of a polyribonucleotide is effected when the subject which suffers of a ciliopathy shows an inflammation of the respiratory system. During an inflammation of the respiratory system the airway epithelium shows lesions and wounds which make precursor cells of the ciliated cells accessible, i.e. the basal cells which have not yet started ciliogenesis. Transfecting these cells with a polyribonucleotide identified in the method according to the present invention which expresses a functional version of the respective protein allows to render these cells into cells which form functional cilia or at least partly functional cilia which can lead to a substantial alleviation of the respective symptoms.

In the context of the present invention, the term "ciliopathy" refers to diseases associated with and/or characterized by defects of ciliated cells. Thus, ciliopathies comprise disorders of ciliary structures, including ciliary anchoring structures, basal bodies to which ciliary structures are anchored to within a cell, and/or ciliary function. Examples of ciliopathies include PCD, Bardet-Biedl syndrome, Simpson-Golabi-Behmel syndrome (type 2), leber congenital amaurosis, nephronophthisis, cranioectodermal dysplasia (Sensenbrenner) (cf. e.g. Mitchison et al., 2017, Ultrastructural Pathology,41 (6):415-427).

The term "ciliopathy" as used herein refers to a ciliopathy which is caused by a genetic defect in the DNA of a subject, e.g. the chromosomal or the mitochondrial DNA. Such a genetic defect may be caused by a mutation and can comprise loss, addition or exchange of a sequence part. Examples are copy number variation, presence/absence variation, deletion (full or partial), insertion, miss-sense mutation, nonsense mutation, splice site variation, or a combination thereof. Such changes in the DNA can lead to changes in the availability of the encoded protein such as a loss or a reduction of the amount of protein, or to a protein with altered function.

In a preferred embodiment the term "ciliopathy" refers to a disease connected with a defect in motile cilia. One example of such a ciliopathy is primary ciliary dyskinesia (PCD). PCD is a rare disease caused by dysfunction of motile cilia. PCD is heterogeneous at the genetic, functional and ultrastructural level. PCD is associated with impaired mucus transport and clearance. Subjects suffering from PCD show recurrent nasal congestions, sinus infections, ear infections, infertility, situs abnormalities such as situs inversus totalis and heterotaxy, also referred to as "situs ambiguous", and/or hydrocephalus. On the molecular level, PCD is associated in most cases with abnormalities in the structure, function, and biogenesis of cilia of the respiratory system. Examples for such abnormalities are absent or shortened dynein arms, defective central pair complex, radial spoke or nexin links. Such abnormalities and thus, ciliary motility defects associated with PCD is caused by mutations in genes encoding the respective components, in particular by mutations in genes listed in **Table 1,** wherein group "A" and "B" refer to genes with pathogenic mutations estimated to account for at least 1 % and less than 1 % of PCD cases, respectively (cf. Zariwala et al., GeneReviews®, 2007, updated 2015, Primary Ciliary Dyskinesia, editors Adam et al., Seattle (WA): University of Washington, Seattle; 1993-2018).

Hence, the polyribonucleotide analyzed in the method according to the present invention is preferably a polyribonucleotide that can be translated into a functional version of a protein listed in **Table 1.**

More preferably, the polyribonucleotide analyzed in the method according to the present invention is an mRNA that can be translated into a functional version of a protein selected from the group consisting of DNAH5, DNAH11, CCDC39, DNAI1, CCDC40, CCDC103, SPAG1, ZMYND10, ARMC4, CCDC151, DNAI2, RSPH1, CCDC114, RSPH4A, DNAAF1 (LRRC50), DNAAF2 (KTU), and LRRC6.

**Table 1**

| **Gene** | **Locus** | **Protein** | **Group** |
|---|---|---|---|
| *DNAH5* | CILD3 | DNAH5 | A |
| *DNAH11* | CILD7 | DNAH11 | A |
| *CCDC39* | CILD14 | CCDC39 | A |
| *DNAI1* | CILD1 | DNAI1 | A |
| *CCDC40* | CILD15 | CCDC40 | A |
| *CCDC103* | CILD17 | CCDC103 | A |
| *SPAG1* | CILD28 | SPAG1 | A |
| *ZMYND10* | CILD22 | ZMYND10 | A |
| *ARMC4* | CILD23 | ARMC4 | A |
| *CCDC151* | CILD30 | CCDC151 | A |
| *DNA12* | CILD9 | DNAI2 | A |
| *RSPH1* | CILD24 | RSPH1 | A |
| *CCDC114* | CILD20 | CCDC114 | A |
| *RSPH4A* | CILD11 | RSPH4A | A |
| *DNAAF1* (*LRRC50*) | CILD13 | DNAAF1 (LRRC50) | A |
| *DNAAF2* (*KTU*) | CILD10 | DNAAF2 (KTU) | A |
| *LRRC6* | CILD19 | LRRC6 | A |
| *C21orf59* | CILD26 | C21 orf59 | B |
| *CCDC65* (*DRC2*) | CILD27 | CCDC65 (DRC2) | B |
| *CCNO* | CILD29 | CCNO | B |
| *DNAAF3* | CILD2 | DNAAF3 | B |
| *DNAH1* | | DNAH1 | B |
| *DNAH8* | | DNAH8 | B |
| *DNAL1* | CILD16 | DNAL1 | B |
| *DRC1* (*CCDC164*) | CILD21 | DRC1 (CCDC164) | B |
| *DYX1C1* | CILD25 | DYX1C1 | B |
| *DNAAF5* (*HEATR2*) | CILD18 | DNAAF5 (HEATR2) | B |
| *HYDIN* | CILD5 | HYDIN | B |
| *MCIDAS* | | MCIDAS | B |
| *NME8* (*TXNDC3*) | CILD6 | NME8 (TXNDC3) | B |
| *RSPH3* | | RSPH3 | B |
| *RSPH9* | CILD12 | RSPH9 | B |

In some embodiments of any of the foregoing or other aspects and embodiments of the disclosure, the polyribonucleotide or modified polyribonucleotide comprises a primary sequence that is at least 85%, at least 90%, at least 92% or at least 95% identical (e.g., at least 95, 96, 97, 98, 99 or 100% identical) to one or more of **SEQ ID NO: 1 or 5 to 11** (e.g., to the sequence set forth in **SEQ ID NO: 1 or 5 to 11**). In some embodiments, the polyribonucleotide is a modified polyribonucleotide having a level and/or type of modification selected from any such level and/or type set forth herein. In certain embodiments, the percent identity of a polyribonucleotide is measured only with respect to the CCDC40 coding sequence-portion of **SEQ ID NO: 1 or 5 to 11** (e.g., UTRs, other non-coding sequence and GFP or epitope tags are not considered when calculating percent identity). In certain embodiments of any of the foregoing, such polyribonucleotide (or modified polyribonucleotide) encodes a functional CCDC40 protein.

In some embodiments of any of the foregoing or other aspects and embodiments of the disclosure, the polyribonucleotide or modified polyribonucleotide comprises a primary sequence that is at least 85%, at least 90%, at least 92%, or at least 95% identical (e.g., at least 95, 96, 97, 98, 99 or 100% identical) to one or more of **SEQ ID NO: 2 or 12 to 14** (e.g., to the sequence set forth in **SEQ ID NO: 2 or 12 to** 14). In some embodiments, the polyribonucleotide is a modified polyribonucleotide having a level and/or type of modification selected from any such level and/or type set forth herein. In certain embodiments, the percent identity of a polyribonucleotide is measured only with respect to the CCDC39 coding sequence-portion of **SEQ ID NO: 2 or 12 to 14** (e.g., UTRs, other non-coding sequence and GFP tags or epitope tags are not considered when calculating percent identity). In certain embodiments of any of the foregoing, such polyribonucleotide (or modified polyribonucleotide) encodes a functional CCDC39 protein.

In some embodiments of any of the foregoing or other aspects and embodiments of the disclosure, the polyribonucleotide or modified polyribonucleotide comprises a primary sequence that is at least 85%, at least 90%, at least 92%, or at least 95% identical (e.g., at least 95, 96, 97, 98, 99 or 100% identical) to **SEQ ID NO: 4** (e.g., to the sequence set forth in **SEQ ID NO: 4**). In some embodiments, the polyribonucleotide is a modified polyribonucleotide having a level and/or type of modification selected from any such level and/or type set forth herein. In certain embodiments, the percent identity of a polyribonucleotide is measured only with respect to the MCIDAS coding sequence-portion of **SEQ ID NO: 4** (e.g., UTRs, other non-coding sequence and GFP or epitope tags are not considered when calculating percent identity). In certain embodiments of any of the foregoing, such polyribonucleotide (or modified polyribonucleotide) encodes a functional MCIDAS protein.

In one embodiment of the method according to the present invention, the ciliopathy mentioned in step (a) is associated with a defect in a coiled-coil domain containing 40 (CCDC40; cf. e.g. NCBI Reference Sequences NM_017950.4 and NP_060420.2 for human mRNA and protein CCDC40 sequence, respectively) protein or with a defect in a coiled-coil domain containing 39 (CCDC39; cf. e.g. NCBI Reference Sequences NM_181426.2 and NP_852091.1 for human mRNA and protein CCDC39 sequence, respectively) protein.

CCDC40 and CCDC39 build a complex that is located between radial spokes and A-tubuli. Defect versions of the CCDC40 or CCDC39 protein can be caused by mutations in the *CCDC40* gene or in the *CCDC39* gene such as insertions, deletions, nonsense and splice site mutations. In particular, a deletion of position 248 and a TGT insertion between positions 2824 and 2825 in the DNA sequence encoding the CCDC40 protein appear to be quite frequent. Defect versions of CCDC40 or CCDC39 give rise to defects in structures of the axoneme such as absent or eccentric central pairs, abnormal radial spokes and nexin links, an abnormal assembly of the dynein regulatory complex, and/or a reduction of inner dynein arms. Hence, the polyribonucleotide comprised in the pharmaceutical composition according to the present invention is preferably an mRNA that can be translated into a functional version of CCDC40 and or of CCDC39. Further information on proteins involved in ciliogenesis as well as genes and molecular pathways associated with ciliopathies can be found e.g. in the review article of Reiter and Leroux (Reiter and Leroux, 2017, Nat Rev Mol Cell Biol,18(9):533-547).

Herein a subject suffering from a ciliopathy, may also be referred to as a patient. Patients may show abnormal ciliary structure and/or function, and/or biogenesis defects that result in retention of mucus and bacteria in the respiratory tract. The diagnosis of a ciliopathy for a given subject may be based for example on clinical findings, molecular analyses and/or ciliary ultrastructural analyses of a biopsy of said subject as e.g. also reviewed in Goutaki et al. (Goutaki et al., 2016, Eur Respir J, 48(4):1081-1095).

In the context of the present invention, the term "polyribonucleotide" refers to a single-stranded sequence built up of adenosine, guanosine, cytidine, and/or uridine residues (in modified or unmodified form, see below). Herein, the term "polyribonucleotide encoding a protein" refers to a polyribonucleotide which contains a coding region which encodes a protein, i.e. which can be translated into a sequence of amino acids. Thus, in the context of the present invention the term "polyribonucleotide encoding a protein" preferably refers to an mRNA, wherein an mRNA should be understood to mean any polyribonucleotide molecule which, if it comes into the cell, is suitable for the expression of a protein or is translatable into a protein.

Herein, the term "protein" encompasses any kind of amino acid sequence, i.e. chains of two or more amino acids which are each linked via peptide bonds. The term "protein" used in this context refers to any amino acid sequence of interest. Preferably, the encoded amino acid sequence is at least 5 amino acids long, more preferably at least 10 amino acids, even more preferably at least 50, 100, 200 or 500 amino acids. Thus, the term "protein" covers short peptides as well as polypeptides. As regards the function of the encoded protein, there is no limitation except that a defect variant of the protein is associated with a ciliopathy. Herein, the term "associated with" is intended to encompass the terms "causing", "being involved in" and/or "enhancing".

Herein, the term "a protein the defect of which" refers to a "defect protein" or "defect version of a protein" and thus, to a version of a protein with altered function compared to a functional version of said protein. However, the term may also encompass a version of said protein with a complete or partial lack of synthesis and thus, availability in the cell. In any case, the defect of the protein version results in a version of said protein that cannot fulfill the protein's native function.

A version of a protein that fulfils its native function is referred to as "functional protein" or "functional version of a protein" herein, and is encoded by the same DNA sequence as the respective defect protein, but without the defect causing change in the DNA sequence and thus, without a mutation in the DNA sequence.

Hence, in the context of the present invention, the "functional protein" is preferably a building block of an A-tubulus, a B-tubulus, or a nexin-dynein regulatory complex, or a radial spoke, an inner dynein arm, and/or an outer dynein arm.

Thus, the term "the polyribonucleotide encodes a functional version of a protein a defect of which is associated with said ciliopathy" preferably refers to an mRNA that encodes a functional protein, which is involved in the structural organization of a ciliar axoneme, an axoneme anchoring structure or a basal body and fulfills its native function. Thus, the polyribonucleotide analyzed in the method according to the present invention preferably refers to an mRNA that encodes a functional protein, the presence of which in the cell of a subject suffering of a ciliopathy is needed or beneficial to moderate or prevent a manifestation of said ciliopathy that is associated with a defect of said protein as encoded by the DNA sequence of the cell or to alleviate the associated symptoms.

In addition, the polyribonucleotide analysed in the method according to the present invention may also comprise further functional regions and/or 3' or 5' non-coding regions. The 3' and/or 5' non-coding regions can be sequences which naturally flank the encoded protein or artificial sequences which contribute to the stabilization and/or regulation of said polyribonucleotide. Suitable sequences may be identified and investigated by routine experiments. Further, said polyribonucleotide can also have further functional regions and may be combined with regulatory elements and target sequences of micro-RNAs for example for spatial and temporal control the activity of the desired polyribonucleotide comprising a sequence which encodes a protein, i.e. for example with respect to specific cells or cell types and/or developmental stages or specific time frames.

The polyribonucleotide employed int the method according to the present invention may comprise a partly or fully codon optimized sequence derived from the natural sequence to be used. Codon optimization refers to a technique which is applied to maximize protein expression by increasing the translational efficiency of the respective polyribonucleotide as in some cases codons exist that are preferentially used by some species for a given amino acid. Further, said polyribonucleotide might comprise further modifications to adjust and/or extend the duration of action. Said polyribonucleotide might also contain an m7GpppG cap, an internal ribosome entry site (IRES) and/or a polyA tail at the 3' end and/or additional sequences for promoting translation.

In some embodiments of the present invention the polyribonucleotide employed in the method according to the present invention may contain unmodified and modified nucleotides. The term "unmodified nucleotide" used herein refers to A, C, G and U nucleotides. The term "modified nucleotide" used herein refers to any naturally occurring or non-naturally occurring isomers of A, C, G and U nucleotides as well as to any naturally occurring or naturally occurring analogs, alternative or modified nucleotide or isomer thereof having for example chemical modifications or substituted residues. Modified nucleotides can have a base modification and/or a sugar modification. Modified nucleotides can also have phosphate group modifications, e.g., with respect to the 5' cap of an mRNA molecule. Modified nucleotides also include nucleotides that are synthesized post-transcriptionally by covalent modification of the nucleotides. Further, any suitable mixture of non-modified and modified nucleotides is possible. A non-limiting number of examples of modified nucleotides can be found in the literature (e.g. Cantara et al., Nucleic Acids Res, 2011, 39(lssue suppl_1):D195-D201; Helm and Alfonzo, Chem Biol, 2014, 21(2):174-185; Carell et al., Angew Chem Int Ed Engl, 2012, 51(29):7110-31) and some preferable modified nucleotides are mentioned exemplarily in the following based on their respective nucleoside residue:
1-methyladenosine, 2-methylthio-N6-hydroxynorvalyl carbamoyladenosine, 2-methyladenosine, 2'-O-ribosylphosphate adenosine, N6-methyl-N6-threonylcarbamoyladenosine, N6-acetyladenosine, N6-glycinylcarbamoyladenosine, N6-isopentenyladenosine, N6-methyladenosine, N6-threonylcarbamoyladenosine, N6,N6-dimethyladenosine, N6-(cis-hydroxyisopentenyl)adenosine, N6-hydroxynorvalylcarbamoyladenosine, 1,2'-O-dimethyladenosine, N6,2'-O-dimethyladenosine, 2'-O-methyladenosine, N6,N6,2'-O-trimethyladenosine, 2-methylthio-N6-(cis-hydroxyisopentenyl)adenosine, 2-methylthio-N6-methyladenosine, 2-methylthio-N6-isopentenyladenosine, 2-methylthio-N6-threonyl carbamoyladenosine, N6-2-methylthio-N6-threonyl carbamoyladenosine, 2-methylthio-N6-(cis-hydroxyisopentenyl)adenosine, 7-methyladenosine, 2-methylthio-adenosine, 2-methoxy-adenosine, 2'-amino-2'-deoxyadenosine, 2'-azido-2'-deoxyadenosine, 2'-fluoro-2'-deoxyadenosine, 2-aminopurine, 2,6-diaminopurine, 7-deaza-adenosine, 7-deaza-8-aza-adenosine, 7-deaza-2-aminopurine, 7-deaza-8-aza-2-aminopurine, 7-deaza-2,6-diaminopurine, 7-deaza-8-aza-2,6-diaminopurine; 2-thiocytidine, 3-methylcytidine, N4-acetylcytidine, 5-formylcytidine, N4-methylcytidine, 5-methylcytidine, 5-hydroxymethylcytidine, 5-hydroxycytidine, lysidine, N4-acetyl-2'-O-methylcytidine, 5-formyl-2'-O-methylcytidine, 5,2'-O-dimethylcytidine, 2-O-methylcytidine, N4,2'-O-dimethylcytidine, N4,N4,2'-O-trimethylcytidine, isocytidine, pseudocytidine, pseudoisocytidine, 2-thio-cytidine, 2'-methyl-2'-deoxycytidine, 2'-amino-2'-deoxycytidine, 2'-fluoro-2'-deoxycytidine, 5-iodocytidine, 5-bromocytidine, 2'-azido-2'-deoxycytidine, 2'-amino-2'-deoxycytidine, 2'-fluor-2'-deoxycytidine, 5-azacytidine, 3-methyl-cytidine, 1-methyl-pseudoisocytidine, pyrrolo-cytidine, pyrrolo-pseudoisocytidine, 2-thio-5-methyl-cytidine, 4-thio-pseudoisocytidine, 4-thio-l-methyl-pseudoisocytidine, 4-thio-l-methyl-1-deaza-pseudoisocytidine, 1-methyl-l-deaza-pseudoisocytidine, 2-methoxy-cytidine, 2-methoxy-5-methyl-cytidine, 4-methoxy-pseudoisocytidine, 4-methoxy-l-methyl-pseudoisocytidine, zebularine,5-aza-zebularine, 5-methyl-zebularine, 5-aza-2-thio-zebularine, 2-thio-zebularine; 1-methylguanosine, N2,7-dimethylguanosine, N2-methylguanosine, 2'-O-ribosylphosphate guanosine, 7-methylguanosine, hydroxywybutosine, 7-aminomethyl-7-deazaguanosine, 7-cyano-7-deazaguanosine, N2,N2-dimethylguanosine, N2,7,2'-O-trimethylguanosine, N2,2'-O-dimethylguanosine, 1,2'-O-dimethylguanosine, 2'-O-methylguanosine, N2,N2,2'-O-trimethylguanosine, N2, N2J-trimethylguanosine, Isoguanosine, 4-demethylwyosine, epoxyqueuosine, undermodified hydroxywybutosine, methylated undermodified hydroxywybutosine, isowyosine, peroxywybutosine, galactosyl-queuosine, mannosyl-queuosine, queuosine, archaeosine, wybutosine, methylwyosine, wyosine, 7-aminocarboxypropyldemethylwyosine, 7-aminocarboxypropylwyosine, 7-aminocarboxypropylwyosinemethylester, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, 8-oxo-guanosine, 7-methyl-8-oxo-guanosine, 1-methyl-6-thio-guanosine, N2-methyl-6-thio-guanosine, N2,N2-dimethyl-6-thio-guanosine, N1-methylguanosine, 2'-amino-3'-deoxyguanosine, 2'-azido-2'-deoxyguanosine, 2'-fluoro-2'-deoxyguanosine, 2-thiouridine, 3-(3-amino-3-carboxypropyl)uridine, 3-methyluridine, 4-thiouridine, 5-methyl-2-thiouridine, 5-methylaminomethyluridine, 5-carboxymethyluridine, 5-carboxymethylaminomethyluridine, 5-hydroxyuridine, 5-methyluridine, 5-taurinomethyluridine, 5-carbamoylmethyluridine, 5-(carboxyhydroxymethyl)uridine methyl ester, dihydrouridine, 5-methyldihydrouridine, 5-methylaminomethyl-2-thiouridine, 5-(carboxyhydroxymethyl)uridine, 5-(carboxyhydroxymethyl)-2'-O-methyluridine methyl ester, 5-(isopentenylaminomethyl)uridine, 5-(isopentenylaminomethyl)-2-thiouridine, 3,2'-O-dimethyluridine, 5-carboxymethylaminomethyl-2'-O-methyluridine, 5-carbamoylhydroxymethyluridine, 5-carbamoylmethyl-2'-O-methyluridine, 5-carbamoylmethyl-2-thiouridine, 5-methoxycarbonylmethyl-2'-O-methyluridine, 5-(isopentenylaminomethyl)-2'-O-methyluridine, 5,2'-O-dimethyluridine, 2'-O-methyluridine, 2'-O-methyl-2-thiorudine, 2-thio-2'-O-methyluridine, uridine 5-oxyacetic acid, 5-methoxycarbonylmethyluridine, uridine 5-oxyacetic acid methyl ester, 5-methoxyuridine, 5-aminomethyl-2-thiouridine, 5-carboxymethylaminomethyl-2-thiouridine, 5-methylaminomethyl-2-selenouridine, 5-methoxycarbonylmethyl-2-thiouridine, 5-taurinomethyl-2-thiouridine, pseudouridine, 1-methyl-3-(3-amino-3-carboxypropyl)pseudouridine, 1-methylpseudouridine, 3-methylpseudouridine, 2'-O-methylpseudouridine, 5-formyluridine, 5-aminomethyl-2-geranyluridine, 5-taurinomethyluridine, 5-iodouridine, 5-bromouridine, 2'-methyl-2'-deoxyuridine, 2'-amino-2'-deoxyuridine, 2'-azido-2'-deoxyuridine, 2'-fluoro-2'-deoxyuridine, inosine, 1-methylinosine, 1,2'-O-dimethylinosine, 2'-O-methylinosine, 5-aza-uridine, 2-thio-5-aza-uridine, 4-thio-pseudouridine, 2-thio-pseudouridine, 5-carboxymethyl-uridine, 1-carboxymethyl-pseudouridine, 5-propynyl-uridine, 1-propynyl-pseudouridine, 1-taurinomethyl-pseudouridine, 5-taurinomethyl-2-thiouridine, 1-taurinomethyl-4-thio-uridine, 5-methyl-uridine, 1-methyl-pseudouridine, 4-thio-l-methyl-pseudouridine, 2-thio-l-methyl-pseudouridine, 1-methyl-l-deaza-pseudouridine, 2-thio-1-methyl-l-deaza-pseudouridine, dihydropseudouridine, 2-thio-dihydrouridine, 2-thio-dihydropseudouridine, 2-methoxyuridine, 2-methoxy-4-thiouridine, 4-methoxy-pseudouridine, 4-methoxy-2-thio-pseudouridine, 1,2'-O-dimethyladenosine, 1,2'-O-dimethylguanosine, 1,2'-O-dimethylinosine, 2,8-dimethyladenosine, 2- methylthiomethylenethio-N6-isopentenyl-adenosine, 2-geranylthiouridine, 2-lysidine, 2-methylthio cyclic N6-threonylcarbamoyladenosine, 2-methylthio-N6-(cis-hydroxyisopentenyl) adenosine, 2-methylthio-N6-hydroxynorvalylcarbamoyladenosine, 2-methylthio-N6-threonylcarbamoyladenosine, 2-selenouridine, 2-thio-2'-O-methyluridine, 2'-O-methyladenosine, 2'-O-methylcytidine, 2'-O-methylguanosine, 2'-O-methylinosine, 2'-O-methylpseudouridine, 2'-O-methyluridine, 2'-O-methyluridine 5-oxyacetic acid methyl ester, 2'-O-ribosyladenosinephosphate, 2'-O-ribosylguanosinephosphate, 3,2'-O-dimethyluridine, 3-(3-amino-3-carboxypropyl)-5,6-dihydrouridine, 3-(3-amino-3-carboxypropyl)pseudouridine, 5,2'-O-dimethylcytidine, 5,2'-O-dimethyluridine, 5-(carboxyhydroxymethyl)-2'-O-methyluridine methyl ester, 55-(isopentenylaminomethyl)-2'-O-methyluridine, 5-aminomethyl-2-geranylthiouridine, 5-aminomethyl-2-selenouridine, 5-aminomethyluridine, 5-carbamoylmethyl-2'-O-methyluridine, 5-carboxyhydroxymethyluridine, 5-carboxymethyl-2-thiouridine, 5-carboxymethylaminomethyl-2-geranylthiouridine, 5-carboxymethylaminomethyl-2-selenouridine, 5-carboxymethylaminomethyl-2'-O-methyluridine, 5-cyanomethyluridine, 5-formyl-2'-O-methylcytidine, 5-methoxycarbonylmethyl-2'-O-methyluridine, 5-methylaminomethyl-2-geranylthiouridine, 7-aminocarboxypropyl-demethylwyosine, 7-methylguanosine, 8-methyladenosine, N2,2'-O-dimethylguanosine, N2,7,2'-O-trimethylguanosine, N2,7-dimethylguanosine, N2,N2,2'-O-trimethylguanosine, N2,N2,7-trimethylguanosine, N2,N2,7-trimethylguanosine , N4,2'-O-dimethylcytidine, N4,N4,2'-O-trimethylcytidine, N4,N4-dimethylcytidine, N4-acetyl-2'-O-methylcytidine, N6,2'-O-dimethyladenosine, N6,N6,2'-O-trimethyladenosine, N6-formyladenosine, N6-hydroxymethyladenosine, agmatidine, 2-methylthio cyclic N6-threonylcarbamoyladenosine, glutamyl-queuosine, guanosine added to any nucleotide, guanylylated 5' end , hydroxy-N6-threonylcarbamoyladenosine; most preferably pseudo-uridine, N1-methyl-pseudo-uridine, 2'-fluoro-2'-deoxycytidine, 5-iodocytidine, 5-methylcytidine, 2-thiouridine, 5-iodouridine and/or 5-methyl-uridine.

Furthermore, the term "modified nucleotide" comprises nucleotides containing isotopes such as deuterium. The term "isotope" refers to an element having the same number of protons but different number of neutrons resulting in different mass numbers. Thus, isotopes of hydrogen for example are not limited to deuterium, but include also tritium. Furthermore, the polyribonucleotide can also contain isotopes of other elements including for example carbon, oxygen, nitrogen and phosphor. It is also possible that modified nucleotides are deuterated or contain another isotope of hydrogen or of oxygen, carbon, nitrogen or phosphor.

The total number of modified nucleotide types in the polyribonucleotide can be 0, 1, 2, 3, or 4. Hence, in some embodiments, at least one nucleotide of one nucleotide type, e.g. at least one U nucleotide, can be a modified nucleotide. In some embodiments, at least one nucleotide of in total two nucleotide types, e.g. at least one U nucleotide and at least one C nucleotide, can be a modified nucleotide. In some embodiments, at least one nucleotide of in total three nucleotide types, e.g. at least one G nucleotide, at least one U nucleotide and at least one C nucleotide, can be a modified nucleotide. In some embodiments, at least one nucleotide of all four nucleotide types can be a modified nucleotide. In all these embodiments one or more nucleotides per nucleotide type can be modified, the percentage of said modified nucleotides of per nucleotide type being 0%, 2.5%, 5%, 7.5%, 10%, 15%, 20%, 25%, 30%, 35%, 40%, 45%, 50%, 55%, 60%, 65%, 70%, 75%, 80%, 85%, 90% or 100%. In some embodiments, the total percentage of modified nucleotides comprised in the mRNA molecules to be purified is 0%, 2.5%, 5 %, 7.5%, 10%, 15%, 20%, 25%, 30%, 35%, 40%, 45%, 50%, 55%, 60%, 65%, 70%, 75%, 80%, 85%, 90% or 100%.

Hence, the polyribonucleotide can for example be characterized in that 0.5 to 50%, preferably 5 to 50% of the U nucleotides and 5 to 50% of the C nucleotides are modified. Said modified U nucleotides are preferably 5-ioduridine and said modified C nucleotides are preferably 5-iodcytidine.

In some embodiments, the polyribonucleotide can be characterized in that 15 to 25% of the U nucleotides and 3 to 15%, preferably 5 to 15% of the C nucleotides are modified, wherein said modified U nucleotides are preferably 5-methyluridine and said modified C nucleotides are preferably 5-iodcytidine.

In some embodiments, the polyribonucleotide can be characterized in that 30 to 50% of the U nucleotides and 10 to 20% of the C nucleotides are modified, wherein said modified U nucleotides are preferably 5-ioduridine and said modified C nucleotides are preferably 5-iodcytidine.

In some embodiments, the polyribonucleotide can be characterized in that 30 to 50% of the U nucleotides and 5 to 15% of the C nucleotides are modified, wherein said modified U nucleotides are preferably 5-ioduridine and said modified C nucleotides are preferably 5-iodcytidine.

In some embodiments, the polyribonucleotide can be characterized in that 0.5 to 5% of the U nucleotides and 25 to 35% of the C nucleotides are modified, wherein said modified U nucleotides are preferably 2-thiouridine and said modified C nucleotides are preferably 5-methylcytidine.

The polyribonucleotide can for example also be characterized in that 50 to 100%, preferably 100%, of the U nucleotides are modified. Said modified U nucleotides are preferably N1-methyl-pseudo-uridine.

The polynucleotide may be transfected into cells by making use of compounds which facilitate transfection of cells with polyribonucleotides. Examples of such compounds are those disclosed in WO 2014/20723.

In particular, it is envisaged that this/these agent(s) or reagent(s) support(s) the delivering and/or introducing the RNA into the cell or tissue. The RNA to be delivered/introduced may also be coupled with (e.g. covalently bound to or complexed with) or uncoupled with (for example only admixed with) this/these agent(s( or reagent(s). Respective agents or reagents are known in the art (e.g. Tavernier, J Control Release 150(3) (2011), 238-47) and are, for example, selected from the group consisting of lipids and liposomes, micelles, polymers and dendrimers, among others. Particular examples of respective agents or reagents are GL67, EDMPC, DOTAP (l,2-dioleyl-3-trimethylammonium propane), DODAP (1,2-dioleyl- 3-dimethylammonium propane), DOTMA (I,2-di-0-octadecenyl-3-trimethylammonium propane), XTC (2,2-Dilinoleyl-4-dimethylaminoethyl-[I,3]-dioxolane) and MC3 (((6Z,9Z,28Z,31Z)-heptatriaconta-6,9,28,31-tetraen-19-yl 4-(dimethylamino)butanoate), ALNY- 100 ((3 aR,5s,6aS)-N,N-dimethyl-2,2- di((9Z, 12Z)-octadeca-9, 12-dienyl)tetrahydro-3aH-cyclopenta[d] [1 ,3]dioxol-5- amine)), NC98-5 (4,7, 13-tris(3-oxo-3-(undecylamino)propyl)-NI,N16-diundecyl- 4,7, 10, 13-tetraazahexadecane-l, 16-diamide), C12-200, DLin-KC2 -DMA, DODAP, I,2-distearyloxy-N,N-dimethyl-3- aminopropane or "DSDMA", I,2-dioleyloxy-N,N-dimethyl-3-aminopropane or "DODMA", I,2-dilinoleyloxy-N,N-dimethyl-3-aminopropane or "DLinDMA", 1,2- dilinolenyloxy-N,N-dimethyl-3-aminopropane or "DLenDMA", N-dioleyl-N,N- dimethylammonium chloride or "DODAC", N,N-distearyl-N,N-dimethylammonium bromide or "DDAB", N-(I,2-dimyristyloxyprop-3-yl)-N,N-dimethyl-N-hydroxy ethyl ammonium bromide or "DMRIE", 3-dimethylamino-2-(cholest-5-en-3-beta- oxybutan-4-oxy)-l-(ci s,cis-9, 12-octadecadienoxy)propane or "CLinDMA", 2-[5'- (cholest-5-en-3-beta-oxy)-3'-oxapentoxy)-3-dimethy I-I-(cis,cis-9', 1-2'- octadecadienoxy)propane or "CpLinDMA", N,N-dimethyl-3,4-dioleyloxybenzylamine or "DMOBA", I,2-N,N'-dioleylcarbamyl-3-dimethylaminopropane or "DOcarbDAP", 2,3-Dilinoleoyloxy-N,N-dimethylpropylamine or "DLinDAP", I,2-N,N'-Dilinoleylcarbamyl-3-dimethylaminopropane or "DLincarbDAP", I,2-Dilinoleoylcarbamyl-3-dimethylaminopropane or "DLinCDAP", 2,2-dilinoleyl-4-dimethylaminomethyl- [l,3]-dioxolane or "DLin-K-DMA", 2,2-dilinoleyl-4-dimethylaminoethyl-[l,3]- dioxolane or "DLin-K-XTC2-DMA", or mixtures thereof (Heyes, J Controlled Release 107 (2005), 276-287; Morrissey, Nat. Biotechnol. 23(8) (2005), 1003-1007; WO2005/121348). Further examples are DC- Chol (N,N-dimethyl-N-ethylcarboxamidocholesterol), I,4-bis(3-N-oleylamino- propyl)piperazine (Gao, Biochem. Biophys. Res. Comm. 179 (1991), 280; Wolf, et al BioTechniques 23 (1997), 139; U.S. Pat. No. 5,744,335). Further examples are LIPOFECTIN (DOTMA:DOPE) (Invitrogen, Carlsbad, Calif), LIPOFECTAMINE (DOSPA:DOPE) (Invitrogen), LIPOFECTAMINE2000. (Invitrogen), FUGENE, TRANSFECTAM (DOGS), and EFFECTENE. Further examples are modified and unmodified polyacrylates, polyalkycyanoacrylates, polylactide, polylactide-polyglycolide copolymers, polycaprolactones, dextran, albumin, gelatin, alginate, collagen, chitosan, cyclodextrins, polylysin, polyarginine, oligo/polyamines and polyethylenimine.

The agents or reagents may be oligomers, polymers or lipidoids. They may comprise oligo(alkylene amine) moieties like, for example, the characteristic oligo(alkylene amine) moieties as described in PCT/EP2014/063756. In particular, the agents or reagents may be the oligomers, polymers or lipidoids as described in PCT/EP2014/063756. One main characteristic of these particular agents or reagents is that they contain a following common structural entity of formula (I):

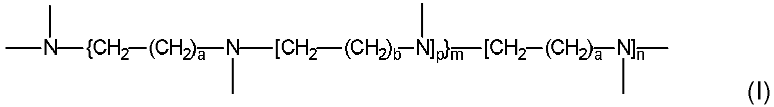

Such agents or reagents may be (a component comprising) an oligo(alkylene amine) selected from:
a) an oligomer or polymer comprising a plurality of groups of formula (II) as a side chain and/or as a terminal group: 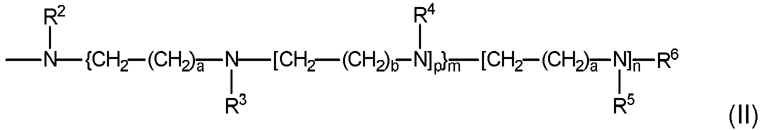 wherein the variables a, b, p, m, n and R² to R⁶ are defined as follows, independently for each group of formula (II) in a plurality of such groups:
   a is 1 and b is an integer of 2 to 4; or a is an integer of 2 to 4 and b is 1,
   p is 1 or 2,
   m is 1 or 2; n is 0 or 1 and m+n is ≥ 2; and
   R² to R⁵ are, independently of each other, selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷ or - CH₂-R⁷ wherein R⁷ is selected from C3-C18 alkyl or C3-C18 alkenyl having one C-C double bond; a protecting group for an amino group; and a poly(ethylene glycol) chain;
   R⁶ is selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R ⁷ or -CH₂-R⁷ wherein R⁷ is selected from C3-C18 alkyl or C3-C18 alkenyl having one C-C double bond; a protecting group for an amino group; -C(NH)-NH₂; a poly(ethylene glycol) chain; and a receptor ligand,
   and wherein one or more of the nitrogen atoms indicated in formula (II) may be protonated to provide a cationic group of formula (II);
b) an oligomer or polymer comprising a plurality of groups of formula (III) as repeating units: 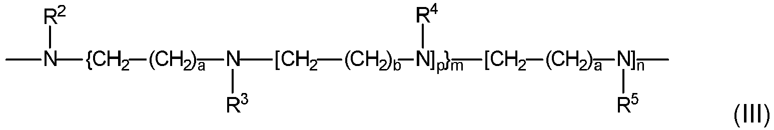 wherein the variables a, b, p, m, n and R² to R⁵ are defined as follows, independently for each group of formula (III) in a plurality of such groups:
   a is 1 and b is an integer of 2 to 4; or a is an integer of 2 to 4 and b is 1,
   p is 1 or 2,
   m is 1 or 2; n is 0 or 1 and m+n is ≥ 2; and
   R² to R⁵ are, independently of each other, selected from hydrogen; a group -CH₂-CH(OH)-R⁷, -CH(R⁷)-CH₂-OH, -CH₂-CH₂-(C=O)-O-R⁷ or -CH₂-CH₂-(C=O)-NH-R⁷ or - CH₂-R⁷ wherein R⁷ is selected from C3-C18 alkyl or C3-C18 alkenyl having one C-C double bond; a protecting group for an amino group; -C(NH)-NH₂; and a polyethylene glycol) chain;
   and wherein one or more of the nitrogen atoms indicated in formula (III) may be protonated to provide a cationic group of formula (III); and
c) a lipidoid having the structure of formula (IV): 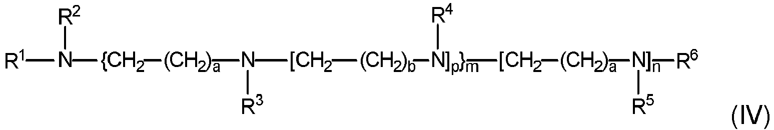 wherein the variables a, b, p, m, n and R¹ to R⁶ are defined as follows:
   a is 1 and b is an integer of 2 to 4; or a is an integer of 2 to 4 and b is 1,
   p is 1 or 2,
   m is 1 or 2; n is 0 or 1 and m+n is ≥ 2; and
   R¹ to R⁶ are independently of each other selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷ or - CH₂-R⁷ wherein R⁷ is selected from C3-C18 alkyl or C3-C18 alkenyl having one C-C double bond; a protecting group for an amino group; -C(NH)-NH₂; a polyethylene glycol) chain; and a receptor ligand; provided that at least two residues among R¹ to R⁶ are a group -CH₂-CH(OH)-R⁷, -CH(R⁷)-CH₂-OH, -CH₂-CH₂-(C=O)-O-R⁷, -CH₂-CH₂-(C=O)-NH-R⁷ or -CH₂-R⁷ wherein R⁷ is selected from C3-C18 alkyl or C3-C18 alkenyl having one C-C double bond;
   and wherein one or more of the nitrogen atoms indicated in formula (IV) may be protonated to provide a cationic lipidoid of formula (IV).

Preferably, such agents or reagents may be (a component comprising) an oligo(alkylene amine) selected from a) and b), wherein
a) is an oligomer or polymer comprising a plurality of groups of formula (IIa) as a side chain and/or as a terminal group:

   -NR²{CH₂-(CH₂)ₐ-NR³-CH₂-(CH₂)_{b}-NR⁴}ₘ-[CH₂-(CH₂)ₐ-NR⁵]ₙ-R⁶ (IIa),

   wherein a, b, m, n, and R² to R⁶ are defined as described above, and wherein one or more of the nitrogen atoms indicated in formula (IIa) may be protonated to provide a cationic oligomer or polymer structure; and
b) is an oligomer or polymer comprising a plurality of groups of formula (IIIa) as repeating units:

   -NR²{CH₂-(CH₂)ₐ-NR³-CH₂-(CH₂)_{b}-NR⁴}ₘ-[CH₂-(CH₂)ₐ-NR⁵]ₙ- (IIIa),

   wherein a, b, m, n, and R² to R⁵ are defined as described above, and wherein one or more of the nitrogen atoms indicated in formula (IIIa) may be protonated to provide a cationic oligomer or polymer structure.

Furthermore, such agents or reagents may be (a component comprising) an oligo(alkylene amine) selected from a lipidoid having the structure of formula (IVa):

R¹-NR²{CH₂-(CH₂)ₐ-NR³-CH₂-(CH₂)_{b}-NR⁴}ₘ-[CH₂-(CH₂)ₐ-NR⁵]ₙ-R⁶ (IVa),

wherein a, b, m, n, and R¹ to R⁶ are defined as described above, and wherein one or more of the nitrogen atoms indicated in formula (IVa) may be protonated to provide a cationic lipidoid.

As to such agents or reagents, in formula (II), (IIa), (III), (IIIa), (IV) or (IVa) n may be 1; or m may be 1 and n may be 1.

Further, as to such agents or reagents, in formula (II), (IIa), (III), (IIIa), (IV) or (IVa) a may be 1 and b may be 2; or a may be 2 and b may be 1.

In some embodiments, the oligomer, polymer or lipidoid may be a cationic (e.g. protonated) oligomer, polymer or lipidoid.

One non-limiting example of such an oligomer, polymer or lipidoid to be employed is a cationic lipid which was prepared by mixing 100mg N,N'-Bis(2-aminoethyl)-1,3-propanediamine (0.623mmol) with 575.07mg 1,2-Epoxydodecane (3.12mmol, (N-1) eq. where N is 2x amount of primary amine plus 1x amount secondary amine per oligo(alkylene amine)) and mixed for 96h at 80°C under constant shaking. Such an oligomer, polymer or lipidoid is also referred to as lipidoid "C12-(2-3-2)".

An agent or reagent, in particular a polymer, to be employed may be a copolymer, in particular a statistical copolymer. Such a copolymer may be a copolymer which contains a statistical/random arrangement of alkylene amine repeating units of alternating length (e.g. in contrast to a less preferred polymer which contains analogous arrangements of alkylene amine repeating units of non-alternating length). The copolymer may be a cationic (e.g. protonated) copolymer. Copolymers to be employed are known in the art and are, for example, described in EP 14 19 9439.2, WO 01/00708, EP-A1 1 198 489 and CA-A1 2,377,207.

In particular, the copolymer may be a statistical copolymer comprising a plurality of repeating units (a) independently selected from repeating units of the following formulae (a1) and (a2):

-CH₂-CH₂-NFH- (a1)

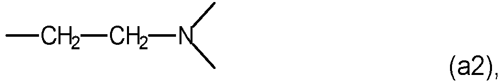

and
a plurality of repeating units (b) independently selected from repeating units of the following formulae (b1) to (b4):

-CH₂₋CH₂₋CH₂₋NH- (b1)

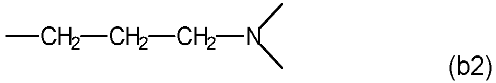

-CH₂-CH₂-CH₂-CH₂-NH- (b3)

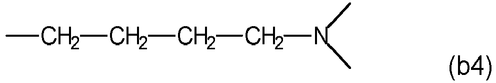

wherein the molar ratio of the sum of the repeating units (a) to the sum of the repeating units (b) lies within the range of 0.7/1.0 to 1.0/0.7, and
wherein one or more of the nitrogen atoms of the repeating units (a) and/or (b) contained in the copolymer may be protonated to provide a cationic copolymer.

The copolymer may be a statistical copolymer, wherein any repeating units (a) and any repeating units (b) are statistically distributed in the copolymer macromolecule. It is typically obtained from the copolymerization of a mixture of monomers yielding, during the polymerization reaction, the repeating units (a) with monomers yielding, during the polymerization reaction, the repeating units (b). Preferably, the copolymer is a random copolymer wherein any repeating units (a) and any repeating units (b) are randomly distributed in the polymer macromolecule.

Such a copolymer can be a linear, branched or dendritic copolymer. As will be understood by the skilled reader, a repeating unit of the formula (a1), (b1) or (b3) with two valencies (i.e. open bonds to neighboring units) leads to a propagation of the copolymer structure in a linear manner. Thus, a linear copolymer may comprise repeating units of formula (a1) and one or more types of the repeating units of formulae (b1) and (b3), but no repeating units of formula (a2), (b2) or (b4). As will be further understood, the presence of a repeating unit of formula (a2), (b2) or (b4) with three valencies provides a branching point in the copolymer structure. Thus, a branched copolymer comprises one or more types of the repeating units of formulae (a2), (b2) and (b4), and may further comprise one or more types of the repeating units of formulae (a1), (b1) and (b3).

Such a copolymer may comprise a plurality of repeating units (a) independently selected from repeating units of formulae (a1) and (a2) defined above, and a plurality of repeating units (b) independently selected from repeating units of formulae (b1) to (b4) defined above. Preferred are copolymers comprising a plurality of repeating units (a) independently selected from repeating units of formulae (a1) and (a2) defined above, and a plurality of repeating units (b) independently selected from repeating units of formulae (b1) and (b2) defined above.

Preferably, such a copolymer is a branched copolymer comprising one or more types of repeating units selected from repeating units (a2), (b2) and (b4), and which optionally further comprises one or more types of the repeating units of formulae (a1), (b1) and (b3), and in particular a copolymer which comprises repeating units of the formula (a2) and one or more type of the repeating units of formulae (b2) and (b4), and which optionally further comprises one or more types of the repeating units of formulae (a1), (b1) and (b3). In line with the above, a more preferred copolymer is thus a branched copolymer which comprises repeating units of the formula (a2) and repeating units of formula (b2), and which optionally further comprises one or more types of the repeating units of formulae (a1) and (b1).

In the copolymers, the total number of the repeating units (a) and repeating units (b) is typically 20 or more, preferably 50 or more and more preferably 100 or more. Typically, the total number of the repeating units (a) and repeating units (b) is 10,000 or less, preferably 5,000 or less, more preferably 1,000 or less.

Furthermore, it is preferred for the copolymers that the repeating units (a) and (b) account for 80 mol% or more, more preferably 90 mol% or more of all repeating units in the copolymer. Further preferred are copolymers wherein repeating units (a) selected from (a1) and (a2) and repeating units (b) selected from (b1) and (b2) account for 80 mol% or more, more preferably 90 mol% or more of all repeating units in the copolymer. It is most preferred that all of the repeating units in the copolymer are repeating units (a) or (b), in particular that all of the repeating units in the copolymer are repeating units (a) selected from (a1) and (a2) or repeating units (b) selected from (b1) and (b2).

The weight average molecular weight of the copolymer, as measured e.g. via size exclusion chromatography relative to linear polyethylene oxide) standards, generally ranges from 1,000 to 500,000 Da, preferably from 2,500 to 250,000 Da and more preferably 5,000-50,000 less.

The terminal groups of such a copolymer typically comprise one or more types of groups (c) independently selected from groups of the formulae (c1) to (c3) below, preferably from groups of the formulae (c1) and (c2) below:

-CH₂-CH₂-NH₂ (c1)

-CH₂-CH₂-CH₂-NH₂ (c2)

-CH₂-CH₂-CH₂-CH₂-NH₂ (c3).

Preferably, the terminal groups in the copolymer consist of one or more types of groups (c) independently selected from groups of the formulae (c1) to (c3) below, preferably from groups of the formulae (c1) and (c2). As will be understood by the skilled person, the number of terminal groups depends on the structure of the copolymer. While a linear copolymer has only two terminals, larger numbers of terminal groups are contained in a branched, in particular in a dendritic copolymer. As will be further understood, also one or more of the nitrogen atoms of the terminal groups (c) contained in the copolymer may be protonated to provide a cationic copolymer.

In the copolymer, the molar ratio of the sum of the repeating units (a) to the sum of the repeating units (b) lies within the range of 0.7/1.0 to 1.0/0.7, and preferably within the range of 0.8/1.0 to 1.0/0.8. This molar ratio can be determined, e.g., via NMR. It will thus be understood that the ratio is usually determined for a plurality of macromolecules of the copolymer, and typically indicates the overall ratio of the sum of repeating units (a) to the sum of repeating units (b) in the plurality of macromolecules.

As indicated above, one or more of the nitrogen atoms of the copolymer may be protonated to result in a copolymer in a cationic form, typically an oligocationic or polycationic form. It will be understood that the primary, secondary, or tertiary amino groups in the repeating units (a) or (b) or in the terminal groups (c) can act as proton acceptors, especially in water and aqueous solutions, including physiological fluids. Thus, such copolymers typically have an overall positive charge in an aqueous solution at a pH of below 7.5. An aqueous solution, as referred to herein, is a solution wherein the solvent comprises 50 % (vol./vol.) or more, preferably 80 or 90 % or more, and most preferably 100 % of water. Also, if the compositions are in contact with a physiological fluid having a pH of below 7.5, including e.g. blood and lung fluid, they typically contain repeating units (a) and (b) wherein the nitrogen atoms are protonated. The pKₐ values of the copolymers used in the compositions can be determined by acid-base titration using an automated pKₐ titrator. The net charge at a given pH value can then be calculated e.g. from the Henderson-Hasselbach equation. Any charge may be shared across several of the basic centres and cannot necessarily be attributed to a single point. Typically, in solutions at physiological pH, the copolymers used in the compositions comprise repeating units with amino groups in protonated state and repeating units with amino groups in unprotonated state.

However, as will be understood by the skilled reader, the copolymers as well as the compositions may also be provided as a dry salt form which contains the copolymer in a cationic form.

As will be further understood, counterions (anions) for the positive charges of protonated amino groups in compositions comprising the copolymer and nucleic acid, in particular RNA, preferably single-stranded RNA such as mRNA, are typically provided by anionic moieties contained in the nucleic acid. If the positively charged groups are present in excess compared to the anionic moieties in the nucleic acid, positive charges may be balanced by other anions, in particular those typically encountered in physiological fluids, such as Cl⁻ or HCO₃⁻.

In line with the above, a preferred copolymer is a random copolymer, wherein
80 mol% or more of all repeating units, more preferably all repeating units, are formed by
a plurality of repeating units (a) independently selected from repeating units of the following formulae (a1) and (a2):

   -CH₂-CH₂-NH- (a1)

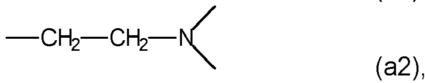

   and
a plurality of repeating units (b) independently selected from repeating units of the following formulae (b1) and (b2):

   -CH₂-CH₂-CH₂-NH- (b1) 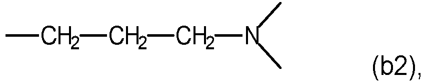
wherein the molar ratio of the sum of the repeating units (a) to the sum of the repeating units (b) lies within the range of 0.7/1.0 to 1.0/0.7, more preferably within the range of 0.8/1.0 to 1.0/0.8;
wherein the terminal groups of the copolymer are formed by
groups (c) independently selected from groups of the formulae (c1) and (c2):

   -CH₂-CH₂-NH₂ (c1)

   -CH₂-CH₂-CH₂-NH₂ (c2);

   and
wherein one or more of the nitrogen atoms of the repeating units (a) and/or (b) and/or of the terminal groups (c) contained in the copolymer may be protonated to provide a cationic copolymer. It is further preferred that the copolymer is a branched copolymer, comprising units (a2) and (b2), optionally together with units (a1) and/or (b1).

The copolymers can be conveniently prepared with procedures analogous to those known for the preparation of polyalkyleneimines, such as branched or linear polyethyleneimine (PEI). It will be understood that the monomers used for the production of the copolymers will have to be adjusted accordingly. Herein, it has been found that the monomers can be conveniently reacted in a quantitative manner, such that the ratio of the units (a) and (b) in the copolymer can be adjusted by adjusting the monomer ratio accordingly in the monomer mixture subjected to polymerization. While polyethyleneimine can be prepared e.g. via ring-opening polymerization of aziridine, the copolymers can be prepared via ring opening polymerization of a monomer mixture comprising or consisting of aziridine, azetidine and, where applicable pyrrolidine, or, in preferred embodiments, of aziridine and azetidine. It will be understood that the expression "where applicable" refers to the presence or absence of repeating units (b3) and (b4) or terminal groups (c3) which would be formed by the pyrrolidine. The ring opening polymerization of the non-substituted cyclic amines usually leads to branched copolymers. Linear copolymers can be prepared, e.g., via polymerization of suitable N-substituted aziridines, N-substituted azetidines and N-substituted pyrrolidines, or N-substituted aziridines and N-substituted azetidines, which may be followed e.g. by a hydrolytic cleavage of N-substituents attached to the resulting polyalkyleneimine chain, e.g. in analogy to the procedure published in Katrien F. Weyts, Eric J. Goethals, New synthesis of linear polyethyleneimine, Polymer Bulletin, 1988, 19(1):13-19. Dendrimers can be synthesized e.g. according to the method described in Yemul et al, Colloid and Polymer Science, 2008, 286(6-7):747-752, Synthesis and characterization of poly(ethylenimine) denrimers.

For the preparation of a dendrimer (or dendritic copolymer), synthetic strategies can be analogously applied which are known for the production of polyethyleneimine or polypropyleneamine dendrimers. Polypropylenimine dendrimers can be synthesized from acrylonitrile building blocks using a repetitive sequence of a Michael addition to a primary amine, followed by a heterogeneously catalyzed hydrogenation (Newkome and Shreiner Poly(amidoamine), polypropylenimine, and related dendrimers and dendrons possessing different 1→2 branching motifs: An overview of the divergent procedures. Polymer 49 (2008) 1-173; De Brabander-Van Den Berg et al. Large-scale production of polypropylenimine dendrimers, Macromolecular Symposia (1994) 77 (1) 51-62). Polyethylenimine dendrimers can be produced using a repetitive sequence of a Michael addition of a vinyl bromide building block to a primary amine followed by a conversion of alkylbromide to amine using a Gabriel amine synthesis method (Yemul & Imae, Synthesis and characterization of poly(ethyleneimine) dendrimers, Colloid Polym Sci (2008) 286:747-752). Hence the person skilled in the art will be able to produce not only dendrimers with strictly alternating layers of e.g. propylenimine and ethylenimine can be produced. Similarly dendrimer generations with layers comprising or consisting of random compositions of repeating units of formula (a2), (b2) and (b4) and preferably repeating units (a2) and (b2) can be generated.

The ring opening polymerization of aziridine and azetidine, or of aziridine, azetidine and pyrrolidine, can be carried out in solution, e.g. in water. The total monomer concentration is not particularly limited, typical concentrations range from 10% wt/wt to 80% wt/wt, preferably 30% wt/wt to 60% wt/wt. Typically, the polymerization is initiated by protons, such that it is preferred to add a Bronsted acid, in particular a mineral acid such as sulphuric acid to the reaction system. Small amounts of acid are generally sufficient, such as 0.001 to 0.01 equivalents, based on the total concentration of monomers. The reaction proceeds at convenient rates e.g. in the temperature range of 50 to 150°C, in particular 90 to 140°C. In these ranges, higher molecular weight copolymers are usually at higher temperatures, and lower molecular weight copolymers at lower temperatures.

In principle, a lipidoid is a preferred agent or reagent to be employed, in particular as compared to an oligomer and, more particular particular, to a polymer.

Further examples of the one or more agent(s) or one or more reagent(s) for delivering and/or introducing the RNA into a target cell or a target tissue are the liposomal transfection reagents (LTR'S) and magnetic particles (MPs) as described herein elsewhere.

One particular mode for delivering and/or introducing the RNA into target cells or target tissue is transfection. Hence, the RNA to be employed can be envisaged to be transfected (into (target) cells or tissue), to be delivered/administered via transfection and/or to be prepared for transfection. Means and methods for transfecting RNA are well known in the art and are, for example, described in Tavernier (loc. cit.), Yamamoto (Eur J Pharm Biopharm. 71(3) (2009), 484-9) and Kormann (Nat Biotechnol. 29(2) (2011), 154-7). Particular modes of transfection are lipofection, magnetofection or magnetolipofection.

Hence, the RNA to be employed may be prepared for lipofection, prepared to be transfected by lipofection, delivered/introduced via lipofection and/or administered via lipofection.

Thus, for transfection, the method according to the invention may (further) may further make use of at least one lipid or liposomal transfection reagent or enhancer (LTR; liposomal transfection reagent). The RNA to be employed may be comprised in, complexed with and/or delivered by the LTR. In particular, the RNA to be employed may be comprised in and/or delivered by (respective) lipofection complexes comprising the RNA and the LTR. The pharmaceutical composition may (further) comprise the lipofection complexes.

LTRs are known in the art and are, for example, distributed by OzBiosciences, Marseille, France. LTRs to be employed may be selected from the group consisting of the above-described agents or reagents for delivering and/or introducing the RNA into a target cell or a target tissue. For example, such LTRs may be lipids or lipidoids, preferably cationic lipids or cationic lipidoids, like the lipidoids as disclosed in PCT/EP2014/063756 (e.g. C12-(2-3-2), the lipids as disclosed in EP2285772 (e.g. Dogtor) and the lipopolyamines as disclosed in EP1003711 (e. g. DreamFect^{™} and DreamFect Gold^{™}). A particular LTR may be selected from the group consisting of
(i) C12-(2-3-2);
(ii) DreamFect^{™}, preferably DreamFect Gold^{™} (DF^{™}/DF-Gold^{™}; OzBiosciences, Marseille, France);
(iii) Dogtor (OzBiosciences, Marseille, France); and
(iv) Lipofectamine like, for example, Lipofectamine 2000 (Invitrogene, CA, USA).

In principle, Dogtor is a preferred, DreamFeet^{™} is a more preferred and DF-Gold^{™} and C12-(2-3-2) are even more preferred LTR(s).

LTRs like Dogtor are, for example, described in EP2285772. LTRs like DF^{™} or DF-Gold^{™} are, for example, described in EP1003711. In principle, the oligomers, polymers or lipidoids as disclosed in PCT/EP2014/063756, the particular cationic lipids as disclosed in EP2285772 and the particular lipopolyamines as disclosed in EP1003711 are preferred LTRs. LTRs like C12-(2-3-2) and DF-Gold^{™} are most preferred.

Non-limiting examples of lipofection complexes are DF-Gold^{™}/RNA lipoplexes and C12-(2-3-2)/RNA lipoplexes.

C12-(2-3-2) is a particularly preferred LTR having the structure shown in formula (V) (cf. Jarz 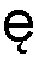 bińska et al., Angew Chem Int Ed Engl., 2016; 55(33):9591-5):

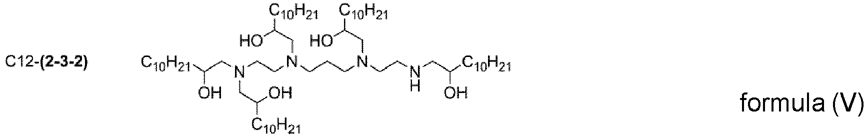

C12-(2-3-2) is preferably prepared as described e.g. in WO 2016/075154 A1, EP 3013964, and Jarz 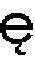 bińska et al. (Angew Chem Int Ed Engl., 2016;55(33):9591-5). The cationic lipidoid can be prepared by mixing N1-(2-aminoethyl)-N3-(2-((3,4-dimethoxybenzyl)amino)ethyl)propane-1,3-diamine (8.9 g, 1 eq., 28.67 mmol) with 1,2-Epoxydodecane (42.27, 8 eq., 229.4 mmol) and mixed for 24 h at 80 °C under constant shaking followed by purification and removal of the 3,4-dimethoxybenzyl protection group.

Different isomers, of C12-(2-3-2) can be used such as a racemate, an S-isomer, and/or an R-isomer. Preferably, C12-(2-3-2) is used as pure R-isomer and has the structure shown in formula (VI). For obtaining pure R-isomers of C12-(2-3-2) it can be prepared as described above for C12-(2-3-2) using the R-isomer of 1,2-Epxoydodecane for synthesis.

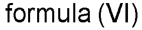

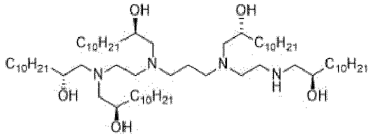

Hence, in preferred embodiments the the polyribonucleotide is transfected into cells by making use of a lipidoid having the structure shown in formula (V), preferably as shown in formula (VI).

A further particularly preferred LTR is a cationic lipidoid having formula (VII), herein also referred to as "dL_P" which can be synthesized via reaction of N,N'-Bis(2-aminoethyl)-1,3-propanediamine with N-Dodecylacrylamide using boric acid as catalyst. For the reaction, the mixture can be stirred at 100 °C under microwave irradiations.

Hence, in further preferred embodiments the cells are transfected with a polyribonucleotide using a lipidoid having having the structure shown in formula (VII).

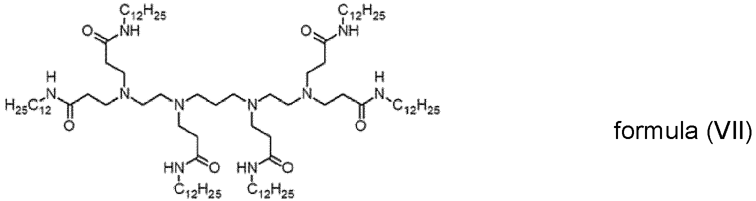

Furthermore, the use of a cationic lipidoid having formula (V), (VI) and/or (VII), preferably dL_P and/or C12-(2-3-2), more preferably dL_P and/or the R-isomer of C12-(2-3-2), comprised in a formulation as described in the following, is preferred. In particular, the herein described agents and reagents for delivering and/or introducing the RNA into a target cell or a target tissue and the herein described LTRs may be combined with one or more (e.g. two, three or four) further lipid(s) (like, for example, cholesterol, DPPC, DOPE and/or PEG-lipids (e.g. DMPE-PEG, DMG-PEG2000)). These further lipids may support the desired function of the agents/reagents and LTRs (support and/or increase the delivering and/or introducing of RNA into the cell or tissue and improve transfection efficiency, respectively) and function as respective "helper lipids". Particular examples of such "helper lipids" are cholesterol, DPPC, DOPE and/or PEG-lipids (e.g. DMPE-PEG, DMG-PEG (e.g. DMG-PEG2000). The further lipids (e.g. "helper lipids") may also be part(s) of the herein disclosed complexes/particles. The skilled person is readily in the position to prepare complexes/particles in accordance with the invention. Examples of further lipids (e.g. "helper lipids") are also known in the art. The skilled person is readily in the position to choose suitable further lipids (e.g. "helper lipids") and ratios of the agents/reagents/LTRs and the further lipids (e.g. "helper lipids"). Such ratios may be molar ratios of 1-4 : 1-5, 3-4 : 4-6, about 4 : about 5, about 4 : about 5.3 of agents/reagents/ LTRs : further lipid(s) (the more narrow ranges are preferred). For example, the agents/reagents/LTRs may be combined with three further lipids, like DPPC, cholesterol, and DMG-PEG2000, at a molar ratio of 8 : 5.3 : 4.4 : 0.9, respectively, or, more particular, 8 : 5.29 : 4.41 : 0.88, respectively.

Preferably, dL_P and/or C12-(2-3-2), more preferably dL_P and/or the R-isomer of C12-(2-3-2), is generated as described above and used with helper lipids DPPC and cholesterol and PEG-lipid DMG-PEG2000 at the molar ratios 8:5.29:4.41:0.88 for formulating lipoid particles.

A composition in which the R-isomer of C12-(2-3-2) (formula VI) is formulated with the lipids DPPC and cholesterol and PEG-lipid DMG-PEG2000 at the molar ratios 8:5.29:4.41 :0.88 is also referred herein as "LF92". A composition in which the dL_P (formula VII) is formulated with the lipids DPPC and cholesterol and PEG-lipid DMG-PEG2000 at the molar ratios 8:5.29:4.41:0.88 is also referred herein as "LF111".

As also exemplarily described e.g. in WO 2016/075154 A1, EP 3013964, and Zhang et al. (TERMIS, 2019, Tissue Engineering: Part A, Vol. 25, Numbers 1 and 2), dL_P and/or C12-(2-3-2) can be used as a non-viral vector, to make a stable lipoplex with mRNA molecules, based on electrostatic interaction between the positive amino groups of lipidoid and negative phosphate groups of mRNA molecules (Anderson, Human Gene Therapy 14, 2003, 191-202). To stabilized the lipoplex structure and reduce the leakage, dL_P and/or C12-(2-3-2), more preferably dL_P and/or the R-isomer of C12-(2-3-2), can be supplied with two helper lipids entitled 1,2-dipalmitoyl-sn-glycero-3-phosphocholine (DPPC) and cholesterol (Anderson, Drug Delivery 11, 2004, 33-39; Liang, Journal of Colliod and Interface Science 278, 2004, 53-62). At the end, 1,2-Dimyristoyl-sn-glycerol, methoxypolyethylene Glycol (DMG-PEG) 2kD (DMG-PEG2000) is to be added to the lipid mix to provide a PEGylated liposome. It is already well known that PEGylation improves the physico-chemical characteristic of liposome formulation by increasing water solubility, protecting from enzymatic degradation, and limiting immunogenic and antigenic reactions (Milla, Current Drug Metabolism 13, 2012, 105-119). Final N/P ratios for entire ethanoic lipid mixture are to be 8 : 5.29 : 4.41 : 0.88 standing for molar ratios of amino group of dL_P and/or C12-(2-3-2) / DPPC / cholesterol / DMG-PEG2000, respectively, to one phosphate group of mRNA molecule.

Hence, in preferred embodiments dL_P and/or C12-(2-3-2), preferably dL_P and/or the R-isomer of C12-(2-3-2), formulated with DPPC, cholesterol, and DMG-PEG2000, is used for transfecting cells with the polyribonucleotide.

Moreover, in particularly preferred embodiments dL_P and/or C12-(2-3-2), preferably dL_P and/or the R-isomer of C12-(2-3-2), formulated with DPPC, cholesterol, and DMG-PEG2000 with final N/P ratios for entire ethanoic lipid mixture of 8 : 5.29 : 4.41 : 0.88 for molar ratios of amino group of dL_P and/or C12-(2-3-2) / DPPC / cholesterol / DMG-PEG2000, respectively, to one phosphate group of mRNA molecule, is used for transfecting cells with the polyribonucleotide.

R-isomers of C12-(2-3-2) formulated with DPPC, cholesterol, and DMG-PEG2000 as stated above are also referred to as LF92 formulation.

Hence, in particularly preferred embodiments an LF92 formulation is used for transfecting cells with the polyribonucleotide.

dL_P formulated with DPPC, cholesterol, and DMG-PEG2000 as stated above are also referred to as LF111 formulation.

Hence, in particularly preferred embodiments an LF111 formulation is used for transfecting cells with the polyribonucleotide.

In a preferred embodiment an LF92 and/or LF111 formulation is/are used for transfecting cells with the polyribonucleotide. LF92 and/or LF111 refers to a carrier formulation that is used for cell transfection. This is advantageous as the use of LF92 and/or LF111 is associated with high transfection efficiencies and thus, ensures that the polyribonucleotide encoding a functional version of a protein a defect of which is associated with the ciliopathy can efficiently enter a cell, preferably an undifferentiated ciliary cell or basal cell, and thus restore ciliated cell function in the subject suffering of PCD.

In the following, polyribonucleotides are described which can be employed in the method according to the invention:
Polyribonucleotides encoding human CCDC40 are shown in any of **SEQ ID NO: 1 or 5 to 11**.

In some embodiments of any of the foregoing or other aspects and embodiments of the disclosure, the polyribonucleotide or modified polyribonucleotide encodes human CCDC40 (e.g., functional human CCDC40) and comprises a primary sequence that is at least 85%, at least 90%, at least 92%, or at least 95% identical (e.g., at least 95, 96, 97, 98, 99 or 100% identical) to one or more of **SEQ ID NO: 1 or 5 to 11** (e.g., to the sequence set forth in **SEQ ID NO: 1 or 5 to 11**).

In certain embodiments, said polyribonucleotide contains at its 5' end a part of the T7 promoter, Ethris' minimal 5' untranslated region (UTR), a CYBA 5' UTR, a Kozak element followed by a codon optimized sequence encoding a functional version of a human CCDC40 protein, a CYBA 3' UTR, and a poly(A) tail (cf. e.g. **SEQ ID NO: 1**). In certain embodiments, said polyribonucleotide contains at its 5' end a part of the T7 promoter, Ethris' minimal 5' untranslated region (UTR), a Kozak element followed by a codon optimized sequence encoding a functional version of a human CCDC40 protein, and a poly(A) tail (cf. e.g. **SEQ ID NO: 5**).

In certain embodiments, said polyribonucleotide contains at its 5' end a part of the T7 promoter, Ethris' minimal 5' untranslated region (UTR), an additional U nucleotide, a TISU element followed by a codon optimized sequence encoding a functional version of a human CCDC40 protein, and a poly(A) tail (cf. e.g. **SEQ ID NO: 6**).

In certain embodiments, said polyribonucleotide contains at its 5' end a part of the T7 promoter, Ethris' minimal 5' untranslated region (UTR), a hAg 5' UTR, a Kozak element followed by a codon optimized sequence encoding a functional version of a human CCDC40 protein, and a poly(A) tail (cf. e.g. **SEQ ID NO: 7**).

In certain embodiments, said polyribonucleotide contains at its 5' end a part of the T7 promoter, Ethris' minimal 5' untranslated region (UTR), a human CMV IE9 5' UTR, a Kozak element followed by a codon optimized sequence encoding a functional version of a human CCDC40 protein, a human Growth hormone 3' UTR, and a poly(A) tail (cf. e.g. **SEQ ID NO: 8**).

In certain embodiments, said polyribonucleotide contains at its 5' end a part of the T7 promoter, Ethris' minimal 5' untranslated region (UTR), a Kozak element, an EGFP encoding sequence, a G4S spacer followed by a codon optimized sequence encoding a functional version of a human CCDC40 protein, and a poly(A) tail (cf. e.g.

### SEQ ID NO: 9).

In certain embodiments, said polyribonucleotide contains at its 5' end a part of the T7 promoter, Ethris' minimal 5' untranslated region (UTR), a Kozak element followed by a codon optimized sequence encoding a functional version of a human CCDC40 protein, a G4S spacer, an EGFP encoding sequence, and a poly(A) tail (cf. e.g. **SEQ ID NO: 10**).

In certain embodiments, said polyribonucleotide contains at its 5' end a part of the T7 promoter, Ethris' minimal 5' untranslated region (UTR), a CYBA 5' UTR, a Kozak element followed by a HA tag, a G4S spacer, a codon optimized sequence encoding a functional version of a human CCDC40 protein, a T2A peptide, a sequence encoding tdTomato, a CYBA 3' UTR, and a poly(A) tail (cf. e.g. **SEQ ID NO: 11**).

In some embodiments, the polyribonucleotide is a modified polyribonucleotide having a level and/or type of modification selected from any such level and/or type set forth herein.

In certain embodiments, the percent identity of a polyribonucleotide is measured only with respect to the CCDC40 coding sequence-portion of **SEQ ID NO: 1 or 5 to 11** (e.g., UTRs, other non-coding sequence and GFP or epitope tags are not considered when calculating percent identity, and the polyribonucleotide may or may not contain such regions).

In certain embodiments of any of the foregoing, such polyribonucleotide (or modified polyribonucleotide) encodes a functional CCDC40 protein.

Polyribonucleotides encoding human CCDC39 are shown in any of **SEQ ID NO: 2 or 12 to 14**.

In some embodiments of any of the foregoing or other aspects and embodiments of the disclosure, the polyribonucleotide or modified polyribonucleotide encodes human CCDC39 and comprises a primary sequence that is at least 85%, at least 90%, at least 92% or at least 95% identical (e.g., at least 95, 96, 97, 98, 99 or 100% identical) to one or more of **SEQ ID NO: 2 or 12 to 14** (e.g., to the sequence set forth in **SEQ ID NO: 2 or 12 to 14**).

In certain embodiments, said polyribonucleotide contains at its 5' end a part of the T7 promoter, Ethris' minimal 5' UTR, a CYBA 5' UTR, a Kozak element followed by a codon optimized sequence encoding a functional version of a human CCDC39 protein, a CYBA 3' UTR, and a poly(A) tail (cf. e.g. **SEQ ID NO: 2**).

In certain embodiments, said polyribonucleotide contains at its 5' end a part of the T7 promoter, Ethris' minimal 5' UTR, a Kozak element followed by a codon optimized sequence encoding a functional version of a human CCDC39 protein, and a poly(A) tail (cf. e.g. **SEQ ID NO: 12**).

In certain embodiments, said polyribonucleotide contains at its 5' end a part of the T7 promoter, Ethris' minimal 5' UTR, an additional U nucleotide, a TISU element followed by a codon optimized sequence encoding a functional version of a human CCDC39 protein, and a poly(A) tail (cf. e.g. **SEQ ID NO: 13**).

In certain embodiments, said polyribonucleotide contains at its 5' end a part of the T7 promoter, Ethris' minimal 5' UTR, a SP30 as 5' UTR (i.e. a random sequence of 30 nucleotides), a Kozak element followed by a codon optimized sequence encoding a functional version of a human CCDC39 protein, and a poly(A) tail (cf. e.g. **SEQ ID NO: 14**).

In some embodiments, the polyribonucleotide is a modified polyribonucleotide having a level and/or type of modification selected from any such level and/or type set forth herein.

In certain embodiments, the percent identity of a polyribonucleotide is measured only with respect to the CCDC39 coding sequence-portion of **SEQ ID NO: 2 or 12 to 14** (e.g., UTRs, other non-coding sequence and GFP or epitope tags are not considered when calculating percent identity, and the polyribonucleotide may or may not contain such regions).

In certain embodiments of any of the foregoing, such polyribonucleotide (or modified polyribonucleotide) encodes a functional CCDC39 protein.

Polyribonucleotides encoding human MCIDAS are shown in **SEQ ID NO: 4.** In some embodiments of any of the foregoing or other aspects and embodiments of the disclosure, the polyribonucleotide or modified polyribonucleotide encodes human MCIDAS and comprises a primary sequence that is at least 85%, at least 90%, at least 92% or at least 95% identical (e.g., at least 95, 96, 97, 98, 99 or 100% identical) to **SEQ ID NO: 4** (e.g., to the sequence set forth in **SEQ ID NO: 4**). In certain embodiments, said polyribonucleotide contains at its 5' end a part of the T7 promoter, Ethris' minimal 5' UTR, a Kozak element followed by a codon optimized sequence encoding a functional version of a human MCIDAS protein, an optional 3' UTR that also functions as reverse primer binding site for PCR based template production as well as a poly(A) tail. In some embodiments, the polyribonucleotide is a modified polyribonucleotide having a level and/or type of modification selected from any such level and/or type set forth herein. In certain embodiments, the percent identity of a polyribonucleotide is measured only with respect to the MCIDAS coding sequence-portion of **SEQ ID NO: 4** (e.g., UTRs, other non-coding sequence and GFP or epitope tags are not considered when calculating percent identity, and the polyribonucleotide may or may not contain such regions). In certain embodiments of any of the foregoing, such polyribonucleotide (or modified polyribonucleotide) encodes a functional MCIDAS protein.

For purposes of determining percentage identity of a first sequence relative to a second sequence, an analog (e.g., methylcytidine) matches cytidine, etc. In certain embodiments, the term "primary sequence" may be used to refer to a polynucleotide sequence without regard to whether or the level of modification, such that a primary sequence identical to CUCUCUA would include that sequence regardless of whether any or all of the recited nucleotides are modified (e.g., analogs of any more or more of C, U and A may be present and would be considered the same primary sequence). In certain embodiments, percent identity is only determined by reference to the portion of a given listed sequence corresponding to the coding sequence for, for example, CCDC40 or CCDC39. While in other embodiments, the percent identity is determined by reference to both the coding sequence and one or more non-coding sequences. In certain embodiments, the percent identity is determinated across the entire length of a listed sequence (e.g., by reference to the entire length of a sequence listed in the sequence listing herein).

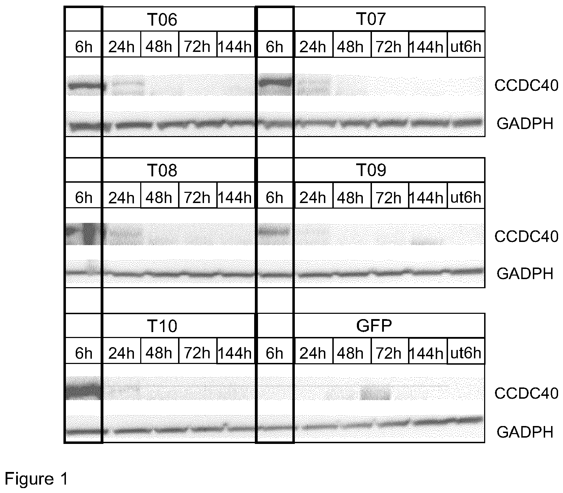

**Figure 1****:** Translation efficiency of CCDC40 mRNA. 2/1.4/0.3/0.2/0.05×10^6 HEK293 cells were seeded in 6-well plates. 24 h after seeding cells were transfected with different CCDC40 constructs (ETH031T06-T10, 2.5 µg/9.5 cm²) using Lipofectamine2000. Cells lysis was performed 6, 24, 48, 72 and 144 h after transfection. 50 µg of total protein lysate were analyzed with SDS-PAGE and Western Blot. CCDC40 was detected using Anti-CCDC40 Antibody (HPA022974) from Atlas Antibodies (1:2000). GAPDH served as a loading control. GFP served as a transfection control.

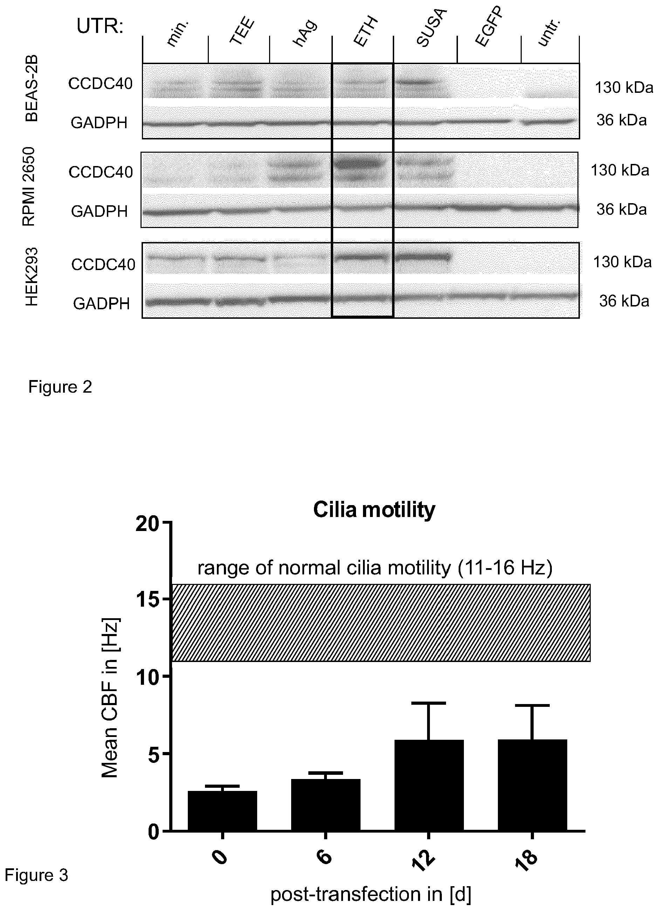

**Figure 2****:** Translation efficiency of CCDC40 mRNA constructs in BEAS-2B, RPMI2650, and HEK293 cells: 2×10^6 HEK293, 7.5×10^5 BEAS-2B and 5×10^5 RPMI 2650 cells were seeded in 6-well plates. 24 h after seeding cells were transfected with different CCDC40 constructs (2.5 µg/9.5 cm²) using Lipofectamine2000. Cells lysis was performed 6 h after transfection. Protein lysates were analyzed with SDS-PAGE and Western Blot (HEK293: 50 µg, RPMI 2650: 20 µg, BEAS-2B: 30 µg of total lysate). CCDC40 was detected using Anti-CCDC40 Antibody (HPA022974) from Atlas Antibodies (1:2000).
**Figure 3****:** High speed video microscopy (HSVM) results after LF92/CCDC40 transfection. Patient derived ALI cultures were transfected every other day with 3 µg LF92/CCDC40. Prior transfection and every 24 h after transfection, videos (20 per insert) were taken and CFB (ciliary beat frequency) was calculated using the SAVA (Sisson-Ammons video analysis) Software. Allover 16 transfections (1 month) were performed. Measurement was done at 37 °C using the 40x maginification. Calculated are the mean values of the ciliary beat frequency measurements (Whole Field Analysis, WFA).

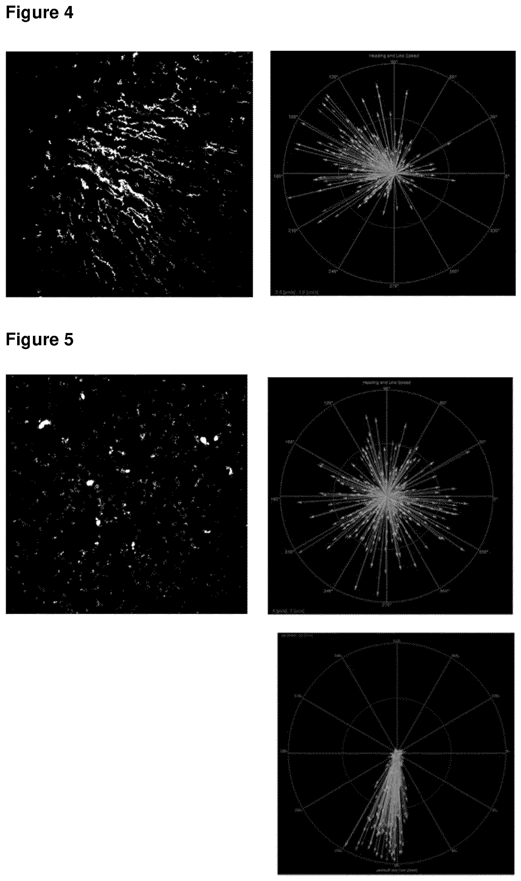

**Figure 4****:** Mucociliary clearance (MCC) shown as Z-Projection and Polargraph. CCDC40 patient ALI culture was transfected with CCDC40 mRNA/LF92 at a concentration of 3 µg/insert, 18 d upon air-lift. ALI cultures were maintained in Medium G during the experiment. Transfections (TFs) were performed once a week for 4 weeks (= 4x TF). Mucociliary Clearance (MCC) was measured using 0.5 µm fluorescent beads at 20x magnification. 30 s videos of different areas were taken and analyzed with the Polargraph software from Nikon one week after the last TF. One exemplary picture is shown.
**Figure 5****:** Mucociliary clearance (MCC) shown as Z-Projection (upper left) and Polargraph (upper right) for a tdTomato and Polargraph of a healthy (lower right) control. Upper row: Cells from patient with CCDC40 mutation in ALI culture were transfected with tdTomato mRNA/LF111 at a concentration of 3 µg/insert, 18 d upon air-lift. ALI cultures were maintained in Medium G during the experiment. Transfections (TFs) were performed once a week for 4 weeks (= 4x TF). Mucociliary Clearance (MCC) was measured using 0.5 µm fluorescent beads at 30x magnification. 20 s videos of different areas were taken and analyzed with the Polargraph software from Nikon one week after the last TF. One exemplary video was shown. Lower right: Particle tracking of healthy WT ALI culture. MCC was measured using 0.5 µm fluorescent beads at 20x magnification. 20 s videos of different areas were taken and analyzed with the Polargraph software from Nikon one week after the last TF. One exemplary picture and two analyzed ROIs are shown.

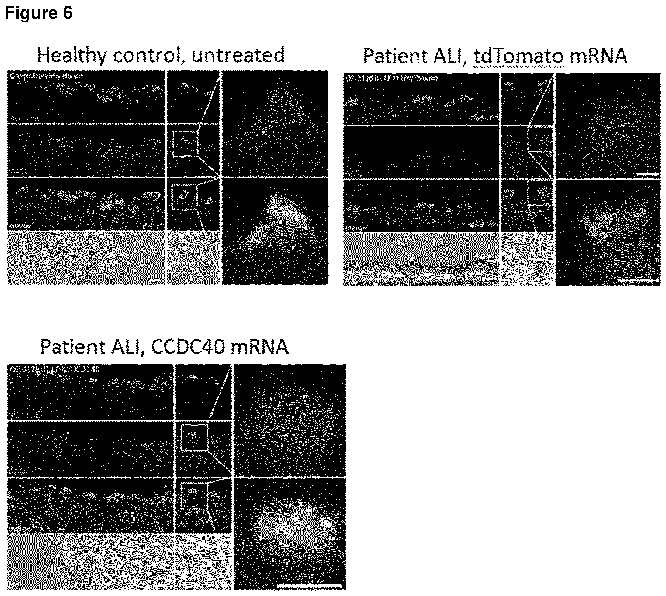

**Figure 6****:** Cells from patient with CCDC40 mutation in ALI culture were transfected either with tdTomato mRNA/LF111 or with CCDC40 mRNA/LF92 at a dose of 3 µg/insert, 18 d upon air-lift. ALI cultures were maintained in Medium G during the experiment. Transfections (TFs) were performed once a week for 4 weeks (= 4x TF). As a control, WT ALI from healthy nose brushes were used. Samples for cryo-embedding were prepared one week after the last TF. CCDC40 mRNA and tdTomato mRNA treated ALI membranes were cut at 20 µm and stained with anti-GAS8 (red), anti-acetylated Tubulin (green) and DAPI (blue). Pictures were taken with the confocal microscope. One exemplary image is shown. Scale bar represents 10 µm.

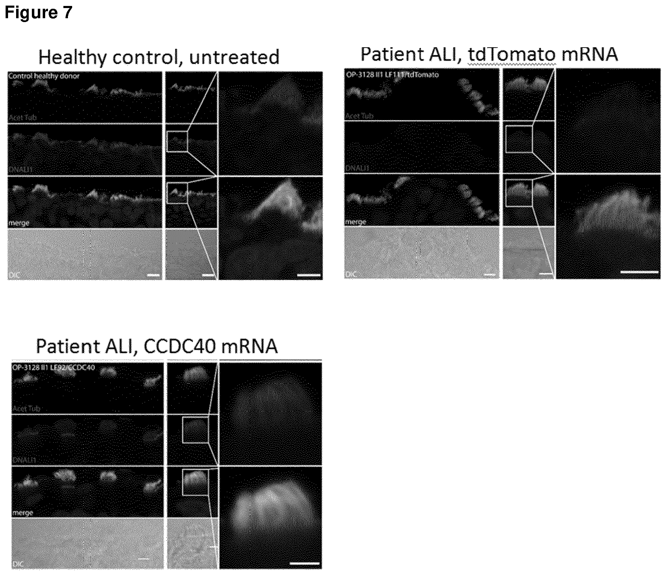

**Figure 7****:** Cells from patient with CCDC40 mutation in ALI culture were transfected either with tdTomato mRNA/LF111 or with CCDC40 mRNA/LF92 at a dose of 3 µg/insert, 18 d upon air-lift. ALI cultures were maintained in Medium G during the experiment. Transfections (TFs) were performed once a week for 4 weeks (= 4x TF). As a control, ALI from healthy controls were used. Samples for cryo-embedding were prepared one week after the last TF. CCDC40 mRNA and tdTomato mRNA treated ALI membranes were cut at 20 µm and stained with anti-DNALI1 (red), anti-acetylated Tubulin (green) and DAPI (blue). Pictures were taken with the confocal microscope. One exemplary image is shown. Scale bar represents 10 µm.

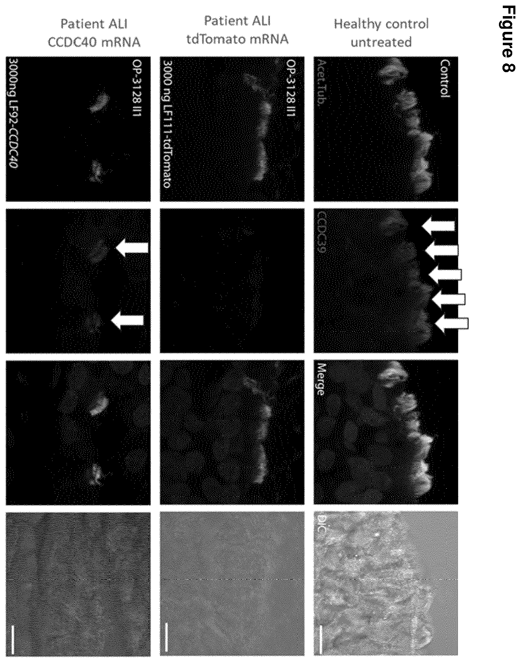

**Figure 8****:** Cells from patient with CCDC40 mutation in ALI culture were transfected either with tdTomato mRNA/LF111 or with CCDC40 mRNA/LF92 at a dose of 3 µg/insert, 18 d upon air-lift. ALI cultures were maintained in Medium G during the experiment. Transfections (TFs) were performed once a week for 4 weeks (= 4x TF). As a control, ALI from healthy controls were used. Samples for cryo-embedding were prepared one week after the last TF. CCDC40 mRNA and tdTomato mRNA treated ALI membranes were cut at 20 µm and stained with anti-CCDC39 (1:200, red), anti-acetylated Tubulin (1:10.000, green) and DAPI (blue). Pictures were taken with the confocal microscope. One exemplary image is shown. Magnification 40x.

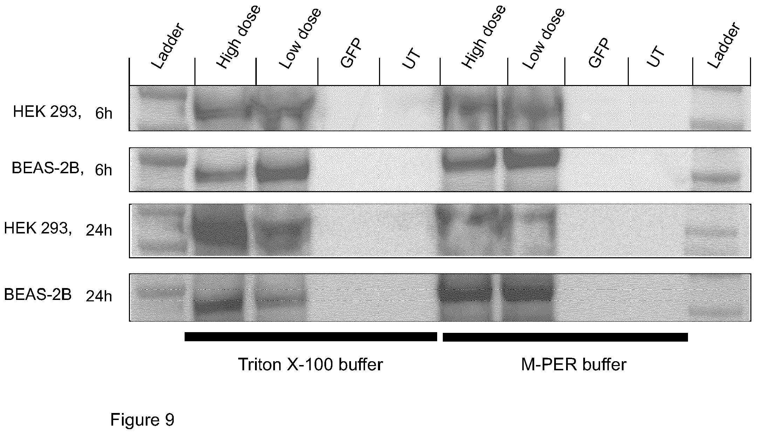

**Figure 9****:** CCDC39 protein (110 kDa) expression in HEK-293 & BEAS-2B, 6 h & 24 h after transfection. 1.4x10^5 HEK-293 cells and 3.5x10^5 BEAS-2B cells were seeded in 6-well plates and transfected with CCDC39-RNA (ETH047T02, minimal 5'UTR, Lipofectamine MessengerMax; Ratio 1:1.5). After 6h and 24h cells were lysed with M-PER and with Triton X-100 buffer. 50 µg of protein lysate were used for Western blot analysis. As a control, lysate of untransfected (UT) and EGFP transfected cells were used. High dose = 0.5 µg/cm², low dose = 0.25 µg/cm².
**Figure 10****:**
   CCDC39 protein (110 kDa) expression in untreated ALI cultures. Two inserts were extracted using the axonemal extraction protocol. 30, 10 and 5 µL of protein lysate were used for Western blot analysis. Active Area: Insert 39.9 = 71.25%, Insert 41.2 = 59.88%
**Figure 11****:**
   CCDC39 protein (110 kDa) expression in 16HBE14o- after treatment with proteasome inhibitor. 6.0×10^5 16HBE14o- cells were seeded in 6-well plates and transfected with CCDC39-RNA (Lipofectamine MessengerMax; Ratio 1:1.5). After 6 h cells were lysed with M-PER buffer. 20 µg of protein lysate were used for Western blot analysis. As a control, lysate of untransfected (UT) and EGFP transfected cells were used. High dose = 0.5 µg/cm², low dose = 0.25 µg/cm². RNA: ETH047T02: minimal 5'UTR, ETH047T03: TISU, ETH047T04: CYBA, ETH047T05: SP30.

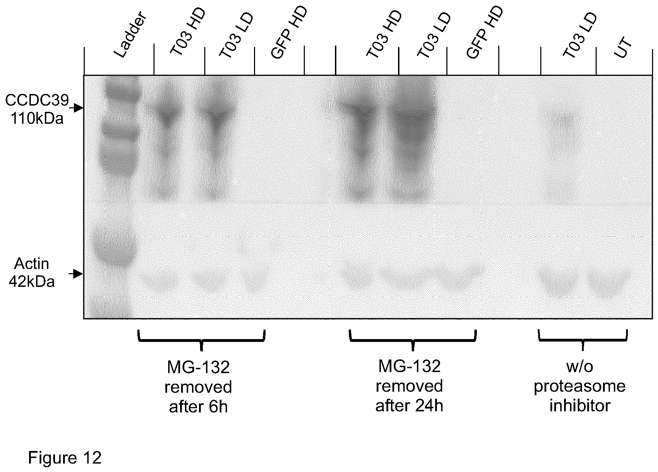

**Figure 12****:** CCDC39 protein (110 kDa) expression in 16HBE14o- after treatment with proteasome inhibitor. 6.0×10^5 16HBE14o- cells were seeded in 6-well plates and transfected with CCDC39-RNA (ETH047T03, TISU 5'UTR, Lipofectamine MessengerMax; Ratio 1:1.5). After 24 h cells were lysed with M-PER buffer. 20 µg of protein lysate were used for Western blot analysis. As a control, lysate of untransfected (UT) and EGFP transfected cells were used. High dose = 0.5 µg/cm², low dose = 0.25 µg/cm².

Other aspects and advantages of the invention will be described in the following examples, which are given for purposes of illustration and not by way of limitation. Each publication, patent, patent application or other document cited in this application is hereby incorporated by reference in its entirety.

### Examples

Methods and materials are described herein for use in the present disclosure; other, suitable methods and materials known in the art can also be used. The materials, methods, and examples are illustrative only and not intended to be limiting.

### I. Material and Methods

### Materials, Devices, Software, and Test system used

Materials are listed in **Table 4.**

**Table 4**

| **Substance/Consumable/Chemical** | **Supplier** | **Cat #** |
|---|---|---|
| DPBS (w/o Mg2+/Ca2+) | Thermo Fisher Scientific | 14190-169 |
| LHC-9 medium | Thermo Fisher Scientific | 12677019 |
| DMEM (1x) - GlutaMAX^{™} Supplement | Thermo Fisher Scientific | 21885 |
| BEAS-2B | ATCC | CRL 9609 |
| HEK 293 | DSMZ | ACC305 |
| Trypsin inhibitor | Thermo Fisher Scientific | R007100 |
| RPMI 2650 | DSMZ | ACC207) |
| Fibronectin | Merck Millipore | FC010 |
| Collagen | Corning Incorporated | 354236 |
| Pierce^{™} Bovine Serum Albumin | Thermo Fisher Scientific | 23209 |
| DPBS (with Mg2+/Ca2+), for air-brush | Thermo Fisher Scientific | 14040133 |
| Lipofectamine^{®} 2000 | Thermo Fisher Scientific | 11668027 |
| Corning^{®} Cell Scrapers | Corning Incorporated | CORN3010 |
| Protease inhibitor cOmplete | Sigma Aldrich | 11873580001 |
| Pierce^{™} BCA Protein Assay Kit | Thermo Fisher Scientific | 23225 |
| Bolt LDS Sample Buffer (4x) | Thermo Fisher Scientific | B0007 |
| Bolt Sample Reducing Agent (10x) | Thermo Fisher Scientific | B0009 |
| Precision Plus Protein^{™} Dual Color Standards | BIO-RAD | 161-0374 |
| Trans-Blot Turbo Transfer Pack Mini 0.2µm PVDF | BIO-RAD | 1704156 |
| Luminata Crescendo Western HRP Substrate | Merck Millipore | WBLUR0500 |
| GAPDH (D16H11)XP^{®} Rabbit mAb | Cell Signaling | 5174 |
| Anti-CCDC40 antibody | Sigma Aldrich | HPA022974 |
| Goat anti-rabbit IgG-HRP | Santa Cruz | sc-2004 |
| Celletta^{™}brush cell collector | Engelbrecht Medizin-und Labortechnik | 9100060 |
| Ultroser G | Pall Life Sciences | 15950 |
| Gelatine | Caelo | 01704140 |
| NaCl | Carl Roth | 0601.1 |
| TRIS | Carl Roth | AE15.3 |
| TRIS/HCl | Carl Roth | A9090.3 |
| EDTA | Carl Roth | X986.1 |
| NaOH (32%) | Carl Roth | T196.1 |
| FBS (submerse culture) | Thermo Fisher Scientific | 10500-064 |
| Penicillin-Streptomycin | Thermo Fisher Scientific | 15140-122 |
| Collagen I, rat tail | Gibco | A1048301 |
| Acetic acid 100 % | Carl Roth | 3738.1 |
| Corning Transwell | Costar | 3470 |
| Trypsin/EDTA 1x | Sigma Aldrich | T3924 |
| Collagenase type 4 | Worthington Biochemical Company | LS004188 |
| Antibiotics /Antimycotics 100 x | Thermo Scientific | 15240 |
| PneumaCult ALI medium | STEMCELL Technologies | 5001 |
| Sucrose (CAS# 57-50-1) | Sigma Aldrich | S1888 |
| Lactate Dehydrogenase Cytotoxicity Assay Kit | Pierce Biotechnology | 88954 |
| NucGreen^{™} Dead 488 ReadyProbes^{®} Reagent | Life Technologies | R37109 |
| NaCl 0.9 % Mini-Plasco^{®} connect (Isotonic Saline Solution) | BBraun | 9511711 |
| FBS (ALI culture) | Thermo Fisher Scientific | A3160801 |
| Shandon Cryomatrix^{™} Frozen Embedding Medium | Thermo Fisher Scientific | 6769006 |
| Superfrost Ultra Plus slides | Thermo Fisher Scientific | J1800AMNZ |
| Paraformaldehyde | Sigma Aldrich | P6148 |
| Goat serum | Abcam | Ab7481 |
| BSA IgG free protease free | Jackson Immunoresearch | 001-000-161 |
| Skim milk (Blotting grade) | Carl Roth | T145.2 |
| Hoechst33342 | Thermo Fisher Scientific | H3570 |
| monoclonal anti-acetylated alpha-tubulin antibody | Sigma Aldrich | T6793 |
| polyclonal anti-CCDC40 antibody | Proteintech | 25049-1-AP |
| polyclonal anti-CCDC39 antibody | Atlas antibodies | HPA035364 |
| Alexa Fluor 546 antibody | Thermo Fisher Scientific | A11035 |
| Alexa Fluor 488 antibody | Thermo Fisher Scientific | A11029 |
| Insulin | Sigma Aldrich | I9278 |
| Bovine Brain Extract | Lonza | CC-4098 |
| Transferrin | Sigma Aldrich | T8158 |
| Hydrocortisone | Sigma Aldrich | H4001 |
| 3,3',5-Triiodo-L-thyronine sodium sal | Sigma Aldrich | T6397 |
| Epinephrine | Sigma Aldrich | E4642 |
| Retinoic Acid | Sigma Aldrich | R2625 |
| Phosphorylethanolamine | Sigma Aldrich | P0503 |
| Ethanolamine | Sigma Aldrich | E0135 |
| DAPT | Tocris | 2634 |
| PBS (w/o Mg2+/Ca2+) | Thermo Fisher Scientific | 14190-94 |
| 10x PBS (with Mg2+/Ca2+) | Thermo Fisher Scientific | AM9625 |
| Triton-X 100 (ALI culture) | Sigma Aldrich | T8787 |
| Triton X-100 (submerse culture) | Sigma Aldrich | T9284 |
| DMEM/Ham's F-12 1:1 | Invitrogen | 11039 |
| Ultroser G | Pall Life Sciences | 15950-017 |
| Circular plasmid | Ethris | |
| *BstBI* | New England BioLabs | |
| Chloroform | Sigma Aldrich | 288306 |
| Ethanol | Sigma Aldrich | 34852-M |
| ARCA cap analogue | Jena Biosciences | |
| Cytidine-5'-triphosphate | Jena Biosciences | |
| 5-lodocytidine-5'-triphosphate | Jena Biosciences | |
| Uridine-5'-triphosphate | Jena Biosciences | |
| 5-Iodouridine-5'-triphosphate | Jena Biosciences | |
| Cytidine-5'-triphosphate | Jena Biosciences | |
| 5-Iodocytidine-5'-triphosphate | Jena Biosciences | |
| Uridine-5'-triphosphate | Jena Biosciences | |
| 5-Methyl-uridine-5'-triphosphate | Jena Biosciences | |
| DNasel | Thermo Fisher Scientific | |
| Ammonium acetate | Sigma | |
| Quick dephosphorylation Kit | New England BioLabs | |
| Poly(A) polymerase | New England BioLabs | |
| β-Mercaptoethanol | Sigma-Aldrich | M3148 |
| 6-Well Plate | Omnilab | CORN3506 |
| BCA Protein Assay Kit | Thermo Fisher Scientific | 23225 |
| Bolt Antioxidant | Thermo Fisher Scientific | BT0005 |
| Bolt^{™} 4-12% SDS-PAGE gel | Thermo Fisher Scientific | NW04120BOX |
| Bolt^{™} MES SDS Running buffer (20x) | Thermo Fisher Scientific | B0002 |
| cOmplete, EDTA-free Protease Inhibitor Cocktail | Sigma-Aldrich | 11873580001 |
| DNase I Solution (2500 U/mL) | Thermo Fisher Scientific | 90083 |
| DPBS | Life Technologies | 14040133 |
| DTT | Sigma Aldrich | 646563 |
| EDTA | Carl Roth | 8040.3 |
| Heat inactivated FBS | Thermo Fisher Scientific | 10500064 |
| HEPES | Carl Roth | HN78.2 |
| LHC-9 Medium | Thermo Fisher Scientific | 12680013 |
| Luminata Classico Western HRP substrate | Merck Millipore | WBLUC0500 |
| MEM GlutaMax | Thermo Fisher Scientific | 41090028 |
| M-PER^{™} buffer | Thermo Fisher Scientific | 78501 |
| MgSO4 | Carl Roth | T888.1 |
| Pen/Strep | Thermo Fisher Scientific | 15140122 |
| SDS 10% | Thermo Fisher Scientific | 24730020 |
| Sodium deoxycholate | Sigma-Aldrich | D6750-10G |
| SuperSignalTM West Festo | Thermo Fisher Scientific | 34095 |
| T175 Flasks | Corning/Omnilab | CORN431080 |
| T75 Flasks | Corning/Omnilab | CORN430641 |
| Trans-Blot^{®} Turbo^{™} Mini PVDF Transfer Packs | Bio-Rad | 1704156 |
| Triton X-100 | Sigma-Aldrich | T9284-100ML |
| TRYPSIN-EDTA ( 0.05%) | Thermo Fisher Scientific | 25300054 |
| Lipofectamine^{®} MessengerMAX^{™} | Thermo Fisher Scientific | LMRNA003 |
| Transfection Reagent | | |
| Aqua bidest. | Kerndl | 22501 |
| ALI membranes | Sigma-Aldrich | CLS3470 |
| Luminata Forte Western HRP substrate | Merck Millipore | WBLUF0500 |
| MMRB (cf. **Table 18**) | | |
| Liquid nitrogen | | |
| WFI | B Braun | 3703444 |
| T7 RNA Polymerase | Thermo Fisher Scientific | |
| Inorganic Pyrophosphatase | Thermo Fisher Scientific | |
| RNAse Inhibitor | Thermo Fisher Scientific | |
| HCL | Roth | 4625.2 |
| T175 Flasks (CORN431080) | Corning/Omnilab | |
| T75 Flasks (CORN430641) | Corning/Omnilab | |

Devices are listed in **Table 5.**

**Table 5**

| **Device** | **Supplier** |
|---|---|
| Tecan Infinite^{®} 200 PRO plate reader | Tecan |
| Thermomixer^{®} C | Eppendorf |
| Power PAC 300 | BIO-RAD |
| Trans-Blot^{®} Turbo^{™} Transfer System | BIO-RAD |
| ChemiDoc^{™} XRS System | BIO-RAD |
| TriStar2 Multimode Reader LB 942 | Berthold Technologies, Bad Wildbad, Germany |
| High-speed video microscopy | Ammons Engineering, Mt Morris, MI, USA |
| Nikon Eclipse Ti-S | Nikon MEA53300 |
| ELWD 40x S Plan Fluor o | Nikon, MEA48430 |
| Minitube SC300 heating system | Minitube International AG |
| Superfrost Ultra Plus slides | Thermo Fisher Scientific |
| 100 MWCO cut of filter | Sartorius |
| Cryostat Microm HM560 | Microm |
| ChemiDoc | Bio-Rad |
| Countess Cell Counting Device (C10281) | Thermo Fisher Scientific |
| Fluorescence microscope (DMi8) | Leica |
| Mini Gel Tank | Thermo Fisher Scientific |
| Trans-Blot^{®} Turbo^{™} Transfer System | Bio-Rad |
| Lysing Matrix A Tubes | MP Biomedicals |
| MP FastPrep-24 (HOM-1) | MP Biomedicals |
| 96-well black microplates (655090) | Greiner |

Software is listed in **Table 6.**

**Table 6**

| **Software** | **Provider** |
|---|---|
| Magellan^{™}- Data Analysis Software | Tecan |
| Image Lab^{™} | BIO-RAD |
| Sisson-Ammons Video Analysis (SAVA software) | Ammons Engineering, Mt Morris, MI, USA |
| Megaplus camera model ES 310 turbo | Redlake Inc., USA |
| NIS-Elements Basic Research | Nikon |
| Nikon NIS Elemets AR Software | Nikon |

The test system is listed in **Table 7.**

**Table 7**

| **Test System** | **Species** | **Strain** |
|---|---|---|
| HEK 293 | human primary embryonal kidney | DSMZ no.: ACC305 |
| RPMI 2650 | anaplastic squamous cell carcinoma | DSMZ no.: ACC207 |
| BEAS-2B | bronchial epithelium | ATCC no.: CRL 9609 |

### I. Submerse cell culture

### 1. Culturing

Cell lines were cultivated in a cell incubator under humidified atmosphere at 37 °C and 5 % CO₂ content. All reagents and solutions were heated to 37 °C in a water bath before usage.

Confluent cells (about 90 %) were first washed with 20 mL DPBS (w/o Mg2+/Ca2+) to remove dead cells. To detach the cells 2 mL of Trypsin/EDTA solution (0.05 %) was added per flask. Cells were then incubated at 37 °C until detachment of cells occurs. 8 mL of the respective medium was used to stop the Trypsin. Because LHC-9 medium was almost serum-free at least an equal volume of Trypsin inhibitor was added to the detached BEAS-2B cells to inactivate trypsinisation. The cell solution had to be centrifuged afterwards for 5 minutes (min) at 1100 revolutions per minute (rpm) to remove the trypsin inhibitor again. The pellet was then resolved in medium. For passaging, the cells were split in different ratios according to the next use.

### HEK 293 (DSMZ no.: ACC305)

This established cell line was from a human primary embryonal kidney transformed by adenovirus type 5 (AD 5). It was cultured in Dulbecco's Modified Eagle Medium (DMEM) - GlutaMAX^{™} Supplement with 10 % heat inactivated (h.i.) fetal calf serum (FBS) and 1 % penicillin/streptomycin (P/S). This cell line was split two times a week.

### RPMI 2650 (DSMZ no.: ACC207)

This cell line had its origin from the pleural effusion of a 52-year-old man with anaplastic squamous cell carcinoma of the nasal septum. It was chosen because of the epitheloid ciliated morphology and the similarity to the bronchiolar epithelium. It was also cultured in Dulbecco's Modified Eagle Medium (DMEM) - GlutaMAX^{™} Supplement with 10 % heat inactivated (h.i.) fetal calf serum (FBS) + 1 % penicillin/streptomycin (P/S) + 1x Non-essential-amino-acid solution (NEAA). This cell line was split 1-2 times a week.

### BEAS-2B (ATCC no.: CRL 9609)

BEAS-2B cells were derived from normal bronchial epithelium obtained from autopsy of non-cancerous individuals. Cells were then infected with a replication-defective SV40/adenovirus 12 hybrid and cloned. The used culture medium was LHC-9 with modification w/o additives. They were also split 1-2 times a week depending on the confluence.

The flasks used for BEAS-2B cells were coated with a mixture of 0.01 mg/mL fibronectin, 0.03 mg/mL collagen and 0.01 mg/mL bovine serum albumin dissolved in LHC-9. The mixture was added at a ratio of 0.2 mL per cm² surface area. Afterwards, incubation at 37 °C for at least 6 h was necessary. Prior to the addition of cells, the flasks were washed three times with Dulbecco's phosphate-buffered saline (DPBS) without Mg²⁺/Ca²⁺.

### 2. In vitro transcription

To generate templates for *in vitro* transcription, circular plasmids were linearized by restriction digestion with *BstBI* and further purified by chloroform ethanol precipitation.

mRNA was produced using a standard *in vitro* transcription mix (including indicated modified triphosphate nucleotides) containing T7 RNA polymerase, inorganic pyrophosphatase, and RNase inhibitor. Co-transcriptional capping was achieved by addition of an ARCA cap analogue. For *in vitro* transcription of chemically modified RNA, 7.5 % of Cytidine-5'-Triphosphate were replaced by 5-lodocytidine-5'-Triphosphate and 30 % Uridine-5'-Triphosphate were replaced by 5-lodouridine-5'-Triphosphate (Jena Biosciences), respectively (cf. e.g **SEQ ID NO: 1**). In another chemically modified RNA production setup 3 % of Cytidine-5'-Triphosphate were replaced by 5-lodocytidine-5'-Triphosphate and 15 % Uridine-5'-Triphosphate were replaced by 5-Methyl-Uridine-5'-Triphosphate (Jena Biosciences), respectively (cf. e.g **SEQ ID NO: 2**). Residual template DNA was digested using DNasel. Subsequently mRNA was purified by ammonium acetate precipitation followed by a washing step using 70 % ethanol.

Dephosphorylation of residual uncapped mRNA was carried out using a *Quick dephosphorylation Kit* followed by purification via ammonium acetate precipitation followed by a washing step using 70 % ethanol and ultrafiltration using a 100 MWCO cut of filter.

mRNA was further polyadenylated by using a poly(A) polymerase. Again mRNA was purified by ammonium acetate precipitation followed by a washing step using 70 % ethanol and ultrafiltration using a 100 MWCO cut of filter. Poly(A) length was determined by capillary gel electrophoresis to be between 100 and 250 nucleotides.

In particular, the CCDC40 mRNA constructs investigated in this experiment comprised an 5' ARCA cap and PPA and were the following: with Ethris' minimal UTR(ETH031T06; T06; **SEQ ID NO: 5**), with TISU 5' UTR but without 3' UTR (ETH031T07; T07; **SEQ ID NO: 6**), with hAg 5' UTR but without 3' UTR (ETH031T08; T08; **SEQ ID NO: 7**), with CYBA 5' and 3' UTR (ETH031T0; T09; **SEQ ID NO: 1**), and with 5' UTR from human CMV IE9 and 3' UTR from human Growth hormone (ETH031T0; T10; **SEQ ID NO: 8**).

### 3. Transfection

To provide at least 90 % confluency, cells were seeded in 6-well plates 24 h before transfection. Cell counts differed according to the time point of read-out and cell type (compare **Table 8**).

**Table 8**

| | **cells/well x10⁶** | | |
|---|---|---|---|
| **Time** | **HEK 293** | **RPMI 2650** | **BEAS-2B** |
| 6h | 2 | 0.6 | 0.75 |
| 24 h | 1.4 | 0.4 | 0.5 |
| 48 h | 1 | 0.3 | - |
| 72 h | 0.75 | 0.2 | - |
| 144 h | 0.125 | 0.05 | - |

Transfection was performed using different CCDC40 mRNA constructs as well as mRNA encoding for enhanced green fluorescent protein (eGFP) as a transfection control. Transfection was performed using Lipofectamine^{®} 2000 Prior to the transfection culture medium was exchanged to provide optimal conditions for the cells. The lipoplex reaction required an mRNA/WFI mix as well as a Lipofectamine^{®} 2000/medium mix. 125 µL of each mix was used with volume ratio of 1:2 from mRNA to Lipofectamine^{®} 2000 as the mRNAs showed a stock concentration of 1 µg/µl. Different mRNA concentrations were used and the mix was adjusted accordingly (see **Table 9** for an exemplary pipetting scheme).

**Table 9**

| **µg RNA/well** | **RNA in µL** | **+WFI in µL** | **Lipofectamine^{®} 2000 in µL** | **+serumfree medium in µL** |
|---|---|---|---|---|
| 5 | 5 | 120 | 10 | 115 |
| 2.5 | 2.5 | 122.5 | 5 | 120 |
| 1 | 1 | 124 | 2 | 123 |

After preparing both mixes the mRNA/WFI Mix was added to the Lipofectamine^{®}2000/Medium Mix and the solution incubated for ≥ 5 min before adding it dropwise to the seeded cells. To obtain a better viability of the cells, an additional medium change was performed 4 h after transfection.

### 4. CCDC40 Western blot

### a. Sampling for Western blot

For cell lysis the culture medium from transfection was removed and the cells were washed with DPBS (without Mg2+/Ca2+) and then scraped off using Corning^{®} Cell Scrapers. The cells were lysed with 100 µL of a Lysis-Buffer solution [10x TritonX-100 lysis buffer (250 mM Tris-HCI, 1 % TritonX-100, pH: 7.8) was adjusted to 1x in WFI and complemented with protease inhibitor cOmplete] before samples were frozen at -20 °C.

Total protein concentration was determined using Pierce^{™} BCA Protein Assay Kit (BCA assay). The assay was performed according to the manufacturers' protocol. Briefly, a 1:50 BCA Working Reagent (WR) dilution was prepared. 5 µL of each sample were transferred to a 96-well plate (flat bottom, transparent) and 200 µL WR were added per well. The last step was an incubation at 37 °C for 30 min. Results were measured on a Tecan Infinite^{®} 200 PRO plate reader using Magellan^{™}- Data Analysis Software.

### b. Preparation of NET-Gelatine

**Table 10**

| **Substance** | **Final concentration** |
|---|---|
| TRIS/HCI, pH 7.5 | 0.2 M |
| EDTA, pH 8.0 | 0.05 M |
| Triton-X100 | 0.5 % (v/v) |
| NaCl | 1.16 M |
| Gelatine | 25 g/L |

### c. SDS-PAGE and Western blot

To perform sodium dodecyl sulfate-polyacrylamide gel electrophoresis (SDS-PAGE) the lysates were mixed with 5 µL Bolt LDS Sample Buffer (4x) and 2 µL Bolt Sample Reducing Agent (10x) and then heated to 70 °C for 10 min and 350 rpm in the Thermomixer^{®} C. To enable comparison between the samples on one gel, the same amount of total protein was loaded. 7 µL of the marker (Precision Plus Protein^{™} Dual Color Standards) were filled into the first pocket of each gel, to enable the accurate protein size estimation afterwards. The gel tank was then connected to the power supply (Power PAC 300) for 30 min at 1 A. Subsequently the cassette was opened with a spatula and the gel was then moved to the blotting station.

The Transfer System (Trans-Blot^{®} Turbo^{™} Transfer System, 30 min, 25 V, 1 A) and suitable membranes (Trans-Blot Turbo Transfer Pack Mini 0.2 µm PVDF) were used for blotting according to the manufacturer's instructions. By scrolling a roller over the "sandwich", any bubbles which could interfere the protein transfer were eliminated. To block free binding sites, the membrane was put into an NET-gelatine and shaken for 60 min on a plate shaker. The gel was cut at the 75 kD band to detect CCDC40 and Glycerinaldehyd-3-phosphat-Dehydrogenase (GAPDH) at the same time. Antibodies were diluted with NET-gelatine (Anti-CCDC40 1:2000 in 6 mL, GAPDH 1:10000 in 10 mL NET-gelatine) and the membranes were incubated with their corresponding antibody on a plate shaker over night at 4 °C. GAPDH functions as a loading control. A list of employed antibodies was shown in **Table 11.** The next day the membrane was washed three times with NET-gelatine for 10 min to remove antibodies that were non-specifically bound or residual. The secondary antibody Goat anti-rabbit IgG-HRP was diluted 1:10,000 with NET-gelatine. After a 60 min incubation on a plate shaker at room temperature (RT), the membranes were washed again three times for 10 min with NET-gelatine. The horseradish peroxidase HRP conjugated secondary antibody could be detected via a molecular imager (ChemiDoc^{™} XRS System). Therefore, 5 mL of Luminata Crescendo Western HRP Substrate was added on the gel and incubated for 5 min. The last picture that was not yet overexposed and the colorimetric picture were then merged and illustrated with the Image Lab^{™} Software. In addition, the band intensities were determined with Image Lab^{™} afterwards.

**Table 11**

| **Name** | **Manufacturer** | **Usage** |
|---|---|---|
| GAPDH (D16H11)XP^{®} Rabbit mAb | Cell Signalling | Primary antibody |
| Anti-CCDC40 | SIGMA-ALDRICH | Primary antibody |
| Goat anti-rabbit IgG-HRP | SCBT | Secondary antibody |

### II. ALI culture

### 1. Sampling of Human Respiratory Epithelial Cells (hREC)

### a. Nasal brushing

RPMI medium was heated to room temperature and the brush was wet with sterile isotonic saline solution. A Celletta^{™}brush cell collector with protective tip was applied. After asking the patient to clean his nose, he had to sit in a chair with the head against a wall to hold the head. The brush was rubbed a few times rapidly against the medial and superior side of the inferior nasal meatus using rotatory and linear movements. When taking the brush out it was immediately put in a collecting tube (15 mL cornicle) with 5 ml pre-warmed RPMI medium. The brush was vigorously shaken within the tube for at least 40 times to detach the cells from the brush.

### b. Stationary culture

The tube was spun at 900 rpm for 5 min at room temperature. After discarding the supernatant, the pellet was resolved in UG medium [DMEM/Ham's F-12 1:1 with 5 mL sterile 100x anibiotics/mycotics and 10 mL sterile Ultroser G]. 5 mL medium were used for a T25 and 25 mL for a T75 flask.

Having transferred the cells to the cell culture flask, the cells were incubated at 37 °C in a humidified incubator with 5 % CO₂ over night. Cells should get attached after 20-24 h. UG medium was replaced 3 times a week (Monday, Wednesday and Friday).

### 2. Generation and transfection of ALI culture

### a. Coating of transwell inserts (0.33 cm²)

Following plates were employed for ALI culture: Corning Transwell with clear polyester-membrane (0.4 µm pores, 6.5 mm diameter of inserts, 24-well plates). Rat-tail collagen was diluted 1:5 in acetic acid and 250 µL were loaded on the apical side of each insert. After incubating the plates over night at room temperature, the remaining liquid was aspirated with a pasteur pipette and then the plate left to dry with an open lid for at least 5 min (sterile). Finally, inserts were washed twice with DPBS (w/o Ca²⁺/Mg²⁺) and then dried over night at room temperature (sterile). Inserts were stored at 4 °C for up to one month.

### b. Cell expansion of hREC as submerse culture (stationary culture)

Cells were fed every 2 to 3 days (Monday, Wednesday and Friday) with 15 mL pre-warmed UG medium. Care had to be taken not to add new medium directly onto the collagen layer to prevent damage of the collagen layer and detachment of the cells. As soon as cells were 80 % confluent or collagen layer began to detach, cells were transferred to ALI filters (approximately after 3 weeks in submerse culture).

### c. Preparation of Medium G

Different ALI media were tested, but the self-made Medium G was preferred.

This medium was prepared for one week but needed to be stored at 4 °C. Batches were frozen in aliquots based on upcoming experiment size. **Table 2** shows the composition of Medium G.

### d. Seeding and air-lift of hREC (ALI culture)

1. Aspirate UG medium, wash once with wash medium [DMEM/Ham's F-12 1:1 with 1x sterile antibiotics/antimycotics (AA)] or DPBS (w/o Ca²⁺/Mg²⁺)
2. In case of coated flasks with self-made rat-tail collagen:
   a. Dissolve collagen layer by adding 9 mL collagenase (1x) and incubate for 45 to 60 min at 37 °C
   b. Transfer cells into a 15 mL cornicle tube and centrifuge at 1000 rpm, room temperature for 5 min
   c. Wash cells with wash buffer (1000 rpm, room temperature, for 5 min)
   d. Add 2 mL Trypsin EDTA (1x) and pipette gently up and down to separate the cells, incubate cells at 37 °C and shake the cornicle until the cell clusters disintegrate (approximately 5 minutes)
   In case of coated flasks with collagen purchased from gibco:
   a. Add 3 ml Trypsin EDTA (1x) to the cell culture flask until the cells detach (approximately 5 min)
3. Stop trypsin by adding 0.5 mL FBS and 5 mL wash medium
4. Transfer cells into a 15 ml falcon and wash cells twice with wash medium (1000 rpm, room temperature, for 5 min)
5. Resuspend pellet in appropriate amount of ALI medium (1-2 mL, depends on pellet size) and count the cells using cell counter
6. Add 250 µL of the cell suspension (400,000 cells/mL) into each collagen coated transwell with 500 µL ALI medium in the basolateral side
7. Incubate cells at 37 °C in a humidified, 5 % CO₂ incubator over night Day 3-5: ALI-Growth
8. Check inserts under the microscope consistently
9. Feed cells with ALI medium every day until 80 -100 % confluence (3-4 days)
   ALI-Differentiation
10.Airlift cells when 80 -100 % confluency is reached
   a. Aspirate ALI medium from both, the apical and basolateral side
   b. Add 500 µL fresh ALI medium to the basolateral compartment
   c. Incubate cells at 37 °C in a humidified, 5 % CO₂ incubator
11. Wash cells with preheated wash medium (apical) once a week and feed cells with ALI medium every 2-3 days (or Monday - Wednesday - Friday)

### e. Transfection of hREC

The formulations (LF111-mRNA) were diluted in 2 % sucrose to a final concentration of 3 µg/insert (= 9 µg/cm²). To remove mucus, the inserts were washed with 200 µL DPBS (w/o Ca²⁺/Mg²⁺) on the apical side and incubated for 20 min at 37 °C (Epithelix ALI) or 200 µL DPBS (w/o Ca²⁺/Mg²⁺) on the apical side and incubated for 5 min at room temperature (hREC, UKM) prior transfection. Afterwards DPBS (w/o Ca²⁺/Mg²⁺) from the apical surface was removed by gentle aspiration without damaging the epithelium. Directly after the mucus wash, the cells were washed again with WFI (100 µL) to remove traces of DPBS (w/o Ca²⁺/Mg²⁺). 25 µL of the formulation was added on the apical side of the insert to allow cellular uptake. Removal of carrier was performed after 6 h of incubation, cells were washed once with 200 µL DPBS (w/o Ca²⁺/Mg²⁺) and culture was maintained as ALI without liquid on the apical side.

### 3. Read-out assays

Various read-out assays are employed. LDH measurement and NucGreen assay were used to investigate toxicity-related effects of transfection. Ciliary beat frequency measurements (CBF) were needed to examine the manner of ciliary beating with regard to beat frequency and percentage of ciliated area. If ciliary beating leads to a directed flow it can be studied using the Mucociliary Clearance (MCC) Assay. Immunofluorescence (IF) stainings for CCDC40, its heterodimer partner CCDC39 and the cilia marker acetylated alpha-tubulin were utilized to detect if, after treatment, CCDC40 protein or its partner CCDC39 can be detected in the cilia. In addition, proteins of the dynein regulator complex (DRC) like GAS8 and DNALI1 were stained. The DRC is anchored by CCDC40/CCDC39 and only present if CCDC40/CCDC39 are correctly integrated into the cilia axoneme.

### a. LDH measurement

The LDH concentration is measured using the Pierce^{™} Lactate Dehydrogenase Cytotoxicity Assay Kit. The procedure is performed as described in the manufacturer's protocol. The ALI cultures were incubated on the apical side with 100 µL DPBS (with Mg2+/Ca2+) for 30 min at 37 °C and 5 % CO₂. 50 µL of the LDH reaction mix and 50 µL of the incubation medium were mixed utilizing a 96-well format and incubated protected from light at room temperature for 30 min adding 50 µL stop solution. The absorbance of the formazan was measured at 490 nm and 680 nm using a microplate reader (TriStar2 Multimode Reader LB 942). The 680 nm value was substracted from the 490 nm for background normalization. A double determination for each insert was carried out.

### b. Ciliary Beat Frequency (CBF) measurement

Ciliary beat frequency (CBF) analyses of hRECs were performed by high-speed video microscopy and Sisson-Ammons Video Analysis. Videos (125 fps, 640×480 pixel resolution) were recorded using a Megaplus camera model ES 310 turbo attached to an inverted phase-contrast microscope equipped with an ELWD 40x S Plan Fluor objective under physiological conditions, by maintaining the temperature at 36 °C by a Minitube SC300 heating system.

### c. Mucociliary Clearance Assay (MCC, fluorescence beads)

ALI medium and DPBS (w/o Ca²⁺/Mg²⁺) were pre-warmed to room temperature and the plate of the microscope was heated to 37 °C. ALI inserts were transferred to a new plate and washed apically twice with pre-warmed ALI medium (alternative DPBS w/o Ca²⁺/Mg²⁺). 100 µL ALI medium was added to the apical compartment before recording 20 videos per insert (40x magnification, Ph2) to get an overall impression of the insert (SAVA software). Red fluorescent particles with a diameter of 0.5 µm were diluted 1:1000 in DPBS (w/o Ca²⁺/Mg²⁺) and vortexed for at least 1 min. 10 µL of the particle dilution were added to 100 µL of ALI medium, vortexed and 110 µL added to the apical compartment. While handling the beads, it was important to vortex before each step to prevent agglutination. Moreover, work with dimmed light was necessary to avoid bleaching.

The flow of the fluorescent particles was recorded with the NIS-Elements Basic Research software. Movement was recorded over 20-30 s using a 20x magnifcation (Ph1) while exciting the particles with a 488 nm laser. The exposure time had to be adjusted to the number of recorded frames per second (fps). For 7.5 fps the exposure time was set to 100 ms and for 15 fps to 50 ms. The exact observation area was analyzed with SAVA (20x magnifcation, Ph1) to visualize ciliary movement and 3D structures of the cell layer.

For evaluation and data analysis, Nikon NIS Elemets AR Software, NIS PLUG-IN <ADVANCED 2D TRACKING> <AR> (see above NIS Elements AR Tracking Modul) is employed.

### d. IF of ALI culture

### Embedding of ALI culture

Prior membrane preparation, the ALI insert was washed twice with DPBS (w/o Mg²⁺/Ca²⁺, 200 µL, 1 min, room temperature) to remove the mucus. The following membrane preparation as carried out according to the following protocol (see below).

The transwell filter membrane was cut out using a scalpel or a syringe before transferring the membrane into a petri dish with 3 mL DPBS (Ca²⁺/Mg²⁺). The membrane was cut into equal pieces (4-6) and cryomolds were prepared with Shandon Cryomatrix^{™} Frozen Embedding Medium (intermediate size). Membrane pieces were put into the cryomatrix and arranged in parallel to each other before cryomolds were incubated on dry ice until the cryomatrix hardens. Cryomolds were stored at -80 °C for at least 2 h before cutting at cryostat.

### Cutting of ALI membranes

ALI membranes embedded in Shandon Cryomatrix^{™} were cut using the cryostat Microm HM560. Therefore, the cryostat was pre-cooled (2-3 h prior cutting) to -20 °C (table at -21°C and chamber at -25°C). The ALI membrane containing cryomolds was transferred from -80°C to -20°C for at least 1 h. Cutting of the membrane was performed at 20 µm, depending on the handling conditions of the membrane. The membrane slices were transferred to Superfrost Ultra Plus slides, dried for at least 1 h at room temperature and stored at -80 °C over night or until use at -80 °C.

### Staining procedure

For applied antibodies, see **Table 12.**
Day 1:
1. Wash with 1x DPBS (Ca²⁺/Mg²⁺) for 5 min at room temperature
2. Fixation: 4 % PFA/1x DPBS (Ca²⁺/Mg²⁺) for 15 min at room temperature
3. Wash 3x DPBS (Ca²⁺/Mg²⁺) (2x quick, 1x 5 min)
4. Permeabilization:
   a. 0.5 % Triton X-100 für 5 min or
   b. 0.2 % Triton X-100 für 10 min (depends on the used antibody)
5. Wash 3x DPBS (Ca²⁺/Mg²⁺) (2x quick, 1x 5 min) at room temperature
6. Blocking: 5 % normal goat serum + 2 % BSA in DPBS (Ca²⁺/Mg²⁺) or 5 % skim milk in DPBS (Ca²⁺/Mg²⁺) → 2 h at room temperature
7. 1^{st} antibody: 200 µl over night at 4°C

Day 2:
8. Wash for 45 min with DPBS (Ca²⁺/Mg²⁺) (3x 15min)
9. 2^{nd} antibody: 200 µl for 1 h at room temperature
10. Hoechst33342 1:1000 in DPBS (Ca²⁺/Mg²⁺) for 10 min at room temperature in the dark
11. Wash 6x 5 min with DPBS (Ca²⁺/Mg²⁺)
12. Mount cover slips with Dako antifade

**Table 12**

| **Name** | **Species** | **Dilution** | **Reactivity** | **Cat. No.** |
|---|---|---|---|---|
| monoclonal anti-acetylated alpha-tubulin | mouse IgG2b, clone 6-11B-1 | 1:10000 | human | T6793 |
| polyclonal anti-CCDC40 | rabbit IgG | 1:200 | human | 25049-1-AP |
| polyclonal anti-CCDC39 | rabbit, affinity purified | 1:300 | human | HPA035364 |
| polyclonal anti-GAS8 | rabbit IgG | 1:200 | Human | HPA041311 |
| polyclonal anti-DNAL11 | rabbit, affinity isolated | 1:200 | human | HPA028305 |
| Alexa Fluor 546 | goat anti-rabbit IgG (H+L) | 1:1000 | human | A11035 |
| Alexa Fluor 488 | goat antimouse IgG (H+L) | 1:1000 | human | A11029 |
| DAP I | - | 1:000 | - | 62249 |

### e. Air-brush on ALI culture

An air-brush model was employed to generate differentiating ALI culture. The protocol was established after Crespin et al. 2011 (Approaches to Study Differentiation and Repair of Human Airway Epithelial Cells).

Cells were shortly washed on the apical side with pre-warmed DPBS (Mg²⁺/Ca²⁺). The compressor connected to the air-brush was set to 1 kg/cm² by running the air-brush and turning the small wheel. The air-brush might need to run for some time to stay at a constant value. Then the small reservoir on top of the air-brush was filled with DPBS (Mg²⁺/Ca²⁺) and the insert put to a new plate without basal medium. The air-brush was started by pressing to the first pressure point and waiting until the pressure on the compressor stays constant. Than the air-brush was put vertically on top of the insert and the lever of the air-brush was pressed very little beyond the first pressure point [disperses the DPBS (Mg²⁺/Ca²⁺) from the reservoir] for 2 s. Following, the apical side of the insert was shortly washed with pre-warmed DPBS (Mg²⁺/Ca²⁺) and the insert was put into fresh medium applicable for ALI differentiation. The wound was checked by microscopic observation. If it was too small (less than 1/3 of insert area) or the cells in the wound only slightly detached, the procedure was repeated. Cultures were incubated in a humidified atmosphere at 37 °C, 5 % CO₂ and cultured as ALI.

### III. Tagged CCDC40 mRNAs

The mRNAs under study are shown in **Table 13**.

**Table 13**

| **RNA-ID** | **ORF** | **5' UTR** | **3' UTR** | **Modifie d NTP I [%]** | **Modifie d NTP I [Name]** | **Modifie d NTP II [%]** | **Modifi ed NTP II [Name]** |
|---|---|---|---|---|---|---|---|
| ETH 031 T28 (**SEQ ID NO: 9**) | eGFP-hCCDC40 | minima l | no | 15 | 5-Methyl-UTP | 3 | 5-Iodo-CTP |
| ETH 031 T30 (**SEQ ID NO: 10**) | hCCDC40-eGFP | minima l | no | 15 | 5-Methyl-UTP | 3 | 5-Iodo-CTP |
| ETH 031 T26 (**SEQ ID NO: 11)** | HA-CCDC40-T2A-tdTomato | CYBA | CYBA | 15 | 5-Methyl-UTP | 3 | 5-Iodo-CTP |

### a. Cell Culture

HEK-293 cells are cultivated in MEM GlutaMAX^{™}, 10 % FBS, 100 U/ml P/S (= cultivation medium) at 37 °C, 5 % CO₂.

Before seeding, cells are washed with DPBS and detached using Trypsin. After trypsinisation, trypsin is deactivated using cultivation medium containing FBS. Therefore, an equal or greater volume of culture medium is used. After centrifugation for 5 min at 1100x g and resuspension in normal growth media, cells are counted using a Countess Cell Counting Device.

### b. In vitro transcription

To generate templates for *in vitro* transcription, circular plasmids were linearized by restriction digestion with *BstBl* and further purified by chloroform ethanol precipitation.

mRNA was produced using a standard *in vitro* transcription mix containing T7 RNA polymerase, inorganic pyrophosphatase, and RNase inhibitor. Co-transcriptional capping was achieved by addition of an ARCA cap analogue. For *in vitro* transcription of chemically modified RNA, 7.5 % of cytidine-5'-triphosphate were replaced by 5-iodocytidine-5'-triphosphate and 30 % uridine-5'-triphosphate were replaced by 5-iodouridine-5'-triphosphate, respectively. In another *SNIM*^{®}RNA production setup 3 % of cytidine-5'-triphosphate was replaced by 5-iodocytidine-5'-triphosphate and 15 % uridine-5'-triphosphate was replaced by 5-methyl-uridine-5'-triphosphate, respectively. Residual template DNA was digested using DNase!. Subsequently mRNA was purified by ammonium acetate precipitation followed by a washing step using 70 % ethanol.

Dephosphorylation of residual uncapped mRNA was carried out using a *Quick dephosphorylation Kit* followed by purification via ammonium acetate precipitation followed by a washing step using 70 % ethanol and ultrafiltration using a 100 MWCO cut of filter.

mRNA was further polyadenylated by using a poly(A) polymerase. Again mRNA was purified by ammonium acetate precipitation followed by a washing step using 70 % ethanol and ultrafiltration using a 100 MWCO cut of filter. Poly(A) length was determined by capillary gel electrophoresis to be between 100 and 250 nucleotides.

### c. Seeding, transfection and readout

Cells were seeded in 96-well black microplates (25,000 cells per well) 24 h before treatment. 24 h after seeding fresh medium was added to each well (100 µL). mRNA was transfected using Lipofectamine^{®} MessengerMAX^{™} in a RNA to Lipofectamine ratio of 1:1.5 (w/v). 250 ng/96-well (≙ 758 ng/cm²) eGFP-CCDC40 (ETH031T28 or ETH031T30) or HA-CCDC40-T2A-tdTomato (ETH031T26) mRNA was employed. All RNAs were having a stock concentration of 1 mg/mL. For lipoplex formation mRNA was diluted in aqua bidest.. Lipofectamine^{®} MessengerMAX^{™} was diluted in SFM (serum-free medium) and was mixed by pipetting. After incubation of 10 min at RT, the RNA solution was added to the Lipofectamine^{®} MessengerMAX^{™} solution, mixed and incubated for another 5 min at RT. Afterwards, the mix was diluted twofold in SFM before adding the Lipoplex solution to the wells. In **Table 14** an example for one RNA was calculated.

**Table 14**

| **ng RNA/well** | **RNA [µL]** | **H2O [µL]** | **MM [µL]** | **SFM [µL]** | **Total volume added to cells [µL]** |
|---|---|---|---|---|---|
| 250 | 4.5 | 108 | 6.76 | 105.76 | 25 |

6 h and 24 h post transfection, transfection efficiency was examined by fluorescence microscopy, pictures were taken at 10x magnification.

### IV. CCDC39

The mRNA (ETH047T02; **SEQ ID NO: 12**) under study encoded hCCDC39 and comprised Ethris minimal UTR. In total, 15% of uridine-5'-triphosphates were replaced by 5-methyl-uridine-5'-triphosphates, and in total 3 % of cytidine-5'-triphosphates by 5-iodo-cytidine-5'-triphosphates. Antibodies used are shown in **Table 15.**

**Table 15**

| **Antibody Type** | **Antibody Number** | **Name** | **Supplier** | **Cat. No** |
|---|---|---|---|---|
| First | 207 | CCDC39 | atlas antibodies | HPA035364 |
| First | 219 | Actin | abcam | ab8227 |
| Second | Goat Anti-Rabbit IgG H&L (HRP) | Abcam | ab205718 | Goat Anti-Rabbit IgG H&L (HRP) |

### 1. CCDC39 Transfection and Western blot in submerse cell culture

### a. Cell Culture

BEAS-2B cells were cultivated in LHC-9 media in coated flasks. For details see I 1 above. Experiments were performed in uncoated plates.

HEK-293 cells were cultivated in MEM GlutaMax media, supplemented with heat inactivated FBS and penicillin/streptomycin (P/S).

Before seeding, cells were washed with DPBS and detached using Trypsin. After trypsinisation, trypsin was deactivated using either a trypsin inhibitor for BEAS-2B or cultivation medium containing FBS for HEK-293. Therefore, an equal or greater volume of trypsin inhibitor/culture medium was used. After centrifugation for 5 min at 1100x g and resuspension in normal growth media, cells were counted using a Countess Cell Counting Device. Considering a seeding volume of 2 mL and a 6-well plate format, 5.0×10⁵ seeded BEAS-2B cells and 1.4×10⁶ seeded HEK-293 cells were obtained.

### b. Transfection

Cells were seeded in their respective density 24 h before treatment. 24 h after seeding 2 mL of fresh medium was added to each well. mRNA was transfected using Lipofectamine^{®} MessengerMAX^{™} in a RNA to Lipofectamine ratio of 1:1.5 (w/v). All RNAs were having a stock concentration of 1 mg/mL. For lipoplex formation mRNA was diluted in acqua bidest.. Lipofectamine^{®} MessengerMAX^{™} was diluted in SFM and was mixed by pipetting. After incubation of 10 min at RT, the RNA solution was added to the Lipofectamine^{®} MessengerMAX^{™} solution, mixed and incubated for another 5 min at RT. Afterwards, the Lipoplex solution was added to the wells. In **Table 16** an example for transfection is calculated for one 6-well plate.

**Table 16**

| | **ng/well** | **RNA [µL]** | **H2O [µL]** | **MM [µL]** | **SFM [µL]** | **Total volume added to cells [µL]** |
|---|---|---|---|---|---|---|
| High dose | 5000 | 5.0 | 120 | 7.5 | 117.5 | 250 |
| Lowe dose | 2500 | 2.5 | 122.5 | 3.75 | 121.25 | 250 |

### c. Cell lysis

To obtain optimal cell lysis, two different lysis buffers were compared, a Triton X-100 buffer and the commercially available lysis buffer M-PER^{™}, both of them complemented with protease inhibitor cOmplete, EDTA-free, and 40 µL/mL DNase I Solution (in a mixture of 23:1:1). Therefore, cells were seeded in a 6-well plate and treated for 6 h and 24 h. After treatment, cells were harvested. Therefore, plates were washed once using 1 mL DPBS. To remove the cells from the plate another 1 mL of DPBS was added and cells were scraped from plates and moved to Eppendorf tubes. To remove DPBS, cells were centrifuged at 6250x g for 2 min at 4 °C. Cell lysis was performed by adding 200 µL of the respective buffer. To ensure complete lysis the cells were incubated on ice for 30 min. After lysis, BCA Assay was performed to determine the total protein concentration using a BCA Protein Assay Kit according to the manufacturer's instructions with the following changes:
- 200 µL working reagent were added to 5 µL cell lysate
- Samples and standard were measured in triplicate
- Before incubation of samples at 37 °C for 30 minutes, the plate was shaken for 30 seconds at 450 rpm

### d. Sample preparation

The samples are mixed with 5 µL Bolt^{®} LDS Sample buffer and 2 µL Bolt^{®} Sample Reducing Agent and then heated for 10 min at 70 °C before SDS-PAGE was performed. 30 µg of total protein was used for SDS-PAGE and Bolt^{™} 4-12% SDS-PAGE gel, 10 well was employed.

### e. SDS Page and Blotting method

Trans-Blot^{®} Turbo^{™} System Transfer was used. SDS-PAGE was performed applying 200 V for 40 min. Transfer was done using the TransBlot^{®} TurboTM Transfer System for 30 minutes.

### f. Antibody and blocking method

After transfer, the membranes were blocked at RT for 1 h. NET-gelatine was used as blocking reagent. Antibody HPA035364 from atlas antibodies was used for the detection of CCDC39, and antibody ab8227 from abcam for the detection of Actin. Membranes were cut at about 60 kDa before the antibodies were added. The membranes were incubated overnight at 4 °C with the primary antibodies. After three washes (10 min each) with blocking solution at RT, horseradish peroxidase-conjugated secondary antibody, was added at RT for 1 h (diluted 1:20 000). The membranes were washed again three times with blocking solution 10 min each at RT.

### g. Chemiluminescent Signal Development

Signals were visualized with a chemiluminescent substrate kit (Luminata Crescendo, Classico or Forte Western HRP substrate, depending on the intensity of the signal) and the ChemiDocTM MP System.

### 2. Western blot of endogenous CCDC39 in differentiated ALI culture

### a. Cell lysis

250 µL ALI lysis buffer (for details of the composition cf. **Table 17**) was added per insert and incubated on ice for 15 min. Before, during (every 5 min) and at the end of incubation, ALI lysis buffer was pipetted back and forth. After transfer into eppendorf tubes and rinsing of each insert with 150 µL ALI lysis buffer, samples were vortexed thoroughly. At this step, samples could optionally be stored at -20 °C until further processing.

**Table 17**

| **Substance** | **Stock** | **Final** | **For 10 ml** |
|---|---|---|---|
| TritonX-100 | 10% | 1 % | 1 ml |
| NaCl | 1.5 M | 150 mM | 1 ml |
| SDS | 1 % | 0.1 % | 1 ml |
| Tris | 250 mM | 50 mM | 2 ml |
| Protease inhibitor cOmplete | 25 x | 1x | 0.4 ml |
| WFI | | | 4.5 ml |

### b. Separation of axonemal lysate from whole cell lysate

Samples were added to Lysing Matrix A Tubes (tubes were washed before with 70 % EthOH and garnet is removed). The tubes were fastened and frozen in liquid nitrogen for 20 seconds while moving the tube. Tubes were quickly inserted into MP FastPrep-24 (HOM-1), settings: 6.5 m/s, 3x 1 min. Subsequently, tubes were left to condense on ice for 15 minutes before transferring the liquid to fresh Eppendorf tubes and spinning at 1100 rpm, 4 °C, for 20 minutes. The supernatant was transferred to new Eppendorf tubes and pellet and supernatant were stored at -20 °C until further processing.

### c. Preparation of axonemal extract lysate: High-salt extraction of ciliary axonemal protein

Each pellet was resuspended in 50 µl MMRB + 0.1 % Triton X-100 (for details of the composition of MMRB + 0.1% TritonX-100 see **Table 18**) and incubated on ice for 30 minutes (with occasional gentle vortexing). After spinning at 14,000 rpm at 4 °C for 10 minutes, the supernatant was transferred to new Eppendorf tubes and stored at - 20 °C until further processing.

**Table 18**

| **Substance** | **Stock** | **Final** | **Volume for 1 ml** |
|---|---|---|---|
| HMEN (cf. **Table 19**) | 1x | - | 982 µl |
| DTT (e.g. Sigma 646563) | 1 M | 2 mM | 2 µl |
| β-Mercaptoethanol (e.g. Sigma M3148) | 100 % | 70 mM | 5 µl |
| TritonX-100 | 10% | 0.1% | 10 µl |
| Protease inhibitor cOmplete (Roche/Sigma 11873580001) | 25x | 1x | 40 µl |

**Table 19**

| **Substance** | **Final** |
|---|---|
| HEPES, pH 7.4 | 30 mM |
| MgSO4 | 5 mM |
| EDTA | 0.1 mM |
| NaCl | 625 mM |

### d. Preparation of lysates for Western blot

Samples were thawed on ice and 30 µl of sample were transferred to new Eppendorf tubes. 12 µL LDS sample buffer and 5 µl 10x Reducing Agent were added before heating the samples at 70 °C, 350 rpm for 10 minutes. 5, 10 and 30 µl were applied per well to a Bolt^{™} 4-12% SDS-PAGE gel, 10 well.
e. SDS Page and Blotting method: cf. IV 1 e
f. Antibody and blocking method: cf. IV 1 f
g. Chemiluminescent Signal Development: cf. IV 1 g

### II. Results

CCDC40 expression was determined in HEK293 cells after 6 h, 24 h, 48 h, 72 h and 144 h post-transfection. In particular, 2/1.4/0.3/0.2/0.05×10⁶ cells were seeded in 6-well plates and cells were transfected 24 h after seeding with different CCDC40 constructs (2.5 µg/9.5 cm²) using Lipofectamine^{®} 2000. The CCDC40 mRNA constructs used in this experiment were T06 to T10 (**SEQ ID NO: 1**, and **SEQ ID NO: 5** to **SEQ ID NO: 8**). Cell lysis was performed 6, 24, 48, 72 and 144 h after transfection and 50 µg of total protein lysate were analyzed with SDS-PAGE and Western Blot.

As it can be seen in **Figure 1****,** a peak translation efficiency was detectable 6 h post-transfection of CCDC40 mRNAs in HEK cells.

The next experiment was performed as described above except that in this case, 2×10⁶ HEK293, 7.5×10⁵ BEAS-2B and 5×10⁵ RPMI 2650 cells were seeded in 6-well plates and protein lysates analyzed with SDS-PAGE and Western Blot (HEK293: 50 µg, RPMI 2650: 20 µg, BEAS-2B: 30 µg of total lysate). CCDC40 was detected using Anti-CCDC40 Antibody (HPA022974) from Atlas Antibodies (1:2000).

Here, it could be observed that T09 (CYBA; **SEQ ID NO: 1**) and T10 (human CMV IE9 and 3' UTR from human Growth hormone **SEQ ID NO: 8**) UTRs led to the highest translation efficiency 6 h post-transfection in BEAS-2B, RPMI2650 and HEK293 cells (**Figure 2**).

Two experiments were then performed to investigate restoration of cilia motility in patient derived ALI cultures. In particular, in the first experiment a non-differentiated ALI culture (data not shown) was used (ciliary cells had a deletion of exon 1 and 2 resulting in very short and less motile cilia), whereas in the second experiment a differentiated ALI culture was used. In both cases, the ALI culture was transfected with 3 µg LF92/CCDC40 (ETH031 T09; **SEQ ID NO: 1**) every other day for 1 month for a total of 16 transfections. As readout a high speed video microscopy (HSVM) was performed every 24 h and immune fluorescence immunocytochemistry (IF-ICC). Prior transfection and every 24 h after transfection, videos (20 per insert) were taken and CFB (ciliary beat frequency) was calculated using the SAVA Software. Allover 16 transfections (1 month) were performed. Measurement was done at 37°C using the 40x magnification. Calculated are the mean values of the cilia beat frequency (CBF).

Repeated transfection of LF92/CCDC40 was well tolerated in patient derived fully differentiated ALI cultures. The CCDC40 protein could be detected in naturally occurring subcellular region, i.e. cilia, of airway epithelial cells after 22 d by IF-ICC and cilia motility increased after LF92/CCDC40 transfection until day 18 to -50% of normal cilia beat frequency as it can be seen in **Figure 3** for the experiment with the differentiated ALI culture.

As described in the Material and Methods section, an undifferentiated ALI culture was obtained using Medium G and transfections (TF) started 18 days post air-lift. Transfections (TFs) of CCDC40 patient ALI culture with CCDC40 mRNA/LF92 were performed once a week for 4 weeks (= 4x TF). Mucociliary clearance (MCC) was measured using 0.5 µm fluorescent beads at 20x magnification. 30 s videos of different areas are taken and analyzed with the Polargraph software from Nikon one week after the last TF.

Importantly, restoration of cilia beating in CCDC40 patient ALI after four weekly CCDC40 mRNA/LF92 transfections could be detected. Moreover, cilia beating was synchronized, which allowed a directed particle transport as it can be seen in **Figure 4**. For comparison, particle transport is shown in **Figure 5** in case of a control, i.e. fourfold tdTomato mRNA/LF111 transfection, and a healthy control.

Thus, successful CCDC40 mRNA/LF92 TF of patient ALI culture with LF92, as indicated by a comparison with the tdTomato control, during the differentiation phase of the ciliary cells resulted in successful cilia growth, successful cilia beating, successful directed particle transport, and successful protein detection (CCDC39 as binding partner of CCDC40 + GAS8/DNALI1 as part of the DRC complex). Moreover, tdTomato control inserts did not show any of the above mentioned effects.

**Figure 6** demonstrates successful incorporation of GAS8 protein within the axonemes after repeated patient ALI treatment with CCDC40 mRNA as in healthy controls which is absent in control (tdTomato mRNA) treated patient ALIs. **Figure 7** demonstrates successful incorporation of DNALI-1 protein within the axonemes after repeated patient ALI treatment with CCDC40 mRNA as in healthy controls which is absent in control (tdTomato mRNA) treated patient ALIs. **Figure 8** demonstrates successful incorporation of CCDC39 protein within the axonemes after repeated patient ALI treatment with CCDC40 mRNA as in healthy controls which is absent in control (tdTomato mRNA) treated patient ALIs.

To summarize, cilia motility could not be restored to a significant level when using the differentiated ALI culture, but partially restored when using the differentiated ALI culture.

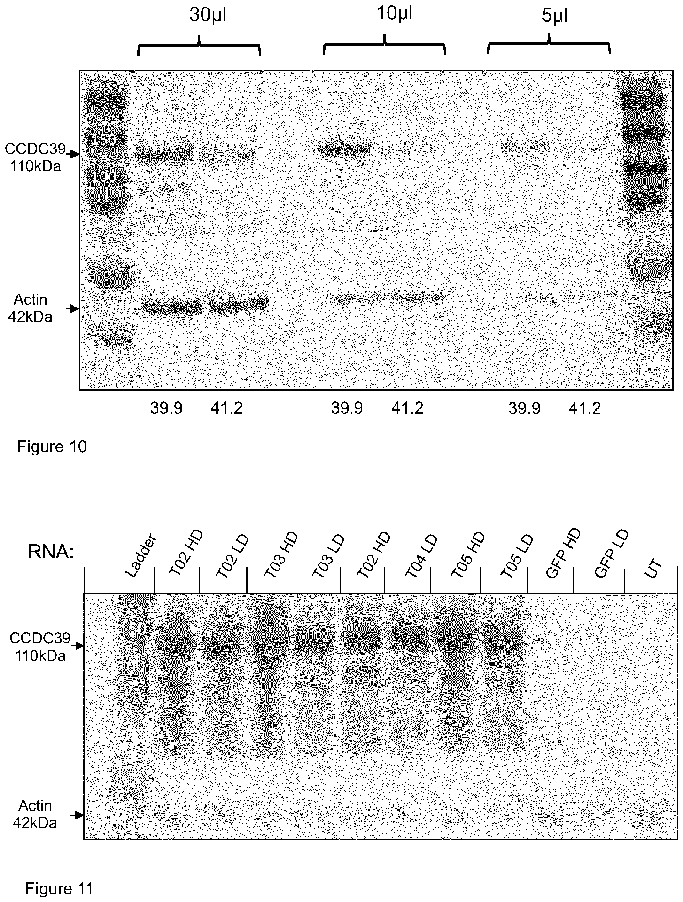

With respect to the CCDC39 experiments, CCDC39 proteins could be detected by Western Blot analyses 6 h and 24 h after transfection in HEK-293 and BEAS-2B cells as it can be seen in **Figure 9****.** As shown in **Figure 11****,** CCDC39 (ETH047T03; **SEQ ID NO: 13**) expression could be detected after 6 h in 16HBE14o- using Proteasome Inhibitor. The same experiment was also performed for ETH047T02 (**SEQ ID NO: 12**), ETH047T04 (**SEQ ID NO: 2**), and ETH047T05 (**SEQ ID NO: 14**) with comparable results. As shown in **Figure 12****,** CCDC39 (ETH047T03; **SEQ ID NO: 13**) expression could also be detected after 24 h in 16HBE14o- using Proteasome Inhibitor.

Exemplary sequences described in the application are provided below. The disclosure provides, in some embodiments, polyribonucleotides comprising, for example, the UTR sequences set forth in **SEQ ID NO: 1 or 2**, or a sequence at least 95%, 96%, 97%, 98%, or 99% identical to such sequences, or a polyribonucleotide sequence, such as an mRNA, corresponding to or encoded by any of the foregoing. In certain embodiments of any of the foregoing, the polynucleotide or polyribonucleotide is modified (e.g., comprises nucleotide analogues, as described herein).
**SEQ ID NO: 1**
   **CCDC40 sequence with CYBA 5' and 3' UTR (ETH031T09):**
   _{Part of the T7 promoter,} **Ethris' minimal 5' UTR,** *CYBA 5' UTR,* ***Kozak element,*** start codon (AUG),codon optimized sequence encoding a functional version of a human CCDC40 protein, stop codon (UGA), CYBA ***3' UTR******,*** part of restriction site for BstBI, ^{polyA}: **tail of "A" produced via post polyadenylation of mRNA** 
GGGAGACCGCGCCUAGCAGUGUCCCAGCCGGGUUCGUGUCGCCGCCACCAUGGCUGAAC
CUGGCGGAGCCGCCGGAAGAUCCCACCCUGAAGAUGGCUCUGCCAGCGAGG
GCGAGAAAGAGGGCAACAACGAGAGCCACAUGGUGUCCCCCCCAGAGAAGGA
CGACGGCCAGAAAGGCGAAGAGGCCGUGGGCUCUACCGAGCACCCUGAGGA
AGUGACCACACAGGCCGAGGCCGCCAUUGAAGAGGGCGAGGUGGAAACAGAG
GGCGAAGCCGCUGUGGAAGGCGAAGAGGAAGCCGUGUCUUACGGCGACGCC
GAGAGCGAGGAAGAGUACUACUACACCGAGACAAGCAGCCCCGAGGGCCAGA
UCUCUGCCGCCGAUACCACCUACCCCUACUUCAGCCCCCCUCAGGAACUGCC
UGGGGAAGAGGCCUACGAUAGCGUGUCCGGCGAAGCUGGCCUGCAGGGCUU
UCAGCAGGAAGCCACAGGCCCUCCCGAGAGCCGGGAAAGAAGAGUGACAAGC
CCCGAGCCUAGCCACGGCGUGCUGGGACCAUCUGAGCAGAUGGGCCAAGUG
ACCUCUGGCCCUGCUGUGGGCAGACUGACAGGCAGCACAGAGGAACCUCAG
GGCCAGGUGCUGCCUAUGGGAGUGCAGCACCGGUUCAGACUGAGCCACGGC
AGCGACAUCGAGAGCAGCGACCUGGAAGAGUUCGUCAGCCAGGAACCCGUGA
UCCCUCCUGGCGUGCCAGAUGCCCAUCCCAGGGAAGGCGAUCUGCCCGUGU
UCCAGGACCAGAUCCAGCAGCCCUCUACCGAAGAGGGGGCUAUGGCCGAGAG
AGUGGAAAGCGAGGGCUCCGACGAAGAAGCCGAGGACGAGGGAUCUCAGCU
GGUGGUGCUGGACCCCGACCACCCUCUGAUGGUGCGGUUUCAGGCCGCCCU
GAAGAACUACCUGAACCGGCAGAUCGAGAAGCUGAAACUGGACCUGCAGGAA
CUGGUGGUGGCCACAAAGCAGAGCAGAGCCCAGAGACAGGAACUGGGCGUG
AACCUGUACGAGGUGCAGCAGCAUCUGGUGCAUCUGCAGAAGCUGCUGGAAA
AGAGCCACGACCGGCACGCCAUGGCCAGCUCUGAGCGCAGACAGAAAGAGGA
AGAACUGCAGGCCGCCAGAGCCCUGUACACCAAGACAUGCGCCGCUGCCAAC
 
GAGGAACGGAAGAAGCUGGCUGCCCUGCAGACCGAGAUGGAAAACCUGGCUC
UGCACCUGUUCUACAUGCAGAAUAUCGACCAGGACAUGCGGGACGACAUCAG
AGUGAUGACCCAGGUCGUGAAGAAGGCCGAGACAGAGAGAAUCCGGGCCGAG
AUUGAGAAGAAAAAGCAGGACCUGUACGUGGACCAGCUGACCACCAGGGCCC
AGCAGCUGGAAGAGGAUAUCGCCCUGUUCGAGGCCCAGUACCUGGCCCAGG
CCGAAGAUACCCGGAUCCUGAGAAAGGCCGUGUCCGAGGCCUGCACCGAGAU
CGAUGCCAUCAGCGUGGAAAAGCGGCGGAUCAUGCAGCAGUGGGCCAGCAG
CCUCGUGGGCAUGAAGCACAGAGAUGAGGCCCACCGGGCCGUGCUGGAAGC
UCUGAGAGGCUGUCAGCACCAGGCCAAGAGCACCGACGGCGAGAUCGAGGC
CUACAAGAAAUCCAUCAUGAAGGAAGAGGAAAAGAACGAGAAACUGGCCAGCA
UCCUGAACAGAACCGAAACCGAGGCCACCCUGCUGCAGAAACUGACCACCCA
GUGCCUGACCAAACAGGUGGCCCUGCAGUCCCAGUUCAACACCUACAGACUG
ACCCUGCAGGACACCGAGGACGCCCUGAGUCAGGAUCAGCUGGAACAGAUGA
UUCUGACCGAGGAACUGCAGGCUAUCCGGCAGGCCAUUCAGGGGGAGCUGG
AACUGCGGAGAAAGACCGACGCCGCCAUCAGAGAGAAGCUGCAGGAACACAU
GACCAGCAACAAGACCACCAAGUACUUCAACCAGCUGAUUCUGCGCCUGCAG
AAAGAAAAGACCAACAUGAUGACACACCUGAGCAAGAUCAACGGCGACAUUGC
CCAGACCACCCUGGACAUCACCCACACCAGCAGCAGACUGGACGCCCACCAG
AAAACCCUGGUGGAACUGGACCAGGAUGUGAAGAAAGUGAACGAGCUGAUCA
CCAACAGCCAGAGCGAGAUCAGCCGGCGGACCAUCCUGAUCGAGAGAAAGCA
GGGCCUGAUCAACUUCCUGAACAAACAGCUGGAAAGAAUGGUGUCCGAGCUG
GGCGGCGAGGAAGUGGGACCUCUGGAACUGGAAAUCAAGCGGCUGAGCAAG
CUGAUCGACGAGCACGACGGCAAGGCCGUGCAGGCUCAAGUGACAUGGCUG
CGGCUGCAGCAGGAAAUGGUCAAAGUGACCCAGGAACAGGAAGAACAGCUGG
CCUCCCUGGACGCCAGCAAGAAAGAACUGCACAUCAUGGAACAGAAAAAGCU
GCGGGUGGAAAGCAAGAUCGAGCAGGAAAAAAAAGAACAGAAAGAAAUCGAGC
ACCACAUGAAGGACCUGGACAACGACCUGAAGAAACUGAAUAUGCUGAUGAAC
AAGAACCGCUGCUCCAGCGAAGAACUGGAACAGAACAACAGAGUGACCGAGA
ACGAGUUCGUGCGGAGCCUGAAGGCCAGCGAGCGGGAAACCAUCAAGAUGCA
GGACAAGCUGAACCAGCUGUCCGAGGAAAAAGCCACACUGCUGAACCAGCUG
GUGGAAGCCGAGCACCAGAUCAUGCUGUGGGAGAAGAAGAUCCAGCUGGCCA
AAGAAAUGCGGAGCAGCGUGGACAGCGAGAUCGGCCAGACCGAAAUCAGAGC
CAUGAAGGGCGAGAUCCACCGGAUGAAAGUGCGGCUGGGACAGCUGCUGAAA
CAGCAGGAAAAGAUGAUCCGGGCCAUGGAACUGGCCGUGGCCAGACGGGAAA
CCGUGACAACCCAGGCUGAGGGCCAGCGGAAGAUGGACAGAAAGGCCCUGAC
CCGGACCGACUUCCACCACAAGCAGCUGGAACUGAGGCGGAAGAUCCGGGAC
GUGCGGAAGGCCACCGAUGAGUGCACAAAGACAGUGCUGGAACUGGAAGAGA
CACAGCGGAACGUGUCCUCCAGCCUGCUGGAAAAACAGGAAAAGCUGAGCGU
GAUCCAGGCCGACUUCGACACCCUGGAAGCUGACCUGACAAGACUGGGAGCC
CUGAAAAGACAGAACCUGUCCGAGAUCGUGGCACUGCAGACCCGGCUGAAAC
AUCUGCAGGCUGUGAAAGAGGGACGCUACGUGUUCCUGUUCAGAUCCAAGCA
GUCUCUGGUGCUGGAAAGACAGCGGCUGGACAAGCGGCUGGCACUGAUUGC
CACCAUCCUGGAUAGAGUGCGCGACGAGUACCCACAGUUCCAGGAAGCACUG
CACAAGGUGUCCCAGAUGAUCGCCAACAAGCUGGAAUCCCCUGGCCCCAGCU
 
GAccUcGccccGGAccUGcccUcccGccAGGUGcAcccAccUGcAAUAAAUGcAGcGAAGcc
GGGAUUCG-polyA
**SEQ ID NO: 2**
   **CCDC39 sequence with CYBA UTRs (ETH047T04):**
   Part of the T7 promoter, **Ethris' minimal 5' UTR,** CYBA *5' UTR**, **Kozak element,*** start codon (AUG), codon optimized sequence encoding a functional version of a human CCDC39 protein, stop codon (UGA), CYBA ***3' UTR******,*** part of restriction site for BstBI, poly(A) tail 
GGGAGACCGCGCCUAGCAGUGUCCCAGCCGGGUUCGUGUCGCCGCCACCAUGAGCAGCG
AGUUUCUGGCCGAACUGCACUGGGAGGACGGCUUCGCUAUUCCCGUGGCCA
ACGAGGAAAACAAGCUGCUGGAAGAUCAGCUGAGCAAGCUGAAGGACGAGAG
AGCCUCUCUGCAGGACGAGCUGAGAGAGUACGAGGAACGGAUCAACAGCAUG
ACCAGCCACUUCAAGAACGUGAAGCAAGAGCUGAGCAUCACCCAGAGCCUGU
GCAAGGCCAGAGAGAGAGAAACCGAGAGCGAGGAACACUUCAAGGCUAUCGC
CCAGCGCGAGCUGGGAAGAGUGAAGGAUGAGAUCCAGCGGCUGGAAAACGA
GAUGGCCAGCAUCCUGGAAAAGAAGUCCGACAAAGAGAACGGCAUCUUCAAG
GCCACACAGAAGCUGGACGGCCUGAAGUGCCAGAUGAACUGGGAUCAGCAGG
CCCUGGAAGCCUGGCUGGAAGAGUCUGCCCACAAGGAUUCUGACGCCCUGAC
ACUGCAGAAGUACGCCCAGCAGGACGACAACAAGAUCCGGGCUCUGACCCUG
CAGCUGGAAAGACUGACCCUGGAAUGCAACCAGAAGCGGAAGAUCCUGGACA
ACGAGCUGACCGAGACAAUCAGCGCCCAGCUGGAACUGGAUAAGGCCGCUCA
GGACUUCAGAAAGAUCCACAACGAGCGGCAAGAACUGAUCAAGCAGUGGGAG
AACACCAUCGAGCAGAUGCAGAAACGCGACGGCGACAUCGACAACUGCGCCC
UGGAACUCGCCCGGAUCAAGCAAGAGACACGCGAGAAAGAGAACCUGGUCAA
AGAGAAGAUCAAGUUCCUCGAGUCCGAGAUCGGCAACAACACCGAGUUCGAG
AAGCGGAUCAGCGUGGCCGACAGAAAGCUGCUGAAGUGCAGAACCGCCUACC
AGGACCACGAGACAAGCCGGAUUCAGCUCAAGGGCGAGCUGGAUUCUCUGAA
GGCCACCGUGAACAGAACCAGCAGCGAUCUGGAAGCCCUGCGGAAGAACAUC
AGCAAGAUCAAGAAGGACAUCCACGAGGAAACCGCCAGGCUGCAGAAAACAAA
GAACCACAAUGAGAUCAUCCAGACCAAGCUGAAAGAGAUCACCGAAAAGACCA
UGAGCGUGGAAGAGAAGGCCACAAACCUGGAAGAUAUGCUCAAAGAGGAAGA
GAAAGACGUCAAAGAGGUGGACGUUCAACUGAACCUGAUUAAGGGCGUGCUG
UUCAAGAAGGCCCAAGAGCUGCAGACCGAAACCAUGAAGGAAAAGGCCGUCC
UGUCUGAGAUCGAGGGCACCAGAUCUAGCCUGAAGCACCUGAACCAUCAGCU
GCAGAAGCUCGACUUCGAGACACUGAAGCAGCAAGAGAUCAUGUACAGCCAG
GAUUUCCACAUCCAGCAGGUCGAGCGGCGGAUGUCUAGACUGAAGGGCGAG
AUCAACUCCGAGGAAAAACAGGCCCUCGAGGCCAAGAUCGUGGAACUGAGAA
AGAGCCUCGAAGAGAAGAAGUCUACCUGCGGCCUGCUGGAAACCCAGAUUAA
GAAGCUGCACAACGACCUGUACUUCAUCAAGAAAGCCCACAGCAAGAACAGCG
ACGAGAAGCAGAGCCUGAUGACCAAGAUCAAUGAGCUGAACCUGUUCAUCGA
 
UCGGAGCGAAAAAGAGCUGGACAAGGCCAAGGGCUUCAAGCAGGACCUGAUG
AUCGAGGACAACCUGCUGAAGCUGGAAGUGAAGCGGACCAGAGAGAUGCUGC
ACAGCAAGGCCGAGGAAGUGCUGUCUCUGGAAAAGCGGAAGCAGCAGCUGUA
CACCGCCAUGGAAGAGAGAACCGAAGAGAUCAAGGUGCACAAGACCAUGCUG
GCUUCCCAGAUCAGAUACGUGGACCAAGAGCGCGAGAACAUCUCCACCGAGU
UUAGAGAGAGACUGUCCAAGAUCGAGAAGCUGAAGAACCGCUACGAGAUCCU
GACCGUCGUGAUGCUGCCUCCUGAGGGCGAAGAGGAAAAGACCCAGGCCUAC
UACGUGAUCAAGGCAGCCCAAGAAAAAGAGGAACUCCAGAGAGAAGGCGACU
GCCUGGACGCCAAGAUUAACAAGGCCGAAAAAGAAAUCUACGCCCUCGAGAA
CACCCUGCAGGUCCUGAACAGCUGCAACAACAACUACAAGCAGAGCUUCAAGA
AAGUCACCCCUAGCUCCGACGAGUACGAGCUGAAGAUUCAGCUGGAAGAACA
GAAAAGAGCCGUGGACGAGAAGUACAGAUACAAGCAGCGGCAGAUCAGAGAG
CUGCAAGAGGAUAUCCAGAGCAUGGAAAACACCCUGGACGUGAUCGAGCACC
UGGCCAACAACGUGAAAGAGAAGCUGUCCGAGAAACAGGCCUACAGCUUUCA
GCUGUCCAAAGAGACAGAGGAACAGAAGCCCAAACUGGAACGCGUGACCAAG
CAGUGCGCCAAGCUGACAAAAGAGAUCCGGCUGCUGAAAGACACCAAGGACG
AAACCAUGGAAGAACAAGACAUCAAGCUGCGCGAGAUGAAGCAGUUCCACAAA
GUGAUCGACGAGAUGCUGGUGGACAUCAUUGAAGAGAACACAGAGAUCCGCA
UCAUCCUGCAGACCUAUUUUCAGCAGAGCGGCCUGGAACUGCCUACCGCCUC
UACAAAGGGCAGCAGACAGAGCAGCAGAUCCCCUAGCCACACAAGCCUGAGC
GCCAGAAGCUCUAGAAGCACCAGCACCUCUACCAGCCAGUCCAGCAUUAAGG
UGCUGGAACUCAAGUUCCCCGCCAGCUCUAGCCUCGUGGGAAGCCCUUCUAG
ACCUAGCAGCGCCUCUAGCAGCUCCAGCAACGUGAAGUCCAAGAAAAGCUCC
AAGUGAccUcGccccGGAccUGcccUcccGccAGGUGcAcccAccUGcAAUAAAUGcAGcG
AAGccGGGAUUCGAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAA
**SEQ ID NO: 3**
   **tdTomato:**
   Part of the T7 promoter, **Ethris' minimal 5' UTR**, ^{vector backbone sequence (pVAX Vektor: Life Technologies)}, ***Kozak element,*** ***start codon*** ***(******AUG******)**,* codon optimized sequence encoding a functional version of a tdTomato protein, ***stop codon*** ***(******UAG******)*** , part of restriction site for NotI_{,} *^{part of the vector backbone sequence (pVAX Vektor: Life Technologies)}* 
GGGAGACCCAAGCUGGCUAGCGUUUAAACUUAAGCUUGGUACCGCGGGCCCGGGAUCCACCGGUCGCCACCAU
GGUGAGCAAGGGCGAGGAGGUCAUCAAAGAGUUCAUGCGCUUCAAGGUGCG
CAUGGAGGGCUCCAUGAACGGCCACGAGUUCGAGAUCGAGGGCGAGGGCGA
GGGCCGCCCCUACGAGGGCACCCAGACCGCCAAGCUGAAGGUGACCAAGGG
CGGCCCCCUGCCCUUCGCCUGGGACAUCCUGUCCCCCCAGUUCAUGUACGG
CUCCAAGGCGUACGUGAAGCACCCCGCCGACAUCCCCGAUUACAAGAAGCUG
UCCUUCCCCGAGGGCUUCAAGUGGGAGCGCGUGAUGAACUUCGAGGACGGC
GGUCUGGUGACCGUGACCCAGGACUCCUCCCUGCAGGACGGCACGCUGAUC
 
UACAAGGUGAAGAUGCGCGGCACCAACUUCCCCCCCGACGGCCCCGUAAUGC
AGAAGAAGACCAUGGGCUGGGAGGCCUCCACCGAGCGCCUGUACCCCCGCG
ACGGCGUGCUGAAGGGCGAGAUCCACCAGGCCCUGAAGCUGAAGGACGGCG
GCCACUACCUGGUGGAGUUCAAGACCAUCUACAUGGCCAAGAAGCCCGUGCA
ACUGCCCGGCUACUACUACGUGGACACCAAGCUGGACAUCACCUCCCACAAC
GAGGACUACACCAUCGUGGAACAGUACGAGCGCUCCGAGGGCCGCCACCACC
UGUUCCUGGGGCAUGGCACCGGCAGCACCGGCAGCGGCAGCUCCGGCACCG
CCUCCUCCGAGGACAACAACAUGGCCGUCAUCAAAGAGUUCAUGCGCUUCAA
GGUGCGCAUGGAGGGCUCCAUGAACGGCCACGAGUUCGAGAUCGAGGGCGA
GGGCGAGGGCCGCCCCUACGAGGGCACCCAGACCGCCAAGCUGAAGGUGAC
CAAGGGCGGCCCCCUGCCCUUCGCCUGGGACAUCCUGUCCCCCCAGUUCAU
GUACGGCUCCAAGGCGUACGUGAAGCACCCCGCCGACAUCCCCGAUUACAAG
AAGCUGUCCUUCCCCGAGGGCUUCAAGUGGGAGCGCGUGAUGAACUUCGAG
GACGGCGGUCUGGUGACCGUGACCCAGGACUCCUCCCUGCAGGACGGCACG
CUGAUCUACAAGGUGAAGAUGCGCGGCACCAACUUCCCCCCCGACGGCCCCG
UAAUGCAGAAGAAGACCAUGGGCUGGGAGGCCUCCACCGAGCGCCUGUACCC
CCGCGACGGCGUGCUGAAGGGCGAGAUCCACCAGGCCCUGAAGCUGAAGGA
CGGCGGCCACUACCUGGUGGAGUUCAAGACCAUCUACAUGGCCAAGAAGCCC
GUGCAACUGCCCGGCUACUACUACGUGGACACCAAGCUGGACAUCACCUCCC
ACAACGAGGACUACACCAUCGUGGAACAGUACGAGCGCUCCGAGGGCCGCCA
CCACCUGUUCCUGUACGGCAUGGACGAGCUGUACAAGUAGGCGGCCAAUUCUGCAGAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAGCGGCC
**SEQ ID NO: 4**
   **MCIDAS sequence with Ethris' minimal 5' UTR and optional 3' UTR:**
   Part of the T7 promoter, **Ethris' minimal 5' UTR,** ***Kozak element,*** start codon (AUG), codon optimized sequence encoding a functional version of a human MCIDAS protein, stop codon (UGA), ***optional 3' UTR*** (also functions as reverse primer binding site for PCR based template production) , restriction site for NheI, ^{poly(A) tail} 
GGGAGACGCCACCAUGCAAGCUUGUGGCGGAGCUGGAAGAAGGGCCU
UCGAUAGCAUCUGCCCCAACCGGAUGCUUGCCCUUCCUGGUAGAGCCCUGCU
GUGCAAGCCUGGCAAGCCCGAGAGAAAGUUCGCCCCUCCAAGAAAGUUCUUC
CCCGGCUGUACUGGCGGCAGCCCUGUGUCUGUGUAUGAGGACCCUCCUGAU
GCCGAGCCUACAGCUCUGCCUGCUCUGACCACAAUCGACCUGCAGGAUCUGG
CCGAUUGCAGCUCUCUGCUGGGAUCUGAUGCUCCUCCUGGCGGAGAUCUGG
CUGCCUCUCAGAAUCACAGCCACCAGACAGAGGCCGACUUCAACCUGCAAGA
CUUCCGGGACACCGUGGACGACCUGAUCAGCGAUAGCAGCAGCAUGAUGAGC
CCCACUCUGGCCAGCGGCGAUUUCCCAUUCAGCCCCUGUGACAUCAGCCCUU
UCGGCCCUUGUCUGAGCCCUCCACUGGAUCCUAGAGCACUGCAGAGCCCACC
UCUGAGGCCUCCAGAUGUUCCUCCACCUGAGCAGUACUGGAAAGAGGUGGCC
 
GACCAGAACCAGAGAGCACUGGGCGACGCUCUGGUGGAAAACAACCAGCUGC
ACGUGACCCUGACACAGAAGCAAGAAGAGAUCGCCAGCCUGAAAGAACGGAA
UGUGCAGCUGAAAGAGCUGGCCUCCAGGACAAGACACCUGGCCAGUGUGCU
GGACAAGCUGAUGAUCACCCAGAGCAGAGAUUGCGGAGCCGCCGCUGAACCU
UUUCUGCUGAAGGCCAAGGCCAAGAGAAGCCUGGAAGAACUGGUGUCUGCCG
CCGGACAGGAUUGCGCUGAAGUGGAUGCCAUCCUGCGCGAGAUCAGCGAGA
GAUGUGAUGAGGCCCUGCAGAGCAGGGACCCCAAAAGACCUAGACUGCUGCC
CGAGCCUGCCAACACCGAUACCAGACCUGGAAAUCUGCACGGCGCCUUCAGA
GGCCUGAGAACCGAUUGCUCUAGAAGCGCCCUGAACCUGAGCCACAGCGAAC
UCGAAGAAGGCGGCAGCUUCAGCACCCGGAUCAGAAGCCACAGCACCAUCAG
AACCCUGGCCUUUCCACAGGGCAACGCCUUCACAAUCAGAACCGCCAACGGC
GGCUACAAGUUCAGAUGGGUGCCAAGCUGAGCUAGCCACCGGGCAAUACGA
GCUCAAGCCAGUCUCAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAA
**SEQ ID NO: 5**
   **Sequence with Ethris' minimal** **5'** **UTR (ETH031T06):**
   _{Part of the T7 promoter,} **Ethris' minimal 5' UTR,** ***Kozak element,*** start codon (AUG), codon optimized sequence encoding a functional version of a human CCDC40 protein, stop codon (UGA), part of restriction site for BstBI, ^{polyA}: **tail of "A" produced via post polyadenylation of mRNA** 
GGGAGACGCCACCAUGGCUGAACCUGGCGGAGCCGCCGGAAGAUCCCACCCUG
AAGAUGGCUCUGCCAGCGAGGGCGAGAAAGAGGGCAACAACGAGAGCCACAU
GGUGUCCCCCCCAGAGAAGGACGACGGCCAGAAAGGCGAAGAGGCCGUGGG
CUCUACCGAGCACCCUGAGGAAGUGACCACACAGGCCGAGGCCGCCAUUGAA
GAGGGCGAGGUGGAAACAGAGGGCGAAGCCGCUGUGGAAGGCGAAGAGGAA
GCCGUGUCUUACGGCGACGCCGAGAGCGAGGAAGAGUACUACACCGAGA
CAAGCAGCCCCGAGGGCCAGAUCUCUGCCGCCGAUACCACCUACCCCUACUU
CAGCCCCCCUCAGGAACUGCCUGGGGAAGAGGCCUACGAUAGCGUGUCCGG
CGAAGCUGGCCUGCAGGGCUUUCAGCAGGAAGCCACAGGCCCUCCCGAGAG
CCGGGAAAGAAGAGUGACAAGCCCCGAGCCUAGCCACGGCGUGCUGGGACCA
UCUGAGCAGAUGGGCCAAGUGACCUCUGGCCCUGCUGUGGGCAGACUGACA
GGCAGCACAGAGGAACCUCAGGGCCAGGUGCUGCCUAUGGGAGUGCAGCAC
CGGUUCAGACUGAGCCACGGCAGCGACAUCGAGAGCAGCGACCUGGAAGAGU
UCGUCAGCCAGGAACCCGUGAUCCCUCCUGGCGUGCCAGAUGCCCAUCCCAG
GGAAGGCGAUCUGCCCGUGUUCCAGGACCAGAUCCAGCAGCCCUCUACCGAA
GAGGGGGCUAUGGCCGAGAGAGUGGAAAGCGAGGGCUCCGACGAAGAAGCC
GAGGACGAGGGAUCUCAGCUGGUGGUGCUGGACCCCGACCACCCUCUGAUG
GUGCGGUUUCAGGCCGCCCUGAAGAACUACCUGAACCGGCAGAUCGAGAAGC
UGAAACUGGACCUGCAGGAACUGGUGGUGGCCACAAAGCAGAGCAGAGCCCA
GAGACAGGAACUGGGCGUGAACCUGUACGAGGUGCAGCAGCAUCUGGUGCA
 
UCUGCAGAAGCUGCUGGAAAAGAGCCACGACCGGCACGCCAUGGCCAGCUCU
GAGCGCAGACAGAAAGAGGAAGAACUGCAGGCCGCCAGAGCCCUGUACACCA
AGACAUGCGCCGCUGCCAACGAGGAACGGAAGAAGCUGGCUGCCCUGCAGAC
CGAGAUGGAAAACCUGGCUCUGCACCUGUUCUACAUGCAGAAUAUCGACCAG
GACAUGCGGGACGACAUCAGAGUGAUGACCCAGGUCGUGAAGAAGGCCGAGA
CAGAGAGAAUCCGGGCCGAGAUUGAGAAGAAAAAGCAGGACCUGUACGUGGA
CCAGCUGACCACCAGGGCCCAGCAGCUGGAAGAGGAUAUCGCCCUGUUCGAG
GCCCAGUACCUGGCCCAGGCCGAAGAUACCCGGAUCCUGAGAAAGGCCGUGU
CCGAGGCCUGCACCGAGAUCGAUGCCAUCAGCGUGGAAAAGCGGCGGAUCAU
GCAGCAGUGGGCCAGCAGCCUCGUGGGCAUGAAGCACAGAGAUGAGGCCCA
CCGGGCCGUGCUGGAAGCUCUGAGAGGCUGUCAGCACCAGGCCAAGAGCAC
CGACGGCGAGAUCGAGGCCUACAAGAAAUCCAUCAUGAAGGAAGAGGAAAAG
AACGAGAAACUGGCCAGCAUCCUGAACAGAACCGAAACCGAGGCCACCCUGC
UGCAGAAACUGACCACCCAGUGCCUGACCAAACAGGUGGCCCUGCAGUCCCA
GUUCAACACCUACAGACUGACCCUGCAGGACACCGAGGACGCCCUGAGUCAG
GAUCAGCUGGAACAGAUGAUUCUGACCGAGGAACUGCAGGCUAUCCGGCAGG
CCAUUCAGGGGGAGCUGGAACUGCGGAGAAAGACCGACGCCGCCAUCAGAGA
GAAGCUGCAGGAACACAUGACCAGCAACAAGACCACCAAGUACUUCAACCAGC
UGAUUCUGCGCCUGCAGAAAGAAAAGACCAACAUGAUGACACACCUGAGCAA
GAUCAACGGCGACAUUGCCCAGACCACCCUGGACAUCACCCACACCAGCAGC
AGACUGGACGCCCACCAGAAAACCCUGGUGGAACUGGACCAGGAUGUGAAGA
AAGUGAACGAGCUGAUCACCAACAGCCAGAGCGAGAUCAGCCGGCGGACCAU
CCUGAUCGAGAGAAAGCAGGGCCUGAUCAACUUCCUGAACAAACAGCUGGAA
AGAAUGGUGUCCGAGCUGGGCGGCGAGGAAGUGGGACCUCUGGAACUGGAA
AUCAAGCGGCUGAGCAAGCUGAUCGACGAGCACGACGGCAAGGCCGUGCAG
GCUCAAGUGACAUGGCUGCGGCUGCAGCAGGAAAUGGUCAAAGUGACCCAGG
AACAGGAAGAACAGCUGGCCUCCCUGGACGCCAGCAAGAAAGAACUGCACAU
CAUGGAACAGAAAAAGCUGCGGGUGGAAAGCAAGAUCGAGCAGGAAAAAAAA
GAACAGAAAGAAAUCGAGCACCACAUGAAGGACCUGGACAACGACCUGAAGAA
ACUGAAUAUGCUGAUGAACAAGAACCGCUGCUCCAGCGAAGAACUGGAACAG
AACAACAGAGUGACCGAGAACGAGUUCGUGCGGAGCCUGAAGGCCAGCGAGC
GGGAAACCAUCAAGAUGCAGGACAAGCUGAACCAGCUGUCCGAGGAAAAAGC
CACACUGCUGAACCAGCUGGUGGAAGCCGAGCACCAGAUCAUGCUGUGGGAG
AAGAAGAUCCAGCUGGCCAAAGAAAUGCGGAGCAGCGUGGACAGCGAGAUCG
GCCAGACCGAAAUCAGAGCCAUGAAGGGCGAGAUCCACCGGAUGAAAGUGCG
GCUGGGACAGCUGCUGAAACAGCAGGAAAAGAUGAUCCGGGCCAUGGAACUG
GCCGUGGCCAGACGGGAAACCGUGACAACCCAGGCUGAGGGCCAGCGGAAG
AUGGACAGAAAGGCCCUGACCCGGACCGACUUCCACCACAAGCAGCUGGAAC
UGAGGCGGAAGAUCCGGGACGUGCGGAAGGCCACCGAUGAGUGCACAAAGA
CAGUGCUGGAACUGGAAGAGACACAGCGGAACGUGUCCUCCAGCCUGCUGGA
AAAACAGGAAAAGCUGAGCGUGAUCCAGGCCGACUUCGACACCCUGGAAGCU
GACCUGACAAGACUGGGAGCCCUGAAAAGACAGAACCUGUCCGAGAUCGUGG
CACUGCAGACCCGGCUGAAACAUCUGCAGGCUGUGAAAGAGGGACGCUACGU
GUUCCUGUUCAGAUCCAAGCAGUCUCUGGUGCUGGAAAGACAGCGGCUGGA
CAAGCGGCUGGCACUGAUUGCCACCAUCCUGGAUAGAGUGCGCGACGAGUAC
 
CCACAGUUCCAGGAAGCACUGCACAAGGUGUCCCAGAUGAUCGCCAACAAGC
UGGAAUCCCCUGGCCCCAGCUGAUUCG-polyA
S**EQ ID NO: 6**
   **Sequence with TISU 5' UTR (ETH031T07):**
   Part of the T7 promoter, **Ethris' minimal 5' UTR with additional U nucleotide**, ***TISU element**,* start codon (AUG), codon optimized sequence encoding a functional version of a human CCDC40 protein, stop codon (UGA), part of restriction site for BstBI, ^{polyA}: **tail of "A" produced via post polyadenylation of mRNA** 
GGGAGACUGCCAAGAUGGCUGAACCUGGCGGAGCCGCCGGAAGAUCCCACCCU
GAAGAUGGCUCUGCCAGCGAGGGCGAGAAAGAGGGCAACAACGAGAGCCACA
UGGUGUCCCCCCCAGAGAAGGACGACGGCCAGAAAGGCGAAGAGGCCGUGG
GCUCUACCGAGCACCCUGAGGAAGUGACCACACAGGCCGAGGCCGCCAUUGA
AGAGGGCGAGGUGGAAACAGAGGGCGAAGCCGCUGUGGAAGGCGAAGAGGA
AGCCGUGUCUUACGGCGACGCCGAGAGCGAGGAAGAGUACUACACCGAG
ACAAGCAGCCCCGAGGGCCAGAUCUCUGCCGCCGAUACCACCUACCCCUACU
UCAGCCCCCCUCAGGAACUGCCUGGGGAAGAGGCCUACGAUAGCGUGUCCG
GCGAAGCUGGCCUGCAGGGCUUUCAGCAGGAAGCCACAGGCCCUCCCGAGA
GCCGGGAAAGAAGAGUGACAAGCCCCGAGCCUAGCCACGGCGUGCUGGGAC
CAUCUGAGCAGAUGGGCCAAGUGACCUCUGGCCCUGCUGUGGGCAGACUGA
CAGGCAGCACAGAGGAACCUCAGGGCCAGGUGCUGCCUAUGGGAGUGCAGC
ACCGGUUCAGACUGAGCCACGGCAGCGACAUCGAGAGCAGCGACCUGGAAGA
GUUCGUCAGCCAGGAACCCGUGAUCCCUCCUGGCGUGCCAGAUGCCCAUCC
CAGGGAAGGCGAUCUGCCCGUGUUCCAGGACCAGAUCCAGCAGCCCUCUACC
GAAGAGGGGGCUAUGGCCGAGAGAGUGGAAAGCGAGGGCUCCGACGAAGAA
GCCGAGGACGAGGGAUCUCAGCUGGUGGUGCUGGACCCCGACCACCCUCUG
AUGGUGCGGUUUCAGGCCGCCCUGAAGAACUACCUGAACCGGCAGAUCGAGA
AGCUGAAACUGGACCUGCAGGAACUGGUGGUGGCCACAAAGCAGAGCAGAGC
CCAGAGACAGGAACUGGGCGUGAACCUGUACGAGGUGCAGCAGCAUCUGGU
GCAUCUGCAGAAGCUGCUGGAAAAGAGCCACGACCGGCACGCCAUGGCCAGC
UCUGAGCGCAGACAGAAAGAGGAAGAACUGCAGGCCGCCAGAGCCCUGUACA
CCAAGACAUGCGCCGCUGCCAACGAGGAACGGAAGAAGCUGGCUGCCCUGCA
GACCGAGAUGGAAAACCUGGCUCUGCACCUGUUCUACAUGCAGAAUAUCGAC
CAGGACAUGCGGGACGACAUCAGAGUGAUGACCCAGGUCGUGAAGAAGGCCG
AGACAGAGAGAAUCCGGGCCGAGAUUGAGAAGAAAAAGCAGGACCUGUACGU
GGACCAGCUGACCACCAGGGCCCAGCAGCUGGAAGAGGAUAUCGCCCUGUUC
GAGGCCCAGUACCUGGCCCAGGCCGAAGAUACCCGGAUCCUGAGAAAGGCCG
UGUCCGAGGCCUGCACCGAGAUCGAUGCCAUCAGCGUGGAAAAGCGGCGGA
UCAUGCAGCAGUGGGCCAGCAGCCUCGUGGGCAUGAAGCACAGAGAUGAGG
CCCACCGGGCCGUGCUGGAAGCUCUGAGAGGCUGUCAGCACCAGGCCAAGA
GCACCGACGGCGAGAUCGAGGCCUACAAGAAAUCCAUCAUGAAGGAAGAGGA
 
AAAGAACGAGAAACUGGCCAGCAUCCUGAACAGAACCGAAACCGAGGCCACCC
UGCUGCAGAAACUGACCACCCAGUGCCUGACCAAACAGGUGGCCCUGCAGUC
CCAGUUCAACACCUACAGACUGACCCUGCAGGACACCGAGGACGCCCUGAGU
CAGGAUCAGCUGGAACAGAUGAUUCUGACCGAGGAACUGCAGGCUAUCCGGC
AGGCCAUUCAGGGGGAGCUGGAACUGCGGAGAAAGACCGACGCCGCCAUCA
GAGAGAAGCUGCAGGAACACAUGACCAGCAACAAGACCACCAAGUACUUCAAC
CAGCUGAUUCUGCGCCUGCAGAAAGAAAAGACCAACAUGAUGACACACCUGA
GCAAGAUCAACGGCGACAUUGCCCAGACCACCCUGGACAUCACCCACACCAG
CAGCAGACUGGACGCCCACCAGAAAACCCUGGUGGAACUGGACCAGGAUGUG
AAGAAAGUGAACGAGCUGAUCACCAACAGCCAGAGCGAGAUCAGCCGGCGGA
CCAUCCUGAUCGAGAGAAAGCAGGGCCUGAUCAACUUCCUGAACAAACAGCU
GGAAAGAAUGGUGUCCGAGCUGGGCGGCGAGGAAGUGGGACCUCUGGAACU
GGAAAUCAAGCGGCUGAGCAAGCUGAUCGACGAGCACGACGGCAAGGCCGUG
CAGGCUCAAGUGACAUGGCUGCGGCUGCAGCAGGAAAUGGUCAAAGUGACCC
AGGAACAGGAAGAACAGCUGGCCUCCCUGGACGCCAGCAAGAAAGAACUGCA
CAUCAUGGAACAGAAAAAGCUGCGGGUGGAAAGCAAGAUCGAGCAGGAAAAA
AAAGAACAGAAAGAAAUCGAGCACCACAUGAAGGACCUGGACAACGACCUGAA
GAAACUGAAUAUGCUGAUGAACAAGAACCGCUGCUCCAGCGAAGAACUGGAA
CAGAACAACAGAGUGACCGAGAACGAGUUCGUGCGGAGCCUGAAGGCCAGCG
AGCGGGAAACCAUCAAGAUGCAGGACAAGCUGAACCAGCUGUCCGAGGAAAA
AGCCACACUGCUGAACCAGCUGGUGGAAGCCGAGCACCAGAUCAUGCUGUGG
GAGAAGAAGAUCCAGCUGGCCAAAGAAAUGCGGAGCAGCGUGGACAGCGAGA
UCGGCCAGACCGAAAUCAGAGCCAUGAAGGGCGAGAUCCACCGGAUGAAAGU
GCGGCUGGGACAGCUGCUGAAACAGCAGGAAAAGAUGAUCCGGGCCAUGGAA
CUGGCCGUGGCCAGACGGGAAACCGUGACAACCCAGGCUGAGGGCCAGCGG
AAGAUGGACAGAAAGGCCCUGACCCGGACCGACUUCCACCACAAGCAGCUGG
AACUGAGGCGGAAGAUCCGGGACGUGCGGAAGGCCACCGAUGAGUGCACAAA
GACAGUGCUGGAACUGGAAGAGACACAGCGGAACGUGUCCUCCAGCCUGCUG
GAAAAACAGGAAAAGCUGAGCGUGAUCCAGGCCGACUUCGACACCCUGGAAG
CUGACCUGACAAGACUGGGAGCCCUGAAAAGACAGAACCUGUCCGAGAUCGU
GGCACUGCAGACCCGGCUGAAACAUCUGCAGGCUGUGAAAGAGGGACGCUAC
GUGUUCCUGUUCAGAUCCAAGCAGUCUCUGGUGCUGGAAAGACAGCGGCUG
GACAAGCGGCUGGCACUGAUUGCCACCAUCCUGGAUAGAGUGCGCGACGAG
UACCCACAGUUCCAGGAAGCACUGCACAAGGUGUCCCAGAUGAUCGCCAACA
AGCUGGAAUCCCCUGGCCCCAGCUGAUUCG-polyA
**SEQ ID NO: 7**
   **Sequence with hAg 5' UTR but without 3' UTR (ETH031T08):**
   Part of the T7 promoter, **Ethris' minimal 5' UTR****,** hAg *5'UTR*, ***Kozak element,*** start codon (AUG), codon optimized sequence encoding a functional version of a human CCDC40 protein, stop codon (UGA), part of restriction site for BstBI, **^{polyA}: tail of "A" produced via post polyadenylation of mRNA** 
GGGAGACUCUUCUGGUCCCCACAGACUCAGAGAGAACGCCACCAUGGCUGAACC
UGGCGGAGCCGCCGGAAGAUCCCACCCUGAAGAUGGCUCUGCCAGCGAGGG
CGAGAAAGAGGGCAACAACGAGAGCCACAUGGUGUCCCCCCCAGAGAAGGAC
GACGGCCAGAAAGGCGAAGAGGCCGUGGGCUCUACCGAGCACCCUGAGGAA
GUGACCACACAGGCCGAGGCCGCCAUUGAAGAGGGCGAGGUGGAAACAGAG
GGCGAAGCCGCUGUGGAAGGCGAAGAGGAAGCCGUGUCUUACGGCGACGCC
GAGAGCGAGGAAGAGUACUACACCGAGACAAGCAGCCCCGAGGGCCAGA
UCUCUGCCGCCGAUACCACCUACCCCUACUUCAGCCCCCCUCAGGAACUGCC
UGGGGAAGAGGCCUACGAUAGCGUGUCCGGCGAAGCUGGCCUGCAGGGCUU
UCAGCAGGAAGCCACAGGCCCUCCCGAGAGCCGGGAAAGAAGAGUGACAAGC
CCCGAGCCUAGCCACGGCGUGCUGGGACCAUCUGAGCAGAUGGGCCAAGUG
ACCUCUGGCCCUGCUGUGGGCAGACUGACAGGCAGCACAGAGGAACCUCAG
GGCCAGGUGCUGCCUAUGGGAGUGCAGCACCGGUUCAGACUGAGCCACGGC
AGCGACAUCGAGAGCAGCGACCUGGAAGAGUUCGUCAGCCAGGAACCCGUGA
UCCCUCCUGGCGUGCCAGAUGCCCAUCCCAGGGAAGGCGAUCUGCCCGUGU
UCCAGGACCAGAUCCAGCAGCCCUCUACCGAAGAGGGGGCUAUGGCCGAGAG
AGUGGAAAGCGAGGGCUCCGACGAAGAAGCCGAGGACGAGGGAUCUCAGCU
GGUGGUGCUGGACCCCGACCACCCUCUGAUGGUGCGGUUUCAGGCCGCCCU
GAAGAACUACCUGAACCGGCAGAUCGAGAAGCUGAAACUGGACCUGCAGGAA
CUGGUGGUGGCCACAAAGCAGAGCAGAGCCCAGAGACAGGAACUGGGCGUG
AACCUGUACGAGGUGCAGCAGCAUCUGGUGCAUCUGCAGAAGCUGCUGGAAA
AGAGCCACGACCGGCACGCCAUGGCCAGCUCUGAGCGCAGACAGAAAGAGGA
AGAACUGCAGGCCGCCAGAGCCCUGUACACCAAGACAUGCGCCGCUGCCAAC
GAGGAACGGAAGAAGCUGGCUGCCCUGCAGACCGAGAUGGAAAACCUGGCUC
UGCACCUGUUCUACAUGCAGAAUAUCGACCAGGACAUGCGGGACGACAUCAG
AGUGAUGACCCAGGUCGUGAAGAAGGCCGAGACAGAGAGAAUCCGGGCCGAG
AUUGAGAAGAAAAAGCAGGACCUGUACGUGGACCAGCUGACCACCAGGGCCC
AGCAGCUGGAAGAGGAUAUCGCCCUGUUCGAGGCCCAGUACCUGGCCCAGG
CCGAAGAUACCCGGAUCCUGAGAAAGGCCGUGUCCGAGGCCUGCACCGAGAU
CGAUGCCAUCAGCGUGGAAAAGCGGCGGAUCAUGCAGCAGUGGGCCAGCAG
CCUCGUGGGCAUGAAGCACAGAGAUGAGGCCCACCGGGCCGUGCUGGAAGC
UCUGAGAGGCUGUCAGCACCAGGCCAAGAGCACCGACGGCGAGAUCGAGGC
CUACAAGAAAUCCAUCAUGAAGGAAGAGGAAAAGAACGAGAAACUGGCCAGCA
UCCUGAACAGAACCGAAACCGAGGCCACCCUGCUGCAGAAACUGACCACCCA
GUGCCUGACCAAACAGGUGGCCCUGCAGUCCCAGUUCAACACCUACAGACUG
ACCCUGCAGGACACCGAGGACGCCCUGAGUCAGGAUCAGCUGGAACAGAUGA
UUCUGACCGAGGAACUGCAGGCUAUCCGGCAGGCCAUUCAGGGGGAGCUGG
AACUGCGGAGAAAGACCGACGCCGCCAUCAGAGAGAAGCUGCAGGAACACAU
GACCAGCAACAAGACCACCAAGUACUUCAACCAGCUGAUUCUGCGCCUGCAG
AAAGAAAAGACCAACAUGAUGACACACCUGAGCAAGAUCAACGGCGACAUUGC
CCAGACCACCCUGGACAUCACCCACACCAGCAGCAGACUGGACGCCCACCAG
AAAACCCUGGUGGAACUGGACCAGGAUGUGAAGAAAGUGAACGAGCUGAUCA
CCAACAGCCAGAGCGAGAUCAGCCGGCGGACCAUCCUGAUCGAGAGAAAGCA
GGGCCUGAUCAACUUCCUGAACAAACAGCUGGAAAGAAUGGUGUCCGAGCUG
GGCGGCGAGGAAGUGGGACCUCUGGAACUGGAAAUCAAGCGGCUGAGCAAG
 
CUGAUCGACGAGCACGACGGCAAGGCCGUGCAGGCUCAAGUGACAUGGCUG
CGGCUGCAGCAGGAAAUGGUCAAAGUGACCCAGGAACAGGAAGAACAGCUGG
CCUCCCUGGACGCCAGCAAGAAAGAACUGCACAUCAUGGAACAGAAAAAGCU
GCGGGUGGAAAGCAAGAUCGAGCAGGAAAAAAAAGAACAGAAAGAAAUCGAGC
ACCACAUGAAGGACCUGGACAACGACCUGAAGAAACUGAAUAUGCUGAUGAAC
AAGAACCGCUGCUCCAGCGAAGAACUGGAACAGAACAACAGAGUGACCGAGA
ACGAGUUCGUGCGGAGCCUGAAGGCCAGCGAGCGGGAAACCAUCAAGAUGCA
GGACAAGCUGAACCAGCUGUCCGAGGAAAAAGCCACACUGCUGAACCAGCUG
GUGGAAGCCGAGCACCAGAUCAUGCUGUGGGAGAAGAAGAUCCAGCUGGCCA
AAGAAAUGCGGAGCAGCGUGGACAGCGAGAUCGGCCAGACCGAAAUCAGAGC
CAUGAAGGGCGAGAUCCACCGGAUGAAAGUGCGGCUGGGACAGCUGCUGAAA
CAGCAGGAAAAGAUGAUCCGGGCCAUGGAACUGGCCGUGGCCAGACGGGAAA
CCGUGACAACCCAGGCUGAGGGCCAGCGGAAGAUGGACAGAAAGGCCCUGAC
CCGGACCGACUUCCACCACAAGCAGCUGGAACUGAGGCGGAAGAUCCGGGAC
GUGCGGAAGGCCACCGAUGAGUGCACAAAGACAGUGCUGGAACUGGAAGAGA
CACAGCGGAACGUGUCCUCCAGCCUGCUGGAAAAACAGGAAAAGCUGAGCGU
GAUCCAGGCCGACUUCGACACCCUGGAAGCUGACCUGACAAGACUGGGAGCC
CUGAAAAGACAGAACCUGUCCGAGAUCGUGGCACUGCAGACCCGGCUGAAAC
AUCUGCAGGCUGUGAAAGAGGGACGCUACGUGUUCCUGUUCAGAUCCAAGCA
GUCUCUGGUGCUGGAAAGACAGCGGCUGGACAAGCGGCUGGCACUGAUUGC
CACCAUCCUGGAUAGAGUGCGCGACGAGUACCCACAGUUCCAGGAAGCACUG
CACAAGGUGUCCCAGAUGAUCGCCAACAAGCUGGAAUCCCCUGGCCCCAGCU
GAUUCG-polyA
**SEQ ID NO: 8**
   **Sequence with human CMV IE9 5' UTR and human Growth hormone 3' UTR (ETH031T10):**
   Part of the T7 promoter, **Ethris' minimal 5' UTR**, human CMV IE9 *5' UTR**, **Kozak element,*** start codon (AUG), codon optimized sequence encoding a functional version of a human CCDC40 protein, stop codon (UGA), human Growth hormone **3'** ***UTR****,* part of restriction site for BstBI, **^{polyA}: tail of "A" produced via post polyadenylation of mRNA** 
GGGAGACCAGAUCGCCUGGAGACGCCAUCCACGCUGUUUUGACCUCCAUAGAAG
ACACCGGGACCGAUCCAGCCUCCGCGGCCGGGAACGGUGCAUUGGAACGCG
GAUUCCCCGUGCCAAGAGUGACUCACCGUCCUUGACACGGCCACCAUGGCUG
AACCUGGCGGAGCCGCCGGAAGAUCCCACCCUGAAGAUGGCUCUGCCAGCGA
GGGCGAGAAAGAGGGCAACAACGAGAGCCACAUGGUGUCCCCCCCAGAGAAG
GACGACGGCCAGAAAGGCGAAGAGGCCGUGGGCUCUACCGAGCACCCUGAG
GAAGUGACCACACAGGCCGAGGCCGCCAUUGAAGAGGGCGAGGUGGAAACAG
AGGGCGAAGCCGCUGUGGAAGGCGAAGAGGAAGCCGUGUCUUACGGCGACG
CCGAGAGCGAGGAAGAGUACUACUACACCGAGACAAGCAGCCCCGAGGGCCA
 
GAUCUCUGCCGCCGAUACCACCUACCCCUACUUCAGCCCCCCUCAGGAACUG
CCUGGGGAAGAGGCCUACGAUAGCGUGUCCGGCGAAGCUGGCCUGCAGGGC
UUUCAGCAGGAAGCCACAGGCCCUCCCGAGAGCCGGGAAAGAAGAGUGACAA
GCCCCGAGCCUAGCCACGGCGUGCUGGGACCAUCUGAGCAGAUGGGCCAAG
UGACCUCUGGCCCUGCUGUGGGCAGACUGACAGGCAGCACAGAGGAACCUCA
GGGCCAGGUGCUGCCUAUGGGAGUGCAGCACCGGUUCAGACUGAGCCACGG
CAGCGACAUCGAGAGCAGCGACCUGGAAGAGUUCGUCAGCCAGGAACCCGUG
AUCCCUCCUGGCGUGCCAGAUGCCCAUCCCAGGGAAGGCGAUCUGCCCGUG
UUCCAGGACCAGAUCCAGCAGCCCUCUACCGAAGAGGGGGCUAUGGCCGAGA
GAGUGGAAAGCGAGGGCUCCGACGAAGAAGCCGAGGACGAGGGAUCUCAGC
UGGUGGUGCUGGACCCCGACCACCCUCUGAUGGUGCGGUUUCAGGCCGCCC
UGAAGAACUACCUGAACCGGCAGAUCGAGAAGCUGAAACUGGACCUGCAGGA
ACUGGUGGUGGCCACAAAGCAGAGCAGAGCCCAGAGACAGGAACUGGGCGU
GAACCUGUACGAGGUGCAGCAGCAUCUGGUGCAUCUGCAGAAGCUGCUGGAA
AAGAGCCACGACCGGCACGCCAUGGCCAGCUCUGAGCGCAGACAGAAAGAGG
AAGAACUGCAGGCCGCCAGAGCCCUGUACACCAAGACAUGCGCCGCUGCCAA
CGAGGAACGGAAGAAGCUGGCUGCCCUGCAGACCGAGAUGGAAAACCUGGCU
CUGCACCUGUUCUACAUGCAGAAUAUCGACCAGGACAUGCGGGACGACAUCA
GAGUGAUGACCCAGGUCGUGAAGAAGGCCGAGACAGAGAGAAUCCGGGCCGA
GAUUGAGAAGAAAAAGCAGGACCUGUACGUGGACCAGCUGACCACCAGGGCC
CAGCAGCUGGAAGAGGAUAUCGCCCUGUUCGAGGCCCAGUACCUGGCCCAG
GCCGAAGAUACCCGGAUCCUGAGAAAGGCCGUGUCCGAGGCCUGCACCGAGA
UCGAUGCCAUCAGCGUGGAAAAGCGGCGGAUCAUGCAGCAGUGGGCCAGCA
GCCUCGUGGGCAUGAAGCACAGAGAUGAGGCCCACCGGGCCGUGCUGGAAG
CUCUGAGAGGCUGUCAGCACCAGGCCAAGAGCACCGACGGCGAGAUCGAGG
CCUACAAGAAAUCCAUCAUGAAGGAAGAGGAAAAGAACGAGAAACUGGCCAGC
AUCCUGAACAGAACCGAAACCGAGGCCACCCUGCUGCAGAAACUGACCACCC
AGUGCCUGACCAAACAGGUGGCCCUGCAGUCCCAGUUCAACACCUACAGACU
GACCCUGCAGGACACCGAGGACGCCCUGAGUCAGGAUCAGCUGGAACAGAUG
AUUCUGACCGAGGAACUGCAGGCUAUCCGGCAGGCCAUUCAGGGGGAGCUG
GAACUGCGGAGAAAGACCGACGCCGCCAUCAGAGAGAAGCUGCAGGAACACA
UGACCAGCAACAAGACCACCAAGUACUUCAACCAGCUGAUUCUGCGCCUGCA
GAAAGAAAAGACCAACAUGAUGACACACCUGAGCAAGAUCAACGGCGACAUUG
CCCAGACCACCCUGGACAUCACCCACACCAGCAGCAGACUGGACGCCCACCA
GAAAACCCUGGUGGAACUGGACCAGGAUGUGAAGAAAGUGAACGAGCUGAUC
ACCAACAGCCAGAGCGAGAUCAGCCGGCGGACCAUCCUGAUCGAGAGAAAGC
AGGGCCUGAUCAACUUCCUGAACAAACAGCUGGAAAGAAUGGUGUCCGAGCU
GGGCGGCGAGGAAGUGGGACCUCUGGAACUGGAAAUCAAGCGGCUGAGCAA
GCUGAUCGACGAGCACGACGGCAAGGCCGUGCAGGCUCAAGUGACAUGGCU
GCGGCUGCAGCAGGAAAUGGUCAAAGUGACCCAGGAACAGGAAGAACAGCUG
GCCUCCCUGGACGCCAGCAAGAAAGAACUGCACAUCAUGGAACAGAAAAAGC
UGCGGGUGGAAAGCAAGAUCGAGCAGGAAAAAAAAGAACAGAAAGAAAUCGAG
CACCACAUGAAGGACCUGGACAACGACCUGAAGAAACUGAAUAUGCUGAUGAA
CAAGAACCGCUGCUCCAGCGAAGAACUGGAACAGAACAACAGAGUGACCGAG
AACGAGUUCGUGCGGAGCCUGAAGGCCAGCGAGCGGGAAACCAUCAAGAUGC
 
AGGACAAGCUGAACCAGCUGUCCGAGGAAAAAGCCACACUGCUGAACCAGCU
GGUGGAAGCCGAGCACCAGAUCAUGCUGUGGGAGAAGAUCCAGCUGGC
CAAAGAAAUGCGGAGCAGCGUGGACAGCGAGAUCGGCCAGACCGAAAUCAGA
GCCAUGAAGGGCGAGAUCCACCGGAUGAAAGUGCGGCUGGGACAGCUGCUG
AAACAGCAGGAAAAGAUGAUCCGGGCCAUGGAACUGGCCGUGGCCAGACGGG
AAACCGUGACAACCCAGGCUGAGGGCCAGCGGAAGAUGGACAGAAAGGCCCU
GACCCGGACCGACUUCCACCACAAGCAGCUGGAACUGAGGCGGAAGAUCCGG
GACGUGCGGAAGGCCACCGAUGAGUGCACAAAGACAGUGCUGGAACUGGAAG
AGACACAGCGGAACGUGUCCUCCAGCCUGCUGGAAAAACAGGAAAAGCUGAG
CGUGAUCCAGGCCGACUUCGACACCCUGGAAGCUGACCUGACAAGACUGGGA
GCCCUGAAAAGACAGAACCUGUCCGAGAUCGUGGCACUGCAGACCCGGCUGA
AACAUCUGCAGGCUGUGAAAGAGGGACGCUACGUGUUCCUGUUCAGAUCCAA
GCAGUCUCUGGUGCUGGAAAGACAGCGGCUGGACAAGCGGCUGGCACUGAU
UGCCACCAUCCUGGAUAGAGUGCGCGACGAGUACCCACAGUUCCAGGAAGCA
CUGCACAAGGUGUCCCAGAUGAUCGCCAACAAGCUGGAAUCCCCUGGCCCCA
GCUGACGGGUGGCAUCCCUGUGACCCCUCCCCAGUGCCUCUCCUGGCCCUG
GAAGUUGCCACUCCAGUGCCCACCAGCCUUGUCCUAAUAAAAUUAAGUUGC
AUCUUCG-polyA
**SEQ ID NO: 9**
   **ETH031T28: N terminal EGFP tag-CCDC40**
   Part of the T7 promoter, **Ethris' minimal 5' UTR,** ***Kozak element,*** start codon (AUG), sequence encoding an *EGFP* protein, ^{G4S spacer}, codon optimized sequence encoding a functional version of a human CCDC40 protein, stop codon (UGA), part of restriction site for BstBI, ^{poly(A) tail} 
GGGAGACGCCACCAUGGUGAGCAAGGGCGAGGAGCUGUUCACCGGGGUGGUGC
CCAUCCUGGUCGAGCUGGACGGCGACGUAAACGGCCACAAGUUCAGCGUGU
CCGGCGAGGGCGAGGGCGAUGCCACCUACGGCAAGCUGACCCUGAAGUUCA
UCUGCACCACCGGCAAGCUGCCCGUGCCCUGGCCCACCCUCGUGACCACCCU
GACCUACGGCGUGCAGUGCUUCAGCCGCUACCCCGACCACAUGAAGCAGCAC
GACUUCUUCAAGUCCGCCAUGCCCGAAGGCUACGUCCAGGAGCGCACCAUCU
UCUUCAAGGACGACGGCAACUACAAGACCCGCGCCGAGGUGAAGUUCGAGGG
CGACACCCUGGUGAACCGCAUCGAGCUGAAGGGCAUCGACUUCAAGGAGGAC
GGCAACAUCCUGGGGCACAAGCUGGAGUACAACUACAACAGCCACAACGUCU
AUAUCAUGGCCGACAAGCAGAAGAACGGCAUCAAGGUGAACUUCAAGAUCCG
CCACAACAUCGAGGACGGCAGCGUGCAGCUCGCCGACCACUACCAGCAGAAC
ACCCCCAUCGGCGACGGCCCCGUGCUGCUGCCCGACAACCACUACCUGAGCA
CCCAGUCCGCCCUGAGCAAAGACCCCAACGAGAAGCGCGAUCACAUGGUCCU
GCUGGAGUUCGUGACCGCCGCCGGGAUCACUCUCGGCAUGGACGAGCUGUA
CAAGGGCGGAGGGGGCAGCGCUGAACCUGGCGGAGCCGCCGGAAGAUCCCACCCUG
AAGAUGGCUCUGCCAGCGAGGGCGAGAAAGAGGGCAACAACGAGAGCCACAU
 
GGUGUCCCCCCCAGAGAAGGACGACGGCCAGAAAGGCGAAGAGGCCGUGGG
CUCUACCGAGCACCCUGAGGAAGUGACCACACAGGCCGAGGCCGCCAUUGAA
GAGGGCGAGGUGGAAACAGAGGGCGAAGCCGCUGUGGAAGGCGAAGAGGAA
GCCGUGUCUUACGGCGACGCCGAGAGCGAGGAAGAGUACUACACCGAGA
CAAGCAGCCCCGAGGGCCAGAUCUCUGCCGCCGAUACCACCUACCCCUACUU
CAGCCCCCCUCAGGAACUGCCUGGGGAAGAGGCCUACGAUAGCGUGUCCGG
CGAAGCUGGCCUGCAGGGCUUUCAGCAGGAAGCCACAGGCCCUCCCGAGAG
CCGGGAAAGAAGAGUGACAAGCCCCGAGCCUAGCCACGGCGUGCUGGGACCA
UCUGAGCAGAUGGGCCAAGUGACCUCUGGCCCUGCUGUGGGCAGACUGACA
GGCAGCACAGAGGAACCUCAGGGCCAGGUGCUGCCUAUGGGAGUGCAGCAC
CGGUUCAGACUGAGCCACGGCAGCGACAUCGAGAGCAGCGACCUGGAAGAGU
UCGUCAGCCAGGAACCCGUGAUCCCUCCUGGCGUGCCAGAUGCCCAUCCCAG
GGAAGGCGAUCUGCCCGUGUUCCAGGACCAGAUCCAGCAGCCCUCUACCGAA
GAGGGGGCUAUGGCCGAGAGAGUGGAAAGCGAGGGCUCCGACGAAGAAGCC
GAGGACGAGGGAUCUCAGCUGGUGGUGCUGGACCCCGACCACCCUCUGAUG
GUGCGGUUUCAGGCCGCCCUGAAGAACUACCUGAACCGGCAGAUCGAGAAGC
UGAAACUGGACCUGCAGGAACUGGUGGUGGCCACAAAGCAGAGCAGAGCCCA
GAGACAGGAACUGGGCGUGAACCUGUACGAGGUGCAGCAGCAUCUGGUGCA
UCUGCAGAAGCUGCUGGAAAAGAGCCACGACCGGCACGCCAUGGCCAGCUCU
GAGCGCAGACAGAAAGAGGAAGAACUGCAGGCCGCCAGAGCCCUGUACACCA
AGACAUGCGCCGCUGCCAACGAGGAACGGAAGAAGCUGGCUGCCCUGCAGAC
CGAGAUGGAAAACCUGGCUCUGCACCUGUUCUACAUGCAGAAUAUCGACCAG
GACAUGCGGGACGACAUCAGAGUGAUGACCCAGGUCGUGAAGAAGGCCGAGA
CAGAGAGAAUCCGGGCCGAGAUUGAGAAGAAAAAGCAGGACCUGUACGUGGA
CCAGCUGACCACCAGGGCCCAGCAGCUGGAAGAGGAUAUCGCCCUGUUCGAG
GCCCAGUACCUGGCCCAGGCCGAAGAUACCCGGAUCCUGAGAAAGGCCGUGU
CCGAGGCCUGCACCGAGAUCGAUGCCAUCAGCGUGGAAAAGCGGCGGAUCAU
GCAGCAGUGGGCCAGCAGCCUCGUGGGCAUGAAGCACAGAGAUGAGGCCCA
CCGGGCCGUGCUGGAAGCUCUGAGAGGCUGUCAGCACCAGGCCAAGAGCAC
CGACGGCGAGAUCGAGGCCUACAAGAAAUCCAUCAUGAAGGAAGAGGAAAAG
AACGAGAAACUGGCCAGCAUCCUGAACAGAACCGAAACCGAGGCCACCCUGC
UGCAGAAACUGACCACCCAGUGCCUGACCAAACAGGUGGCCCUGCAGUCCCA
GUUCAACACCUACAGACUGACCCUGCAGGACACCGAGGACGCCCUGAGUCAG
GAUCAGCUGGAACAGAUGAUUCUGACCGAGGAACUGCAGGCUAUCCGGCAGG
CCAUUCAGGGGGAGCUGGAACUGCGGAGAAAGACCGACGCCGCCAUCAGAGA
GAAGCUGCAGGAACACAUGACCAGCAACAAGACCACCAAGUACUUCAACCAGC
UGAUUCUGCGCCUGCAGAAAGAAAAGACCAACAUGAUGACACACCUGAGCAA
GAUCAACGGCGACAUUGCCCAGACCACCCUGGACAUCACCCACACCAGCAGC
AGACUGGACGCCCACCAGAAAACCCUGGUGGAACUGGACCAGGAUGUGAAGA
AAGUGAACGAGCUGAUCACCAACAGCCAGAGCGAGAUCAGCCGGCGGACCAU
CCUGAUCGAGAGAAAGCAGGGCCUGAUCAACUUCCUGAACAAACAGCUGGAA
AGAAUGGUGUCCGAGCUGGGCGGCGAGGAAGUGGGACCUCUGGAACUGGAA
AUCAAGCGGCUGAGCAAGCUGAUCGACGAGCACGACGGCAAGGCCGUGCAG
GCUCAAGUGACAUGGCUGCGGCUGCAGCAGGAAAUGGUCAAAGUGACCCAGG
AACAGGAAGAACAGCUGGCCUCCCUGGACGCCAGCAAGAAAGAACUGCACAU
 
CAUGGAACAGAAAAAGCUGCGGGUGGAAAGCAAGAUCGAGCAGGAAAAAAAA
GAACAGAAAGAAAUCGAGCACCACAUGAAGGACCUGGACAACGACCUGAAGAA
ACUGAAUAUGCUGAUGAACAAGAACCGCUGCUCCAGCGAAGAACUGGAACAG
AACAACAGAGUGACCGAGAACGAGUUCGUGCGGAGCCUGAAGGCCAGCGAGC
GGGAAACCAUCAAGAUGCAGGACAAGCUGAACCAGCUGUCCGAGGAAAAAGC
CACACUGCUGAACCAGCUGGUGGAAGCCGAGCACCAGAUCAUGCUGUGGGAG
AAGAAGAUCCAGCUGGCCAAAGAAAUGCGGAGCAGCGUGGACAGCGAGAUCG
GCCAGACCGAAAUCAGAGCCAUGAAGGGCGAGAUCCACCGGAUGAAAGUGCG
GCUGGGACAGCUGCUGAAACAGCAGGAAAAGAUGAUCCGGGCCAUGGAACUG
GCCGUGGCCAGACGGGAAACCGUGACAACCCAGGCUGAGGGCCAGCGGAAG
AUGGACAGAAAGGCCCUGACCCGGACCGACUUCCACCACAAGCAGCUGGAAC
UGAGGCGGAAGAUCCGGGACGUGCGGAAGGCCACCGAUGAGUGCACAAAGA
CAGUGCUGGAACUGGAAGAGACACAGCGGAACGUGUCCUCCAGCCUGCUGGA
AAAACAGGAAAAGCUGAGCGUGAUCCAGGCCGACUUCGACACCCUGGAAGCU
GACCUGACAAGACUGGGAGCCCUGAAAAGACAGAACCUGUCCGAGAUCGUGG
CACUGCAGACCCGGCUGAAACAUCUGCAGGCUGUGAAAGAGGGACGCUACGU
GUUCCUGUUCAGAUCCAAGCAGUCUCUGGUGCUGGAAAGACAGCGGCUGGA
CAAGCGGCUGGCACUGAUUGCCACCAUCCUGGAUAGAGUGCGCGACGAGUAC
CCACAGUUCCAGGAAGCACUGCACAAGGUGUCCCAGAUGAUCGCCAACAAGC
UGGAAUCCCCUGGCCCCAGCUGAUUCGAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAA
**SEQ ID NO: 10**
   **ETH031T30: C terminal EGFP tag-CCDC40**
   Part of the T7 promoter, **Ethris' minimal 5' UTR****,** ***Kozak element,*** start codon (AUG), codon optimized sequence encoding a functional version of a human CCDC40 protein, ^{G4S} ^{spacer}, sequence encoding an *EGFP* protein, stop codon (UGA), part of restriction site for BstBI, ^{poly(A) tail} 
GGGAGACGCCACCAUGGCUGAACCUGGCGGAGCCGCCGGAAGAUCCCACCCUG
AAGAUGGCUCUGCCAGCGAGGGCGAGAAAGAGGGCAACAACGAGAGCCACAU
GGUGUCCCCCCCAGAGAAGGACGACGGCCAGAAAGGCGAAGAGGCCGUGGG
CUCUACCGAGCACCCUGAGGAAGUGACCACACAGGCCGAGGCCGCCAUUGAA
GAGGGCGAGGUGGAAACAGAGGGCGAAGCCGCUGUGGAAGGCGAAGAGGAA
GCCGUGUCUUACGGCGACGCCGAGAGCGAGGAAGAGUACUACACCGAGA
CAAGCAGCCCCGAGGGCCAGAUCUCUGCCGCCGAUACCACCUACCCCUACUU
CAGCCCCCCUCAGGAACUGCCUGGGGAAGAGGCCUACGAUAGCGUGUCCGG
CGAAGCUGGCCUGCAGGGCUUUCAGCAGGAAGCCACAGGCCCUCCCGAGAG
CCGGGAAAGAAGAGUGACAAGCCCCGAGCCUAGCCACGGCGUGCUGGGACCA
UCUGAGCAGAUGGGCCAAGUGACCUCUGGCCCUGCUGUGGGCAGACUGACA
GGCAGCACAGAGGAACCUCAGGGCCAGGUGCUGCCUAUGGGAGUGCAGCAC
CGGUUCAGACUGAGCCACGGCAGCGACAUCGAGAGCAGCGACCUGGAAGAGU
 
UCGUCAGCCAGGAACCCGUGAUCCCUCCUGGCGUGCCAGAUGCCCAUCCCAG
GGAAGGCGAUCUGCCCGUGUUCCAGGACCAGAUCCAGCAGCCCUCUACCGAA
GAGGGGGCUAUGGCCGAGAGAGUGGAAAGCGAGGGCUCCGACGAAGAAGCC
GAGGACGAGGGAUCUCAGCUGGUGGUGCUGGACCCCGACCACCCUCUGAUG
GUGCGGUUUCAGGCCGCCCUGAAGAACUACCUGAACCGGCAGAUCGAGAAGC
UGAAACUGGACCUGCAGGAACUGGUGGUGGCCACAAAGCAGAGCAGAGCCCA
GAGACAGGAACUGGGCGUGAACCUGUACGAGGUGCAGCAGCAUCUGGUGCA
UCUGCAGAAGCUGCUGGAAAAGAGCCACGACCGGCACGCCAUGGCCAGCUCU
GAGCGCAGACAGAAAGAGGAAGAACUGCAGGCCGCCAGAGCCCUGUACACCA
AGACAUGCGCCGCUGCCAACGAGGAACGGAAGAAGCUGGCUGCCCUGCAGAC
CGAGAUGGAAAACCUGGCUCUGCACCUGUUCUACAUGCAGAAUAUCGACCAG
GACAUGCGGGACGACAUCAGAGUGAUGACCCAGGUCGUGAAGAAGGCCGAGA
CAGAGAGAAUCCGGGCCGAGAUUGAGAAGAAAAAGCAGGACCUGUACGUGGA
CCAGCUGACCACCAGGGCCCAGCAGCUGGAAGAGGAUAUCGCCCUGUUCGAG
GCCCAGUACCUGGCCCAGGCCGAAGAUACCCGGAUCCUGAGAAAGGCCGUGU
CCGAGGCCUGCACCGAGAUCGAUGCCAUCAGCGUGGAAAAGCGGCGGAUCAU
GCAGCAGUGGGCCAGCAGCCUCGUGGGCAUGAAGCACAGAGAUGAGGCCCA
CCGGGCCGUGCUGGAAGCUCUGAGAGGCUGUCAGCACCAGGCCAAGAGCAC
CGACGGCGAGAUCGAGGCCUACAAGAAAUCCAUCAUGAAGGAAGAGGAAAAG
AACGAGAAACUGGCCAGCAUCCUGAACAGAACCGAAACCGAGGCCACCCUGC
UGCAGAAACUGACCACCCAGUGCCUGACCAAACAGGUGGCCCUGCAGUCCCA
GUUCAACACCUACAGACUGACCCUGCAGGACACCGAGGACGCCCUGAGUCAG
GAUCAGCUGGAACAGAUGAUUCUGACCGAGGAACUGCAGGCUAUCCGGCAGG
CCAUUCAGGGGGAGCUGGAACUGCGGAGAAAGACCGACGCCGCCAUCAGAGA
GAAGCUGCAGGAACACAUGACCAGCAACAAGACCACCAAGUACUUCAACCAGC
UGAUUCUGCGCCUGCAGAAAGAAAAGACCAACAUGAUGACACACCUGAGCAA
GAUCAACGGCGACAUUGCCCAGACCACCCUGGACAUCACCCACACCAGCAGC
AGACUGGACGCCCACCAGAAAACCCUGGUGGAACUGGACCAGGAUGUGAAGA
AAGUGAACGAGCUGAUCACCAACAGCCAGAGCGAGAUCAGCCGGCGGACCAU
CCUGAUCGAGAGAAAGCAGGGCCUGAUCAACUUCCUGAACAAACAGCUGGAA
AGAAUGGUGUCCGAGCUGGGCGGCGAGGAAGUGGGACCUCUGGAACUGGAA
AUCAAGCGGCUGAGCAAGCUGAUCGACGAGCACGACGGCAAGGCCGUGCAG
GCUCAAGUGACAUGGCUGCGGCUGCAGCAGGAAAUGGUCAAAGUGACCCAGG
AACAGGAAGAACAGCUGGCCUCCCUGGACGCCAGCAAGAAAGAACUGCACAU
CAUGGAACAGAAAAAGCUGCGGGUGGAAAGCAAGAUCGAGCAGGAAAAAAAA
GAACAGAAAGAAAUCGAGCACCACAUGAAGGACCUGGACAACGACCUGAAGAA
ACUGAAUAUGCUGAUGAACAAGAACCGCUGCUCCAGCGAAGAACUGGAACAG
AACAACAGAGUGACCGAGAACGAGUUCGUGCGGAGCCUGAAGGCCAGCGAGC
GGGAAACCAUCAAGAUGCAGGACAAGCUGAACCAGCUGUCCGAGGAAAAAGC
CACACUGCUGAACCAGCUGGUGGAAGCCGAGCACCAGAUCAUGCUGUGGGAG
AAGAAGAUCCAGCUGGCCAAAGAAAUGCGGAGCAGCGUGGACAGCGAGAUCG
GCCAGACCGAAAUCAGAGCCAUGAAGGGCGAGAUCCACCGGAUGAAAGUGCG
GCUGGGACAGCUGCUGAAACAGCAGGAAAAGAUGAUCCGGGCCAUGGAACUG
GCCGUGGCCAGACGGGAAACCGUGACAACCCAGGCUGAGGGCCAGCGGAAG
AUGGACAGAAAGGCCCUGACCCGGACCGACUUCCACCACAAGCAGCUGGAAC
 
UGAGGCGGAAGAUCCGGGACGUGCGGAAGGCCACCGAUGAGUGCACAAAGA
CAGUGCUGGAACUGGAAGAGACACAGCGGAACGUGUCCUCCAGCCUGCUGGA
AAAACAGGAAAAGCUGAGCGUGAUCCAGGCCGACUUCGACACCCUGGAAGCU
GACCUGACAAGACUGGGAGCCCUGAAAAGACAGAACCUGUCCGAGAUCGUGG
CACUGCAGACCCGGCUGAAACAUCUGCAGGCUGUGAAAGAGGGACGCUACGU
GUUCCUGUUCAGAUCCAAGCAGUCUCUGGUGCUGGAAAGACAGCGGCUGGA
CAAGCGGCUGGCACUGAUUGCCACCAUCCUGGAUAGAGUGCGCGACGAGUAC
CCACAGUUCCAGGAAGCACUGCACAAGGUGUCCCAGAUGAUCGCCAACAAGC
UGGAAUCCCCUGGCCCCAGCGGCGGAGGGGGCAGCGUGAGCAAGGGCGAGGAGCUG
UUCACCGGGGUGGUGCCCAUCCUGGUCGAGCUGGACGGCGACGUAAACGGC
CACAAGUUCAGCGUGUCCGGCGAGGGCGAGGGCGAUGCCACCUACGGCAAG
CUGACCCUGAAGUUCAUCUGCACCACCGGCAAGCUGCCCGUGCCCUGGCCCA
CCCUCGUGACCACCCUGACCUACGGCGUGCAGUGCUUCAGCCGCUACCCCGA
CCACAUGAAGCAGCACGACUUCUUCAAGUCCGCCAUGCCCGAAGGCUACGUC
CAGGAGCGCACCAUCUUCUUCAAGGACGACGGCAACUACAAGACCCGCGCCG
AGGUGAAGUUCGAGGGCGACACCCUGGUGAACCGCAUCGAGCUGAAGGGCA
UCGACUUCAAGGAGGACGGCAACAUCCUGGGGCACAAGCUGGAGUACAACUA
CAACAGCCACAACGUCUAUAUCAUGGCCGACAAGCAGAAGAACGGCAUCAAG
GUGAACUUCAAGAUCCGCCACAACAUCGAGGACGGCAGCGUGCAGCUCGCCG
ACCACUACCAGCAGAACACCCCCAUCGGCGACGGCCCCGUGCUGCUGCCCGA
CAACCACUACCUGAGCACCCAGUCCGCCCUGAGCAAAGACCCCAACGAGAAG
CGCGAUCACAUGGUCCUGCUGGAGUUCGUGACCGCCGCCGGGAUCACUCUC
GGCAUGGACGAGCUGUACAAGUGAUUCGAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAA
**SEQ ID NO: 11**
   **ETH031T26: N terminal HA Tag-CCDC40-T2A peptide-tdTomato with 5' and 3' CYBA UTRs**
   Part of the T7 promoter, **Ethris' minimal 5' UTR,** CYBA *5'* ***UTR******, Kozak element,*** start codon (AUG), *_{HA Tag,}* ^{G4S spacer}, codon optimized sequence encoding a functional version of a human CCDC40 protein,, **^{T2A peptide}****,** *tdTomato**,* stop codon (UGA), CYBA ***3' UTR***, part of restriction site for BstBI, ^{poly(A) **tail**} 
GGGAGACCGCGCCUAGCAGUGUCCCAGCCGGGUUCGUGUCGCCGCCACCAUGUA
CCCCUACGACGUGCCCGACUACGCCGGCGGAGGGGGCAGCGCUGAACCUGGCGGAGCCGCCGGA
AGAUCCCACCCUGAAGAUGGCUCUGCCAGCGAGGGCGAGAAAGAGGGCAACA
ACGAGAGCCACAUGGUGUCCCCCCCAGAGAAGGACGACGGCCAGAAAGGCGA
AGAGGCCGUGGGCUCUACCGAGCACCCUGAGGAAGUGACCACACAGGCCGA
GGCCGCCAUUGAAGAGGGCGAGGUGGAAACAGAGGGCGAAGCCGCUGUGGA
AGGCGAAGAGGAAGCCGUGUCUUACGGCGACGCCGAGAGCGAGGAAGAGUA
CUACUACACCGAGACAAGCAGCCCCGAGGGCCAGAUCUCUGCCGCCGAUACC
ACCUACCCCUACUUCAGCCCCCCUCAGGAACUGCCUGGGGAAGAGGCCUACG
 
AUAGCGUGUCCGGCGAAGCUGGCCUGCAGGGCUUUCAGCAGGAAGCCACAG
GCCCUCCCGAGAGCCGGGAAAGAAGAGUGACAAGCCCCGAGCCUAGCCACGG
CGUGCUGGGACCAUCUGAGCAGAUGGGCCAAGUGACCUCUGGCCCUGCUGU
GGGCAGACUGACAGGCAGCACAGAGGAACCUCAGGGCCAGGUGCUGCCUAU
GGGAGUGCAGCACCGGUUCAGACUGAGCCACGGCAGCGACAUCGAGAGCAG
CGACCUGGAAGAGUUCGUCAGCCAGGAACCCGUGAUCCCUCCUGGCGUGCC
AGAUGCCCAUCCCAGGGAAGGCGAUCUGCCCGUGUUCCAGGACCAGAUCCAG
CAGCCCUCUACCGAAGAGGGGGCUAUGGCCGAGAGAGUGGAAAGCGAGGGC
UCCGACGAAGAAGCCGAGGACGAGGGAUCUCAGCUGGUGGUGCUGGACCCC
GACCACCCUCUGAUGGUGCGGUUUCAGGCCGCCCUGAAGAACUACCUGAACC
GGCAGAUCGAGAAGCUGAAACUGGACCUGCAGGAACUGGUGGUGGCCACAAA
GCAGAGCAGAGCCCAGAGACAGGAACUGGGCGUGAACCUGUACGAGGUGCA
GCAGCAUCUGGUGCAUCUGCAGAAGCUGCUGGAAAAGAGCCACGACCGGCAC
GCCAUGGCCAGCUCUGAGCGCAGACAGAAAGAGGAAGAACUGCAGGCCGCCA
GAGCCCUGUACACCAAGACAUGCGCCGCUGCCAACGAGGAACGGAAGAAGCU
GGCUGCCCUGCAGACCGAGAUGGAAAACCUGGCUCUGCACCUGUUCUACAUG
CAGAAUAUCGACCAGGACAUGCGGGACGACAUCAGAGUGAUGACCCAGGUCG
UGAAGAAGGCCGAGACAGAGAGAAUCCGGGCCGAGAUUGAGAAGAAAAAGCA
GGACCUGUACGUGGACCAGCUGACCACCAGGGCCCAGCAGCUGGAAGAGGA
UAUCGCCCUGUUCGAGGCCCAGUACCUGGCCCAGGCCGAAGAUACCCGGAUC
CUGAGAAAGGCCGUGUCCGAGGCCUGCACCGAGAUCGAUGCCAUCAGCGUG
GAAAAGCGGCGGAUCAUGCAGCAGUGGGCCAGCAGCCUCGUGGGCAUGAAG
CACAGAGAUGAGGCCCACCGGGCCGUGCUGGAAGCUCUGAGAGGCUGUCAG
CACCAGGCCAAGAGCACCGACGGCGAGAUCGAGGCCUACAAGAAAUCCAUCA
UGAAGGAAGAGGAAAAGAACGAGAAACUGGCCAGCAUCCUGAACAGAACCGAA
ACCGAGGCCACCCUGCUGCAGAAACUGACCACCCAGUGCCUGACCAAACAGG
UGGCCCUGCAGUCCCAGUUCAACACCUACAGACUGACCCUGCAGGACACCGA
GGACGCCCUGAGUCAGGAUCAGCUGGAACAGAUGAUUCUGACCGAGGAACUG
CAGGCUAUCCGGCAGGCCAUUCAGGGGGAGCUGGAACUGCGGAGAAAGACC
GACGCCGCCAUCAGAGAGAAGCUGCAGGAACACAUGACCAGCAACAAGACCA
CCAAGUACUUCAACCAGCUGAUUCUGCGCCUGCAGAAAGAAAAGACCAACAUG
AUGACACACCUGAGCAAGAUCAACGGCGACAUUGCCCAGACCACCCUGGACA
UCACCCACACCAGCAGCAGACUGGACGCCCACCAGAAAACCCUGGUGGAACU
GGACCAGGAUGUGAAGAAAGUGAACGAGCUGAUCACCAACAGCCAGAGCGAG
AUCAGCCGGCGGACCAUCCUGAUCGAGAGAAAGCAGGGCCUGAUCAACUUCC
UGAACAAACAGCUGGAAAGAAUGGUGUCCGAGCUGGGCGGCGAGGAAGUGG
GACCUCUGGAACUGGAAAUCAAGCGGCUGAGCAAGCUGAUCGACGAGCACGA
CGGCAAGGCCGUGCAGGCUCAAGUGACAUGGCUGCGGCUGCAGCAGGAAAU
GGUCAAAGUGACCCAGGAACAGGAAGAACAGCUGGCCUCCCUGGACGCCAGC
AAGAAAGAACUGCACAUCAUGGAACAGAAAAAGCUGCGGGUGGAAAGCAAGAU
CGAGCAGGAAAAAAAAGAACAGAAAGAAAUCGAGCACCACAUGAAGGACCUGG
ACAACGACCUGAAGAAACUGAAUAUGCUGAUGAACAAGAACCGCUGCUCCAGC
GAAGAACUGGAACAGAACAACAGAGUGACCGAGAACGAGUUCGUGCGGAGCC
UGAAGGCCAGCGAGCGGGAAACCAUCAAGAUGCAGGACAAGCUGAACCAGCU
GUCCGAGGAAAAAGCCACACUGCUGAACCAGCUGGUGGAAGCCGAGCACCAG
 
AUCAUGCUGUGGAGAAGAUCCAGCUGGCCAAAGAAAUGCGGAGCAGCG
UGGACAGCGAGAUCGGCCAGACCGAAAUCAGAGCCAUGAAGGGCGAGAUCCA
CCGGAUGAAAGUGCGGCUGGGACAGCUGCUGAAACAGCAGGAAAAGAUGAUC
CGGGCCAUGGAACUGGCCGUGGCCAGACGGGAAACCGUGACAACCCAGGCU
GAGGGCCAGCGGAAGAUGGACAGAAAGGCCCUGACCCGGACCGACUUCCACC
ACAAGCAGCUGGAACUGAGGCGGAAGAUCCGGGACGUGCGGAAGGCCACCG
AUGAGUGCACAAAGACAGUGCUGGAACUGGAAGAGACACAGCGGAACGUGUC
CUCCAGCCUGCUGGAAAAACAGGAAAAGCUGAGCGUGAUCCAGGCCGACUUC
GACACCCUGGAAGCUGACCUGACAAGACUGGGAGCCCUGAAAAGACAGAACC
UGUCCGAGAUCGUGGCACUGCAGACCCGGCUGAAACAUCUGCAGGCUGUGAA
AGAGGGACGCUACGUGUUCCUGUUCAGAUCCAAGCAGUCUCUGGUGCUGGA
AAGACAGCGGCUGGACAAGCGGCUGGCACUGAUUGCCACCAUCCUGGAUAGA
GUGCGCGACGAGUACCCACAGUUCCAGGAAGCACUGCACAAGGUGUCCCAGA
UGAUCGCCAACAAGCUGGAAUCCCCUGGCCCCAGCGGCAGCGGCGAGGGCAGAGG
CAGCCUGCUGACCUGCGGCGACGUGGAGGAGAACCCCGGCCCCAUGGUGAGCAAGGGCGAGGAGG
UCAUCAAAGAGUUCAUGCGCUUCAAGGUGCGCAUGGAGGGCUCCAUGAACGG
CCACGAGUUCGAGAUCGAGGGCGAGGGCGAGGGCCGCCCCUACGAGGGCAC
CCAGACCGCCAAGCUGAAGGUGACCAAGGGCGGCCCCCUGCCCUUCGCCUG
GGACAUCCUGUCCCCCCAGUUCAUGUACGGCUCCAAGGCGUACGUGAAGCAC
CCCGCCGACAUCCCCGAUUACAAGAAGCUGUCCUUCCCCGAGGGCUUCAAGU
GGGAGCGCGUGAUGAACUUCGAGGACGGCGGUCUGGUGACCGUGACCCAGG
ACUCCUCCCUGCAGGACGGCACGCUGAUCUACAAGGUGAAGAUGCGCGGCAC
CAACUUCCCCCCCGACGGCCCCGUAAUGCAGAAGAAGACCAUGGGCUGGGAG
GCCUCCACCGAGCGCCUGUACCCCCGCGACGGCGUGCUGAAGGGCGAGAUC
CACCAGGCCCUGAAGCUGAAGGACGGCGGCCACUACCUGGUGGAGUUCAAGA
CCAUCUACAUGGCCAAGAAGCCCGUGCAACUGCCCGGCUACUACUACGUGGA
CACCAAGCUGGACAUCACCUCCCACAACGAGGACUACACCAUCGUGGAACAG
UACGAGCGCUCCGAGGGCCGCCACCACCUGUUCCUGGGGCAUGGCACCGGC
AGCACCGGCAGCGGCAGCUCCGGCACCGCCUCCUCCGAGGACAACAACAUGG
CCGUCAUCAAAGAGUUCAUGCGCUUCAAGGUGCGCAUGGAGGGCUCCAUGAA
CGGCCACGAGUUCGAGAUCGAGGGCGAGGGCGAGGGCCGCCCCUACGAGGG
CACCCAGACCGCCAAGCUGAAGGUGACCAAGGGCGGCCCCCUGCCCUUCGCC
UGGGACAUCCUGUCCCCCCAGUUCAUGUACGGCUCCAAGGCGUACGUGAAGC
ACCCCGCCGACAUCCCCGAUUACAAGAAGCUGUCCUUCCCCGAGGGCUUCAA
GUGGGAGCGCGUGAUGAACUUCGAGGACGGCGGUCUGGUGACCGUGACCCA
GGACUCCUCCCUGCAGGACGGCACGCUGAUCUACAAGGUGAAGAUGCGCGG
CACCAACUUCCCCCCCGACGGCCCCGUAAUGCAGAAGAAGACCAUGGGCUGG
GAGGCCUCCACCGAGCGCCUGUACCCCCGCGACGGCGUGCUGAAGGGCGAG
AUCCACCAGGCCCUGAAGCUGAAGGACGGCGGCCACUACCUGGUGGAGUUCA
AGACCAUCUACAUGGCCAAGAAGCCCGUGCAACUGCCCGGCUACUACUACGU
GGACACCAAGCUGGACAUCACCUCCCACAACGAGGACUACACCAUCGUGGAA
CAGUACGAGCGCUCCGAGGGCCGCCACCACCUGUUCCUGUACGGCAUGGAC
GAGCUGUACAAGUGACCUCGCCCCGGACCUGCCCUCCCGCCAGGUGCACCC
ACCUGCAAUAAAUGCAGCGAAGCCGGGAUUCGAAAAAAAAAAAAAAAAAAAAAAAAAAAAAA
 
AAAAAAAAAAAAAAAAAAAAAAAAAAAAAAAAAAAAAAAAAAAAAAAAAAAAAAAAAAAAAAAAAAAAAAAAAAAAAAAAAAAAAA

AAAA
**SEQ ID NO: 12**
   ETH047T02 (CCDC39 with Ethris' minimal UTR):
   Part of the T7 promoter, **Ethris' minimal 5' UTR,** ***Kozak element,*** start codon (AUG), codon optimized sequence encoding a functional version of a human CCDC39 protein, stop codon (UGA), part of restriction site for BstBI, ^{poly(A) **tail**} 
GGGAGACGCCACCAUGAGCAGCGAGUUUCUGGCCGAACUGCACUGGGAGGACG
GCUUCGCUAUUCCCGUGGCCAACGAGGAAAACAAGCUGCUGGAAGAUCAGCU
GAGCAAGCUGAAGGACGAGAGAGCCUCUCUGCAGGACGAGCUGAGAGAGUAC
GAGGAACGGAUCAACAGCAUGACCAGCCACUUCAAGAACGUGAAGCAAGAGC
UGAGCAUCACCCAGAGCCUGUGCAAGGCCAGAGAGAGAGAAACCGAGAGCGA
GGAACACUUCAAGGCUAUCGCCCAGCGCGAGCUGGGAAGAGUGAAGGAUGAG
AUCCAGCGGCUGGAAAACGAGAUGGCCAGCAUCCUGGAAAAGAAGUCCGACA
AAGAGAACGGCAUCUUCAAGGCCACACAGAAGCUGGACGGCCUGAAGUGCCA
GAUGAACUGGGAUCAGCAGGCCCUGGAAGCCUGGCUGGAAGAGUCUGCCCA
CAAGGAUUCUGACGCCCUGACACUGCAGAAGUACGCCCAGCAGGACGACAAC
AAGAUCCGGGCUCUGACCCUGCAGCUGGAAAGACUGACCCUGGAAUGCAACC
AGAAGCGGAAGAUCCUGGACAACGAGCUGACCGAGACAAUCAGCGCCCAGCU
GGAACUGGAUAAGGCCGCUCAGGACUUCAGAAAGAUCCACAACGAGCGGCAA
GAACUGAUCAAGCAGUGGGAGAACACCAUCGAGCAGAUGCAGAAACGCGACG
GCGACAUCGACAACUGCGCCCUGGAACUCGCCCGGAUCAAGCAAGAGACACG
CGAGAAAGAGAACCUGGUCAAAGAGAAGAUCAAGUUCCUCGAGUCCGAGAUC
GGCAACAACACCGAGUUCGAGAAGCGGAUCAGCGUGGCCGACAGAAAGCUGC
UGAAGUGCAGAACCGCCUACCAGGACCACGAGACAAGCCGGAUUCAGCUCAA
GGGCGAGCUGGAUUCUCUGAAGGCCACCGUGAACAGAACCAGCAGCGAUCUG
GAAGCCCUGCGGAAGAACAUCAGCAAGAUCAAGAAGGACAUCCACGAGGAAA
CCGCCAGGCUGCAGAAAACAAAGAACCACAAUGAGAUCAUCCAGACCAAGCUG
AAAGAGAUCACCGAAAAGACCAUGAGCGUGGAAGAGAAGGCCACAAACCUGG
AAGAUAUGCUCAAAGAGGAAGAGAAAGACGUCAAAGAGGUGGACGUUCAACU
GAACCUGAUUAAGGGCGUGCUGUUCAAGAAGGCCCAAGAGCUGCAGACCGAA
ACCAUGAAGGAAAAGGCCGUCCUGUCUGAGAUCGAGGGCACCAGAUCUAGCC
UGAAGCACCUGAACCAUCAGCUGCAGAAGCUCGACUUCGAGACACUGAAGCA
GCAAGAGAUCAUGUACAGCCAGGAUUUCCACAUCCAGCAGGUCGAGCGGCGG
AUGUCUAGACUGAAGGGCGAGAUCAACUCCGAGGAAAAACAGGCCCUCGAGG
CCAAGAUCGUGGAACUGAGAAAGAGCCUCGAAGAGAAGAAGUCUACCUGCGG
CCUGCUGGAAACCCAGAUUAAGAAGCUGCACAACGACCUGUACUUCAUCAAG
AAAGCCCACAGCAAGAACAGCGACGAGAAGCAGAGCCUGAUGACCAAGAUCAA
UGAGCUGAACCUGUUCAUCGAUCGGAGCGAAAAAGAGCUGGACAAGGCCAAG
GGCUUCAAGCAGGACCUGAUGAUCGAGGACAACCUGCUGAAGCUGGAAGUGA
 
AGCGGACCAGAGAGAUGCUGCACAGCAAGGCCGAGGAAGUGCUGUCUCUGG
AAAAGCGGAAGCAGCAGCUGUACACCGCCAUGGAAGAGAGAACCGAAGAGAU
CAAGGUGCACAAGACCAUGCUGGCUUCCCAGAUCAGAUACGUGGACCAAGAG
CGCGAGAACAUCUCCACCGAGUUAGAGAGACUGUCCAAGAUCGAGAAGC
UGAAGAACCGCUACGAGAUCCUGACCGUCGUGAUGCUGCCUCCUGAGGGCG
AAGAGGAAAAGACCCAGGCCUACUACGUGAUCAAGGCAGCCCAAGAAAAAGAG
GAACUCCAGAGAGAAGGCGACUGCCUGGACGCCAAGAUUAACAAGGCCGAAA
AAGAAAUCUACGCCCUCGAGAACACCCUGCAGGUCCUGAACAGCUGCAACAA
CAACUACAAGCAGAGCUUCAAGAAAGUCACCCCUAGCUCCGACGAGUACGAG
CUGAAGAUUCAGCUGGAAGAACAGAAAAGAGCCGUGGACGAGAAGUACAGAU
ACAAGCAGCGGCAGAUCAGAGAGCUGCAAGAGGAUAUCCAGAGCAUGGAAAA
CACCCUGGACGUGAUCGAGCACCUGGCCAACAACGUGAAAGAGAAGCUGUCC
GAGAAACAGGCCUACAGCUUUCAGCUGUCCAAAGAGACAGAGGAACAGAAGC
CCAAACUGGAACGCGUGACCAAGCAGUGCGCCAAGCUGACAAAAGAGAUCCG
GCUGCUGAAAGACACCAAGGACGAAACCAUGGAAGAACAAGACAUCAAGCUGC
GCGAGAUGAAGCAGUUCCACAAAGUGAUCGACGAGAUGCUGGUGGACAUCAU
UGAAGAGAACACAGAGAUCCGCAUCAUCCUGCAGACCUAUUUUCAGCAGAGC
GGCCUGGAACUGCCUACCGCCUCUACAAAGGGCAGCAGACAGAGCAGCAGAU
CCCCUAGCCACACAAGCCUGAGCGCCAGAAGCUCUAGAAGCACCAGCACCUC
UACCAGCCAGUCCAGCAUUAAGGUGCUGGAACUCAAGUUCCCCGCCAGCUCU
AGCCUCGUGGGAAGCCCUUCUAGACCUAGCAGCGCCUCUAGCAGCUCCAGCA
ACGUGAAGUCCAAGAAAAGCUCCAAGUGAUUCGAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAA

AAAA
**SEQ ID NO: 13**
   RNA encoding for human CCDC39 sequence and contains **TISU element** as 5' UTR (**ETH047T03**)
   Part of the T7 promoter, **Ethris' minimal 5' UTR with additional U nucleotide****,** ***TISU element**,* start codon (AUG), codon optimized sequence encoding a functional version of a human CCDC39 protein, stop codon (UGA), part of restriction site for BstBI, ^{poly(A) tail} 
GGGAGACUGCCAAGAUGAGCAGCGAGUUUCUGGCCGAACUGCACUGGGAGGAC
GGCUUCGCUAUUCCCGUGGCCAACGAGGAAAACAAGCUGCUGGAAGAUCAGC
UGAGCAAGCUGAAGGACGAGAGAGCCUCUCUGCAGGACGAGCUGAGAGAGUA
CGAGGAACGGAUCAACAGCAUGACCAGCCACUUCAAGAACGUGAAGCAAGAG
CUGAGCAUCACCCAGAGCCUGUGCAAGGCCAGAGAGAGAGAAACCGAGAGCG
AGGAACACUUCAAGGCUAUCGCCCAGCGCGAGCUGGGAAGAGUGAAGGAUGA
GAUCCAGCGGCUGGAAAACGAGAUGGCCAGCAUCCUGGAAAAGAAGUCCGAC
AAAGAGAACGGCAUCUUCAAGGCCACACAGAAGCUGGACGGCCUGAAGUGCC
AGAUGAACUGGGAUCAGCAGGCCCUGGAAGCCUGGCUGGAAGAGUCUGCCC
 
ACAAGGAUUCUGACGCCCUGACACUGCAGAAGUACGCCCAGCAGGACGACAA
CAAGAUCCGGGCUCUGACCCUGCAGCUGGAAAGACUGACCCUGGAAUGCAAC
CAGAAGCGGAAGAUCCUGGACAACGAGCUGACCGAGACAAUCAGCGCCCAGC
UGGAACUGGAUAAGGCCGCUCAGGACUUCAGAAAGAUCCACAACGAGCGGCA
AGAACUGAUCAAGCAGUGGGAGAACACCAUCGAGCAGAUGCAGAAACGCGAC
GGCGACAUCGACAACUGCGCCCUGGAACUCGCCCGGAUCAAGCAAGAGACAC
GCGAGAAAGAGAACCUGGUCAAAGAGAAGAUCAAGUUCCUCGAGUCCGAGAU
CGGCAACAACACCGAGUUCGAGAAGCGGAUCAGCGUGGCCGACAGAAAGCUG
CUGAAGUGCAGAACCGCCUACCAGGACCACGAGACAAGCCGGAUUCAGCUCA
AGGGCGAGCUGGAUUCUCUGAAGGCCACCGUGAACAGAACCAGCAGCGAUCU
GGAAGCCCUGCGGAAGAACAUCAGCAAGAUCAAGAAGGACAUCCACGAGGAA
ACCGCCAGGCUGCAGAAAACAAAGAACCACAAUGAGAUCAUCCAGACCAAGCU
GAAAGAGAUCACCGAAAAGACCAUGAGCGUGGAAGAGAAGGCCACAAACCUG
GAAGAUAUGCUCAAAGAGGAAGAGAAAGACGUCAAAGAGGUGGACGUUCAAC
UGAACCUGAUUAAGGGCGUGCUGUUCAAGAAGGCCCAAGAGCUGCAGACCGA
AACCAUGAAGGAAAAGGCCGUCCUGUCUGAGAUCGAGGGCACCAGAUCUAGC
CUGAAGCACCUGAACCAUCAGCUGCAGAAGCUCGACUUCGAGACACUGAAGC
AGCAAGAGAUCAUGUACAGCCAGGAUUUCCACAUCCAGCAGGUCGAGCGGCG
GAUGUCUAGACUGAAGGGCGAGAUCAACUCCGAGGAAAAACAGGCCCUCGAG
GCCAAGAUCGUGGAACUGAGAAAGAGCCUCGAAGAGAAGAAGUCUACCUGCG
GCCUGCUGGAAACCCAGAUUAAGAAGCUGCACAACGACCUGUACUUCAUCAA
GAAAGCCCACAGCAAGAACAGCGACGAGAAGCAGAGCCUGAUGACCAAGAUC
AAUGAGCUGAACCUGUUCAUCGAUCGGAGCGAAAAAGAGCUGGACAAGGCCA
AGGGCUUCAAGCAGGACCUGAUGAUCGAGGACAACCUGCUGAAGCUGGAAGU
GAAGCGGACCAGAGAGAUGCUGCACAGCAAGGCCGAGGAAGUGCUGUCUCU
GGAAAAGCGGAAGCAGCAGCUGUACACCGCCAUGGAAGAGAGAACCGAAGAG
AUCAAGGUGCACAAGACCAUGCUGGCUUCCCAGAUCAGAUACGUGGACCAAG
AGCGCGAGAACAUCUCCACCGAGUUUAGAGAGAGACUGUCCAAGAUCGAGAA
GCUGAAGAACCGCUACGAGAUCCUGACCGUCGUGAUGCUGCCUCCUGAGGG
CGAAGAGGAAAAGACCCAGGCCUACUACGUGAUCAAGGCAGCCCAAGAAAAA
GAGGAACUCCAGAGAGAAGGCGACUGCCUGGACGCCAAGAUUAACAAGGCCG
AAAAAGAAAUCUACGCCCUCGAGAACACCCUGCAGGUCCUGAACAGCUGCAAC
AACAACUACAAGCAGAGCUUCAAGAAAGUCACCCCUAGCUCCGACGAGUACGA
GCUGAAGAUUCAGCUGGAAGAACAGAAAAGAGCCGUGGACGAGAAGUACAGA
UACAAGCAGCGGCAGAUCAGAGAGCUGCAAGAGGAUAUCCAGAGCAUGGAAA
ACACCCUGGACGUGAUCGAGCACCUGGCCAACAACGUGAAAGAGAAGCUGUC
CGAGAAACAGGCCUACAGCUUUCAGCUGUCCAAAGAGACAGAGGAACAGAAG
CCCAAACUGGAACGCGUGACCAAGCAGUGCGCCAAGCUGACAAAAGAGAUCC
GGCUGCUGAAAGACACCAAGGACGAAACCAUGGAAGAACAAGACAUCAAGCU
GCGCGAGAUGAAGCAGUUCCACAAAGUGAUCGACGAGAUGCUGGUGGACAUC
AUUGAAGAGAACACAGAGAUCCGCAUCAUCCUGCAGACCUAUUUUCAGCAGA
GCGGCCUGGAACUGCCUACCGCCUCUACAAAGGGCAGCAGACAGAGCAGCAG
AUCCCCUAGCCACACAAGCCUGAGCGCCAGAAGCUCUAGAAGCACCAGCACC
UCUACCAGCCAGUCCAGCAUUAAGGUGCUGGAACUCAAGUUCCCCGCCAGCU
CUAGCCUCGUGGGAAGCCCUUCUAGACCUAGCAGCGCCUCUAGCAGCUCCAG
 
CAACGUGAAGUCCAAGAAAAGCUCCAAGUGAUUCGAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAA
AAAAAAA
**SEQ ID NO: 14**
   ETH047T05: SP30; RNA encoding for human CCDC39 and containing ***random*** ***sequence*** **of 30** ***nucleotides (SP30)*** which was used as 5' UTR
   Part of the T7 promoter, **Ethris' minimal 5' UTR,** ***SP30*** **as** **5' UTR,** ***Kozak element,*** start codon (AUG), codon optimized sequence encoding a functional version of a human CCDC39 protein, stop codon (UGA), part of restriction site for BstBI, ^{poly(A) tail}

GGGAGACAUUGAAAUUUAUCUCUUGUGUUGUGGUCGCGCCACCAUGAGCAGCGAGUUU
CUGGCCGAACUGCACUGGGAGGACGGCUUCGCUAUUCCCGUGGCCAACGAG
GAAAACAAGCUGCUGGAAGAUCAGCUGAGCAAGCUGAAGGACGAGAGAGCCU
CUCUGCAGGACGAGCUGAGAGAGUACGAGGAACGGAUCAACAGCAUGACCAG
CCACUUCAAGAACGUGAAGCAAGAGCUGAGCAUCACCCAGAGCCUGUGCAAG
GCCAGAGAGAGAGAAACCGAGAGCGAGGAACACUUCAAGGCUAUCGCCCAGC
GCGAGCUGGGAAGAGUGAAGGAUGAGAUCCAGCGGCUGGAAAACGAGAUGG
CCAGCAUCCUGGAAAAGAAGUCCGACAAAGAGAACGGCAUCUUCAAGGCCAC
ACAGAAGCUGGACGGCCUGAAGUGCCAGAUGAACUGGGAUCAGCAGGCCCUG
GAAGCCUGGCUGGAAGAGUCUGCCCACAAGGAUUCUGACGCCCUGACACUGC
AGAAGUACGCCCAGCAGGACGACAACAAGAUCCGGGCUCUGACCCUGCAGCU
GGAAAGACUGACCCUGGAAUGCAACCAGAAGCGGAAGAUCCUGGACAACGAG
CUGACCGAGACAAUCAGCGCCCAGCUGGAACUGGAUAAGGCCGCUCAGGACU
UCAGAAAGAUCCACAACGAGCGGCAAGAACUGAUCAAGCAGUGGGAGAACAC
CAUCGAGCAGAUGCAGAAACGCGACGGCGACAUCGACAACUGCGCCCUGGAA
CUCGCCCGGAUCAAGCAAGAGACACGCGAGAAAGAGAACCUGGUCAAAGAGA
AGAUCAAGUUCCUCGAGUCCGAGAUCGGCAACAACACCGAGUUCGAGAAGCG
GAUCAGCGUGGCCGACAGAAAGCUGCUGAAGUGCAGAACCGCCUACCAGGAC
CACGAGACAAGCCGGAUUCAGCUCAAGGGCGAGCUGGAUUCUCUGAAGGCCA
CCGUGAACAGAACCAGCAGCGAUCUGGAAGCCCUGCGGAAGAACAUCAGCAA
GAUCAAGAAGGACAUCCACGAGGAAACCGCCAGGCUGCAGAAAACAAAGAACC
ACAAUGAGAUCAUCCAGACCAAGCUGAAAGAGAUCACCGAAAAGACCAUGAGC
GUGGAAGAGAAGGCCACAAACCUGGAAGAUAUGCUCAAAGAGGAAGAGAAAG
ACGUCAAAGAGGUGGACGUUCAACUGAACCUGAUUAAGGGCGUGCUGUUCAA
GAAGGCCCAAGAGCUGCAGACCGAAACCAUGAAGGAAAAGGCCGUCCUGUCU
GAGAUCGAGGGCACCAGAUCUAGCCUGAAGCACCUGAACCAUCAGCUGCAGA
AGCUCGACUUCGAGACACUGAAGCAGCAAGAGAUCAUGUACAGCCAGGAUUU
CCACAUCCAGCAGGUCGAGCGGCGGAUGUCUAGACUGAAGGGCGAGAUCAAC
UCCGAGGAAAAACAGGCCCUCGAGGCCAAGAUCGUGGAACUGAGAAAGAGCC
UCGAAGAGAAGAAGUCUACCUGCGGCCUGCUGGAAACCCAGAUUAAGAAGCU
GCACAACGACCUGUACUUCAUCAAGAAAGCCCACAGCAAGAACAGCGACGAGA

AGCAGAGCCUGAUGACCAAGAUCAAUGAGCUGAACCUGUUCAUCGAUCGGAG
CGAAAAAGAGCUGGACAAGGCCAAGGGCUUCAAGCAGGACCUGAUGAUCGAG
GACAACCUGCUGAAGCUGGAAGUGAAGCGGACCAGAGAGAUGCUGCACAGCA
AGGCCGAGGAAGUGCUGUCUCUGGAAAAGCGGAAGCAGCAGCUGUACACCGC
CAUGGAAGAGAGAACCGAAGAGAUCAAGGUGCACAAGACCAUGCUGGCUUCC
CAGAUCAGAUACGUGGACCAAGAGCGCGAGAACAUCUCCACCGAGUUUAGAG
AGAGACUGUCCAAGAUCGAGAAGCUGAAGAACCGCUACGAGAUCCUGACCGU
CGUGAUGCUGCCUCCUGAGGGCGAAGAGGAAAAGACCCAGGCCUACUACGUG
AUCAAGGCAGCCCAAGAAAAAGAGGAACUCCAGAGAGAAGGCGACUGCCUGG
ACGCCAAGAUUAACAAGGCCGAAAAAGAAAUCUACGCCCUCGAGAACACCCUG
CAGGUCCUGAACAGCUGCAACAACAACUACAAGCAGAGCUUCAAGAAAGUCAC
CCCUAGCUCCGACGAGUACGAGCUGAAGAUUCAGCUGGAAGAACAGAAAAGA
GCCGUGGACGAGAAGUACAGAUACAAGCAGCGGCAGAUCAGAGAGCUGCAAG
AGGAUAUCCAGAGCAUGGAAAACACCCUGGACGUGAUCGAGCACCUGGCCAA
CAACGUGAAAGAGAAGCUGUCCGAGAAACAGGCCUACAGCUUUCAGCUGUCC
AAAGAGACAGAGGAACAGAAGCCCAAACUGGAACGCGUGACCAAGCAGUGCG
CCAAGCUGACAAAAGAGAUCCGGCUGCUGAAAGACACCAAGGACGAAACCAU
GGAAGAACAAGACAUCAAGCUGCGCGAGAUGAAGCAGUUCCACAAAGUGAUC
GACGAGAUGCUGGUGGACAUCAUUGAAGAGAACACAGAGAUCCGCAUCAUCC
UGCAGACCUAUUUUCAGCAGAGCGGCCUGGAACUGCCUACCGCCUCUACAAA
GGGCAGCAGACAGAGCAGCAGAUCCCCUAGCCACACAAGCCUGAGCGCCAGA
AGCUCUAGAAGCACCAGCACCUCUACCAGCCAGUCCAGCAUUAAGGUGCUGG
AACUCAAGUUCCCCGCCAGCUCUAGCCUCGUGGGAAGCCCUUCUAGACCUAG
CAGCGCCUCUAGCAGCUCCAGCAACGUGAAGUCCAAGAAAAGCUCCAAGUGA
UUCGAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAA

## Claims

1. A method for analyzing the effect of a polyribonucleotide on ciliogenesis, wherein said polyribonucleotide encodes a protein involved in and/or required for ciliogenesis, said method comprising the steps of:
(a) obtaining a nose brush of a subject having a ciliopathy, said nose brush comprising undifferentiated basal cells and differentiated ciliated cells,
(b) culturing the cells obtained from step (a) as a submerse cell culture for obtaining undifferentiated basal cells and dedifferentiated ciliated cells,
(c) culturing undifferentiated basal cells and dedifferentiated ciliated cells obtained from step (b) as an air liquid interface cell culture and performing an air lift,
(d) transfecting cells obtained from step (c) with a polyribonucleotide encoding a protein involved in and/or required for ciliogenesis,
(e) culturing the transfected cells obtained from step (d) for obtaining differentiated ciliated cells, and
(f) determining the effect of said polyribonucleotide on ciliogenesis using a lactate dehydrogenase measurement, a NucGreen assay, a high speed video microscopy, a ciliary beat frequency measurement, a mucociliary clearance assay, and/or immunofluorescence staining.

2. The method according to claim 1, wherein the cells are transfected within 0 to 48 hours after the air lift is performed in step (c).

3. The method according to claim 1 or 2, wherein the cells are transfected with a polyribonucleotide encoding a protein involved in and/or required for ciliogenesis using a lipidoid having the structure shown in formula (V):

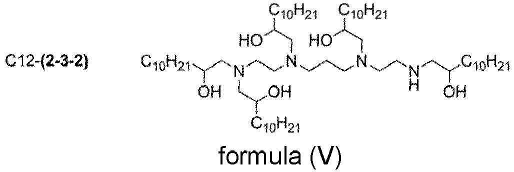

4. The method according to any of claims 1 to 3, wherein the cells are cultured in steps (b) to (e) using Medium G having the following composition:
| **Substance** | **Volume** | **Final concentration** |
|---|---|---|
| DMEM/Ham's F12 | 100 mL | |
| Ultroser G | 2 mL | 2% |
| Fetal Clone II | 2 mL | 2% |
| Insulin | 100 µL | 2.5 µg/mL |
| Bovine Brain Extract | 250 µL | 22.5 µg/mL |
| Transferrin | 100 µL | 2.5 µg/mL |
| Hydrocortisone | 20 µL | 20 nM |
| 3,3',5-Triiodo-L-thyronine | 3.33 µL | 500 nM |
| Epinephrine | 10 µL | 1.5 µM |
| Retinoic Acid | 10 µL | 10 nM |
| Phosphorylethanolamine | 5 µL | 250 nM |
| Ethanolamine | 100 µL | 250 nM |
| DAPT | 10 µL | 1 µM |

5. The method according to any of claims 1 to 4, wherein the nose brush further comprises fibroblasts and wherein growth of said fibroblasts is inhibited in steps (b) to (e).

## Patentansprüche

1. Verfahren zur Untersuchung der Wirkung eines Polyribonucleotids auf die Ciliogenese, wobei das Polyribonucleotid ein Protein codiert, das an der Ciliogenese beteiligt ist und/oder für diese erforderlich ist, wobei das Verfahren die Schritte umfasst:
(a) Erhalten eines Nasenabstrichs eines Individuums, das an einer Ciliopathie leidet, wobei der Nasenabstrich undifferenzierte Basalzellen und differenzierte ciliierte Zellen umfasst,
(b) Züchten der aus Schritt (a) erhaltenen Zellen als submerse Zellkultur, um undifferenzierte Basalzellen und dedifferenzierte ciliierte Zellen zu erhalten,
(c) Züchten von aus Schritt (b) erhaltenen undifferenzierten Basalzellen und dedifferenzierten ciliierten Zellen als Air-liquid-Interface-Zellkultur und Durchführen eines Air-Lifts,
(d) Transfizieren der aus Schritt (c) erhaltenen Zellen mit einem Polyribonucleotid, das ein Protein codiert, das an der Ciliogenese beteiligt und/oder für diese erforderlich ist,
(e) Züchten der aus Schritt (d) erhaltenen transfizierten Zellen, um differenzierte ciliierte Zellen zu erhalten, und
(f) Bestimmen der Wirkung des Polyribonucleotids auf die Ciliogenese unter Verwendung einer Lactatdehydrogenasemessung, eines NucGreen-Assays, von Hochgeschwindigkeits-Videomikroskopie, einer Messung der Cilienschlagfrequenz, eines mukociliären Clearance-Tests und/oder von Immunfluoreszenzfärbung.

2. Verfahren nach Anspruch 1, wobei die Zellen innerhalb von 0 bis 48 Stunden nach Durchführung des in Schritt (c) durchgeführten Air-Lifts transfiziert werden.

3. Verfahren nach Anspruch 1 oder 2, wobei die Zellen mit einem Polyribonucleotid, das ein Protein codiert, das an der Ciliogenese beteiligt und/oder für diese erforderlich ist, unter Verwendung eines Lipidoids mit der in Formel (V) gezeigten Struktur transfiziert werden:

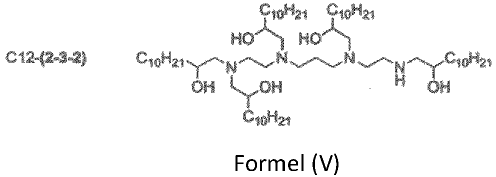

4. Verfahren nach einem der Ansprüche 1 bis 3, wobei die Zellen in den Schritten (b) bis (e) unter Verwendung von Medium G mit der folgenden Zusammensetzung:
| **Substanz** | **Volumen** | **Endkonzentration** |
|---|---|---|
| DMEM/Ham's F12 | 100 mL | |
| Ultroser G | 2 mL | 2 % |
| FetalClone II | 2 mL | 2 % |
| Insulin | 100 µL | 2,5 µg/mL |
| Rindergehirnextrakt | 250 µL | 22,5 µg/mL |
| Transferrin | 100 µL | 2,5 µg/mL |
| Hydrocortison | 20 µL | 20 nM |
| 3,3',5-Triiod-L-thyronin | 3,33 µL | 500 nM |
| Epinephrin | 10 µL | 1,5 µM |
| Retinolsäure | 10 µL | 10 nM |
| Phosphorylethanolamin | 5 µL | 250 nM |
| Ethanolamin | 100 µL | 250 nM |
| DAPT | 10 µL | 1 µM |
gezüchtet werden.

5. Verfahren nach einem der Ansprüche 1 bis 4, wobei der Nasenabstrich des Weiteren Fibroblasten umfasst und wobei das Wachstum der Fibroblasten in den Schritten (b) bis (e) inhibiert wird.

## Revendications

1. Méthode pour analyser l'effet d'un polyribonucléotide sur la ciliogenèse, dans laquelle ledit polyribonucléotide code une protéine impliquée dans et/ou requise pour la ciliogenèse, ladite méthode comprenant les étapes de :
(a) obtention d'un écouvillonnage nasal d'un sujet ayant une ciliopathie, ledit écouvillonnage nasal comprenant des cellules basales indifférenciées et des cellules ciliées différenciées,
(b) culture des cellules obtenues dans l'étape (a) sous la forme d'une culture cellulaire immergée pour obtenir des cellules basales indifférenciées et des cellules ciliées dédifférenciées,
(c) culture de cellules basales indifférenciées et de cellules ciliées dédifférenciées obtenues dans l'étape (b) sous la forme d'une culture cellulaire à interface liquide et réalisation d'une agitation par circulation d'air,
(d) transfection de cellules obtenues dans l'étape (c) avec un polyribonucléotide codant une protéine impliquée dans et/ou requise pour la ciliogenèse,
(e) culture des cellules transfectées obtenues dans l'étape (d) pour obtenir des cellules ciliées différenciées, et
(f) détermination de l'effet dudit polyribonucléotide sur la ciliogenèse utilisant une mesure de lactate déshydrogénase, un dosage NucGreen, une microscopie vidéo à grande vitesse, une mesure de fréquence des battements ciliaires, un dosage de clairance mucociliaire, et/ou une coloration par immunofluorescence.

2. Méthode selon la revendication 1, dans laquelle les cellules sont transfectées dans les 0 à 48 heures après que l'agitation par circulation d'air a été réalisée dans l'étape (c).

3. Méthode selon la revendication 1 ou 2, dans laquelle les cellules sont transfectées avec un polyribonucléotide codant une protéine impliquée dans et/ou requise pour la ciliogenèse utilisant un lipidoïde ayant la structure représentée par la formule (V) :

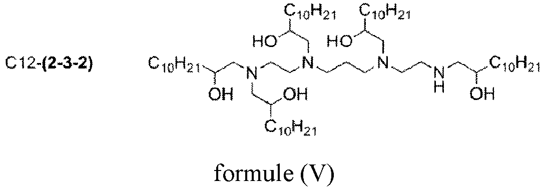

4. Méthode selon l'une quelconque des revendications 1 à 3, dans laquelle les cellules sont cultivées dans l'étape (b) et (e) par utilisation de milieu G ayant la composition suivante :
| Substance | Volume | Concentration finale |
|---|---|---|
| DMEM/F12 de Ham | 100 ml | |
| Ultroser G | 2 ml | 2 % |
| Clone foetal II | 2 ml | 2 % |
| Insuline | 100 µl | 2,5 µg/ml |
| Extrait de cerveau de bovin | 250 µl | 22,5 µg/ml |
| Transferrine | 100 µl | 2,5 µg/ml |
| Hydrocortisone | 20 µl | 20 nM |
| 3,3',5-triiodo-L-thyronine | 3,33 µl | 500 nM |
| Epinéphrine | 10 µl | 1,5 µm |
| Acide rétinoïque | 10 µl | 10 nM |
| Phosphoryléthanolamine | 5 µl | 250 nM |
| Ethanolamine | 100 µl | 250 nM |
| DAPT | 10 µl | 1 µM |

5. Méthode selon l'une quelconque des revendications 1 à 4, dans laquelle l'écouvillonnage nasal comprend en outre des fibroblastes, et dans laquelle la croissance desdits fibroblastes est inhibée dans les étapes (b) à (e).
